(19) Europäisches Patentamt
European Patent Office
Office européen des brevets

(11) Publication number: 0 283 147 B1

# (12) EUROPEAN PATENT SPECIFICATION

(45) Date of publication of patent specification: 11.05.94

(51) Int. Cl.[5]: C07C 251/32, C07D 285/10, C07D 215/20, C07C 225/02, A61K 31/15, A61K 31/12, A61K 31/47, A61K 31/41

(21) Application number: 88301615.6

(22) Date of filing: 25.02.88

## (54) Labile ketone derivatives of 3-substituted-1-alkylamino-2-propanols and their use as beta-adrenergic blockers.

(30) Priority: 16.03.87 US 26002

(43) Date of publication of application: 21.09.88 Bulletin 88/38

(45) Publication of the grant of the patent: 11.05.94 Bulletin 94/19

(84) Designated Contracting States: AT BE CH DE ES FR GB GR IT LI LU NL SE

(56) References cited:
FR-A- 2 070 067
US-A- 4 521 414

J. MED. CHEM, vol. 31, no. 1, January 1988, pages 100-106, American Chemical Society; N. BODOR et al.: "Improved delivery through biological membranes. 26. Design, synthesis and pharmacological activity of a novel chemical delivery system for beta-adrenergic blocking agents"

(73) Proprietor: UNIVERSITY OF FLORIDA
223 Grinter Hall
Gainesville, Florida 32611(US)

(72) Inventor: Bodor, Nicholas S.
7211 SW 97th Lane
Gainesville Florida 32608(US)

(74) Representative: Hedley, Nicholas James Matthew et al
Stephenson Harwood
One, St. Paul's Churchyard
London EC4M 8SH (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to 3-substituted-1-alkylamino-2-propanol derivatives having β-adrenergic blocking properties.

### Prior Art

β-adrenergic blockers were first reported to be useful for the therapeutic treatment of glaucoma in 1967 [Phillips et al, Brit. J. Ophthal., 1967, 51, 222]. In 1978 timolol was approved for market use and since that time the drug has become very popular with ophthalmologists as an effective antiglaucoma agent. Recently, however, a vast number of serious cardiovascular, respiratory, CNS, and ocular side effects secondary to topical ocular timolol administration has been reported [Ahmad, The Lancet, 1979, 2, 1028; Buskirk, Ophthalmology, 1980, 87, 447; Mishra et al, J. Anaesth., 1983, 55, 897; and Linkewich et al, Am. J. Hosp. Pharm., 1981, 38, 699]. Currently, timolol is no longer the sole β-blocker used to treat glaucoma. Befanolol, carteolol and metipranolol were introduced recently and a number of other newer β-adrenergic antagonists (e.g., L-bunolol, betaxolol, celiprolol, cetamolol, etc.) are currently under investigation as antiglaucoma agents.

It became desirable to design an antiglaucoma drug which could be delivered to the eye compartments in a sustained and controlled manner with minimal systemic absorption and/or no systemic side effects.

It was previously found that after topical application to the eye, esters of adrenalone but not adrenalone itself can be converted via a reduction-hydrolysis sequence to deliver adrenaline (epinephrine) at the iris-ciliary body, the desired site of action [Bodor et al, Exp. Eye. Res., 1984, 38, 621]. Research was conducted to ascertain whether lipophilic ketones could also be reduced in the iris-ciliary body.

It was hypothesized that ketone precursors of β-blockers which are also β-hydroxylamines like adrenaline could then possibly be converted to the active β-blockers in the iris-ciliary body by a reductive process. Various attempts, however, to synthesize the ketones corresponding to a number of β-blockers (i.e., propranolol, timolol, carteolol, etc.) failed, due to the chemical instability of these β-amino-ketone esters.

It is an object of the present invention to provide novel hydrolytically sensitive precursors of the ketone precursors of the β-adrenergic blocking β-hydroxylamines which are readily converted to the active β-blockers in the iris-ciliary body by combined hydrolytic and reductive processes.

### SUMMARY OF THE INVENTION

This and other objects are realized by the present invention which provides novel compounds having the formula:

$$Ar - X - CH_2 - \overset{\overset{\displaystyle Y}{\|}}{C} - CH_2 - NHR \qquad (I)$$

Wherein:

-X- is -O-, $-CH_2-$ or — ;

=Y is a derivatized keto group which is hydrolyzable or enzymatically convertible to a keto group;

R is substituted or unsubstituted alkyl having from 1 to 12 carbon atoms or substituted or unsubstituted aralkyl having from 7 to 20 carbon atoms,said substituents not adversely affecting the β-adrenergic blocking and other pharmaceutical properties of the compound and

Ar is the residue of a 1-alkylamino-2-propanol having a cyclic substituent at the 3-position thereof, said substituted propanol having β-adrenergic blocking properties; and

acid-addition salts thereof with pharmaceutically acceptable acids; the invention also provides the compounds as defined for use in therapy.

Another embodiment of the invention comprises a pharmaceutical composition in unit dosage form comprising a β-adrenergic blocking effective amount of one of the above described compounds or salts and a pharmaceutically acceptable carrier therefor.

A further embodiment of the invention comprises use of one of the above described compounds or salts in the preparation of a medicament having a β-adrenergic blocking effect.

Another embodiment of the invention comprises an ophthalmic pharmaceutical composition comprising an effective interocular pressure reducing amount of one of the above described compounds or salts, or a corresponding compound or salt wherein =Y is =O, and a non-toxic ophthalmically acceptable carrier therefor.

Yet another embodiment of the invention comprises use of a compound or salt of formula (I), or a corresponding compound or salt wherein =Y is =O, in the preparation of a medicament for treating glaucoma or for lowering intraocular pressure.

DETAILED DESCRIPTION OF THE INVENTION

The present invention is predicated on the discovery that the hydrolytically sensitive oxime-type and other labile ketone groups of the compounds of the invention would enable the delivery to and hydrolysis and reduction of the derivative at the iris-ciliary site of action to the active β-adrenergic blocking amino-alcohol.

The preferred compounds of the invention are those of the formula (I) above wherein

Y is $=N-OR_1$, $=N-NH_2$, $=N-NR_1R_2$,

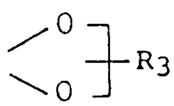, 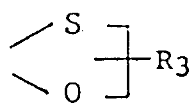 or 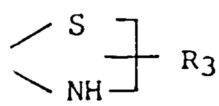

Wherein: $R_1$ and $R_2$ may be the same or different and are H, alkyl having from 1 to 8 carbon atoms or aryl or aralkyl having from 6 to 15 carbon atoms;

$R_3$ is $R_1$, $-COOR_1$ or $-CON(R_1)_2$, and

Ar and R have the meanings set forth above.

It will be understood, however, that Y may be any group or substituent which is readily hydrolyzed or enzymatically converted to a hydroxyl group at the intended site of action, preferably at the cornea or iris-ciliary body after administration of the parent compound to the animal undergoing treatment. In addition, in the ophthalmic composition and uses of the invention, =Y in the compounds of formula (I) and their salts can be =O, i.e. the ketones corresponding to formula (I) can be utilized.

It is further preferred to employ those compounds of the above formula wherein R is a sterically hindered group such as the secondary or tertiary alkyls e.g., isopropyl, t-butyl, etc. Suitable aralkyl groups include benzyl, 3,4-dimethoxyphenethyl, 1-phenethylethyl, etc.; it being understood that by the term, "aralkyl", is intended any hydrocarbyl group.

Ar may be any aromatic or heterocyclic residue of the known 3-aromatic or 3-heterocyclic substituted 1-alkylamino-2-propanol β-blockers, e.g., those residues having the formulae:

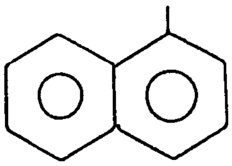 , 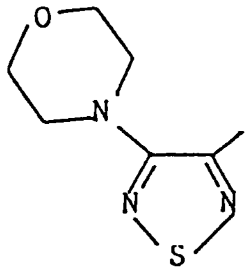 , 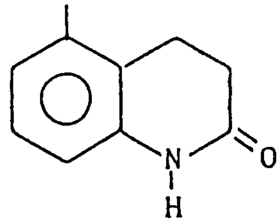 ,

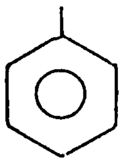 , 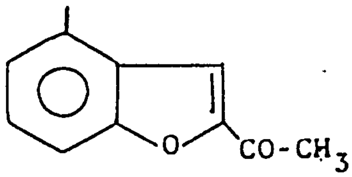 , 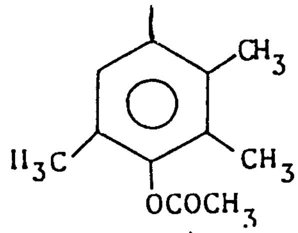 ,

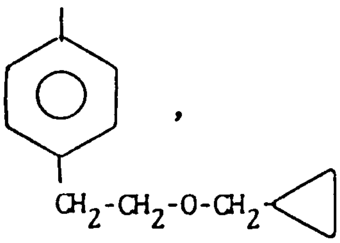 , 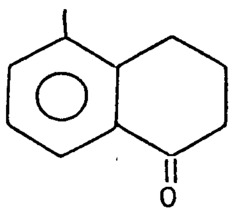 , 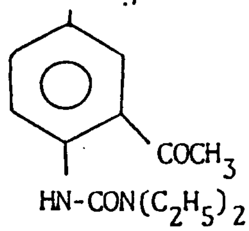 ,

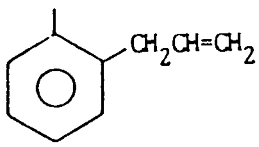 , 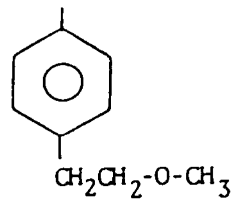 , 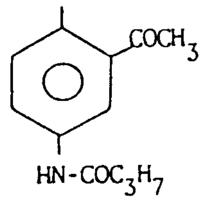 ,

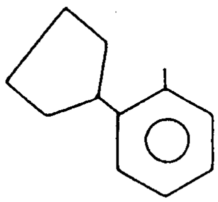 , 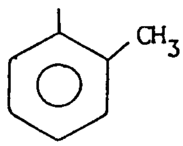 , 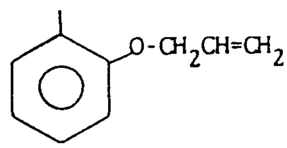 ,

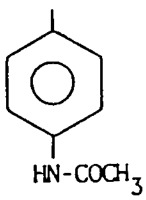 , 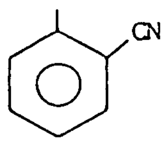 , 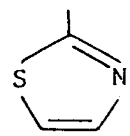 ,

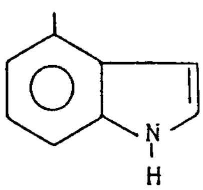 , 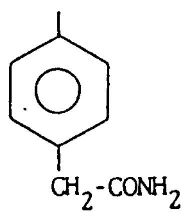 , 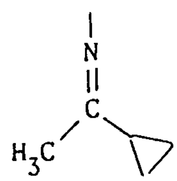 , 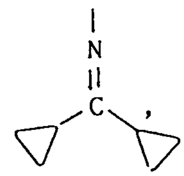 ,

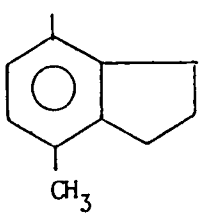 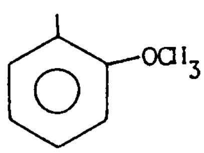 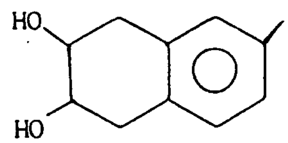

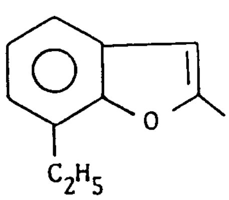 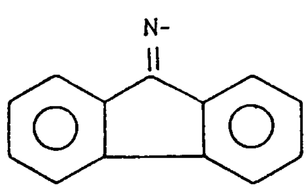 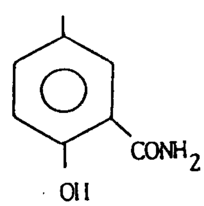 , etc.

Any pharmaceutically acceptable acid may be used to form the acid-addition salts of the invention, e.g., HCl, $H_2SO_4$, $H_3PO_4$, maleic, succinic, methanesulfonic, citric acid , etc.

The preferred compounds according to the present invention are those having the formulae in Table A.

TABLE A

Ar - X - $CH_2$ - Z - $CH_2$ - NH - R

| X | Z = CH(OH) β-blocker No. | Name | Z = C(=N-OH) Compound No. | Ar | R |
|---|---|---|---|---|---|
| O | 1 | Propranolol | 1a | 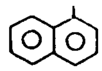 | $-CH(CH_3)_2$ |
| O | 2 | Timolol | 1b | 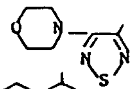 | $-C(CH_3)_3$ |
| O | 3 | Carteolol | 1c | 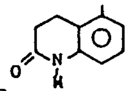 | $-C(CH_3)_3$ |
| O | 4 | I-pr-Timolol | 1d | 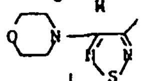 | $-CH(CH_3)_2$ |
| O | 5 | Alprenolol | 1e | 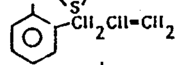 | $-CH(CH_3)_2$ |
| O | 6 | Atenolol | 1f | 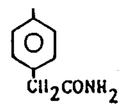 | $-CH(CH_3)_2$ |
| O | 7 | Befunolol | 1g | 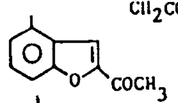 | $-CH(CH_3)_2$ |
| O | 8 | Betaxolol | 1h | 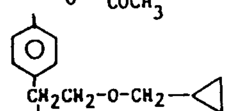 | $-CH(CH_3)_2$ |
| O | 9 | Bevantolol | 1i | 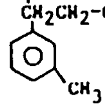 | 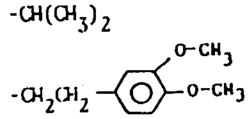 |
| O | 10 | Bufuralol | 1j | 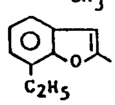 | $-C(CH_3)_3$ |
| O | 11 | Bunitrolol | 1k | 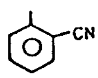 | $-C(CH_3)_3$ |
| O | 12 | Bupranolol | 1l | 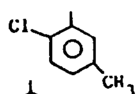 | $-C(CH_3)_3$ |
| O | 13 | Celiprolol | 1m | 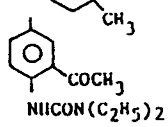 | $-C(CH_3)_3$ |

TABLE A (continued)

$Ar - X - CH_2 - Z - CH_2 - NH - R$

| X | Z = CH(OH) β-blocker No. | Name | Z = C(=N-OH) Compound No. | Ar | R |
|---|---|---|---|---|---|
| O | 14 | Cetamolol | 1n | 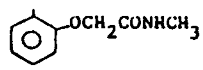 | $-C(CH_3)_3$ |
| O | 15 | Falintolol | 1o | 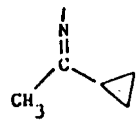 | $-C(CH_3)_3$ |
| O | 16 | ICI-118,551 | 1p | 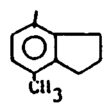 | $-C(CH_3)_3$ |
| O | 17 | IPS-339 | 1r | 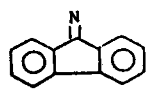 | $-C(CH_3)_3$ |
| O | 18 | Labetolol | 1s | 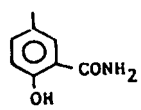 | 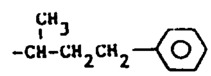 |
| O | 19 | Levobunolol | 1t | 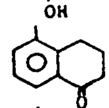 | $-C(CH_3)_3$ |
| O | 20 | Mepindolol | 1u | 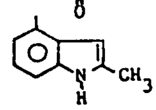 | $-(CH(CH_3)_2$ |
| O | 21 | Metipranolol | 1v | 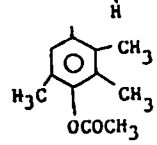 | $-CH(CH_3)_2$ |
| O | 22 | Metoprolol | 1x | 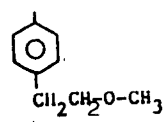 | $-CH(CH_3)_2$ |
| O | 23 | L-Moprolol | 1y | 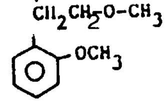 | $-CH(CH_3)_2$ |
| O | 24 | Nadolol | 1z | 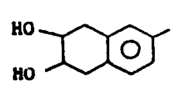 | $-C(CH_3)_3$ |
| O | 25 | Diacetyl-nadolol | 1aa | 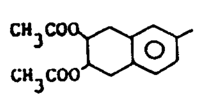 | $-C(CH_3)_3$ |
| O | 26 | Oxprenolol | 1bb | 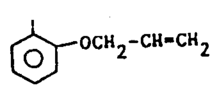 | $-CH(CH_3)_2$ |

TABLE A (continued)

Ar - X - $CH_2$ - Z - $CH_2$ - NH - R

| X | Z = CH(OH) β-blocker No. | Name | Z = C(=N-OH) Compound No. | Ar | R |
|---|---|---|---|---|---|
| 0 | 27 | Penbutolol | 1cc | 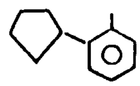 | $-C(CH_3)_3$ |
| 0 | 28 | Pindolol | 1dd | 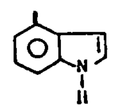 | $-CH(CH_3)_2$ |
| 0 | 29 | Pivaloyl Pindolol | 1ee | 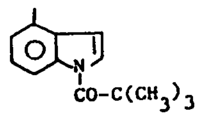 | $-CH(CH_3)_2$ |
| 0 | 30 | Toliprolol | 1gg | 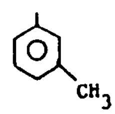 | $-CH(CH_3)_2$ |

Particularly preferred are compounds 1e, 1h, 1l, 1cc, 1k, 1p, 1t, 1v, 1bb, 1i and 1s.

The following overall reaction scheme may be employed to prepare the products of the invention:

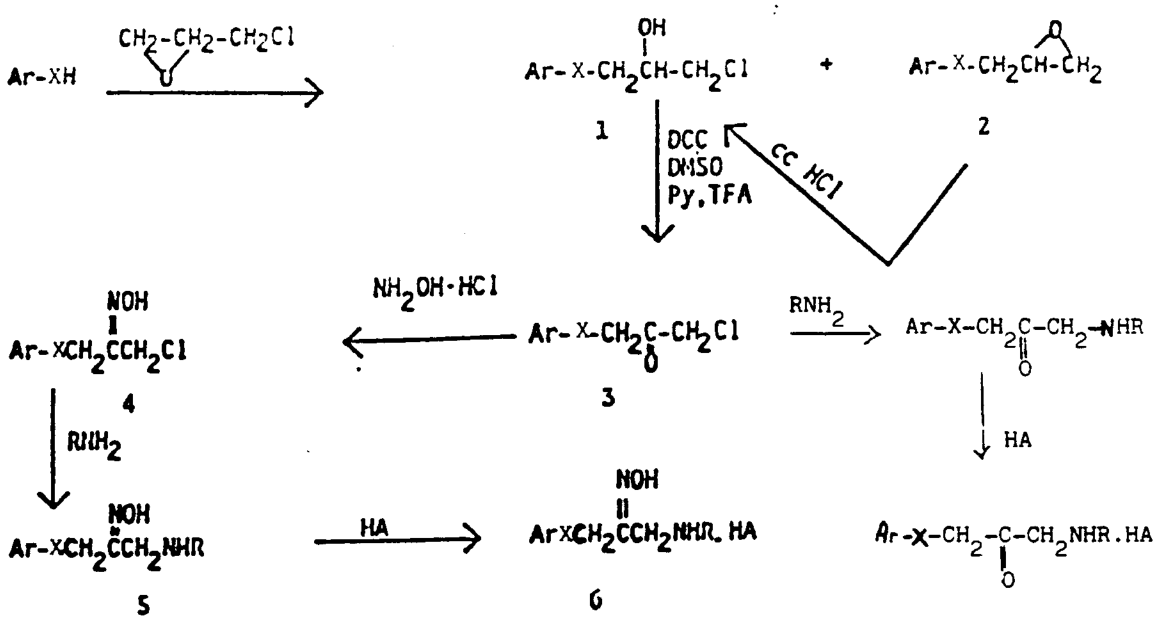


(X = - O -, - $CH_2$- or a valence bond)

The conventional reaction of Ar-OH and epichlorohydrin with a small amount of morpholine as catalyst affords a mixture of the chlorohydrin 1 and the epoxide 2; the latter being converted to 1 by treatment with conc. HCl. Oxidation of 1 by the Pfitzner-Moffat method [Pfitzner et al, 1965, J. Am. Chem. Soc., 87, 5661 and 5670] yields the ketone 3. Subsequent reaction of 3 with hydroxylamine HCl gives the oxime 4, which is a mixture of the Z- and E-isomers. In the usual case the ratio is about 1:2 , as determined by NMR [Silverstein, "Spectrometric identification of organic compounds", 1974, 3rd ed.: G. Clayton Bassler and Terence C. Morril, New York, Wiley].

The major product, 4 (E-), can be isolated by recrystallization from benzene. Treatment of 4 with isopropylamine in THF gives the oxime 5 essentially as the pure Z-isomer, which can be converted to the HCl salt 6. Alternately, the oxidation of the racemic β-blocker

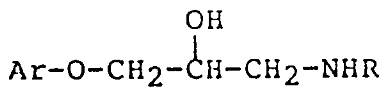

with DCC/$(COCl)_2$ at -20° to -78° will result in the ketone

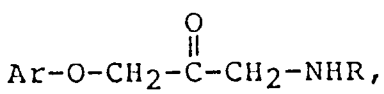

which can be converted to 6 without isolation by adding $H_2NOH$ HCl.

The invention is illustrated by the following non-limiting examples wherein melting points were determined with a Fisher-Johns melting point apparatus and are uncorrected. The 90-MHz NMR spectra were taken on a Varian EM390 NMR spectrometer. TLC was performed on 0.25/mm Merck silica gel 60 F-254 glass plates.

EXAMPLE 1

The synthesis of the propanolone oxime (6a) is a typical example.

1-(Isopropylamino)-3-(1-naphthyloxy)-2-propanone oxime HCl, Propranolone oxime hydrochloride (6a)

3-Chloro-1-(1-naphthyloxy)-2-propanol (1a)

A mixture of 1-naphthol (20g, 0.14 mole), ephichlorohydrin (51.3g, 0.55 mole), and morpholine (0.7 ml) was heated at 100-120°C for 7.5 h. Excess epichlorohydrin and morpholine were removed under reduced pressure, the residue was dissolved in chloroform and shaken with 10 ml of conc. HCl to convert (2a) to the chlorohydrin (1a). The organic layer was separated and washed with water, then with dil. $NaHCO_3$ and finally with water. It was dried over anhydrous $MgSO_4$ and concentrated to yield 29.8g (98%) of the crude product. This was used in the next step (oxidation) without purification.

Purification of a sample of the crude (1a) was carried out by column chromatography (silica gel: Aldrich 100-200 mesh, 60 Å x4W, eluent $CHCl_3$). NMR ($CDCl_3$) δ 8.15 (m, 1H), δ 7.75 (m, 1H) δ 7.5-7.2 (m, 4H), δ 6.7 (d, d, J=7 Hz, J=1 Hz, 1H), δ 4.35-3.50 (m, 5H), δ 2.9 (d, J=6 Hz, 1H).

3-Chloro-1-(1-naphthyloxy)-2-propanone (3a)

To a solution of 1,3-dicyclohexylcarbodiimide (DCC) (47.1g, 0.228 mole DMSO (36 ml), and pyridine (3.6 ml) in diethyl ether (300 ml) was added a solution of (1a) (18.0g, 76 mmole) in diethyl ether (36 ml). To this solution was then added dropwise a solution of trifluoroacetic acid (1.8 ml) in diethyl ether under ice-water cooling, and the mixture was stirred at room temperature for 1 h and allowed to stand overnight. A solution of oxalic acid (18g) in MeOH was added to the reaction mixture in small portions, and the stirring was continued for 0.5 h. The dicyclohexylurea was filtered and washed with ether. The filtrate was washed

with a 5% $NaHCO_3$ solution, then with water and dried over anhydrous $MgSO_4$. From the filtrate 6.3g of the desired compound was recovered. The mother liquor was concentrated under reduced pressure and the residue was recrystallized from 2-propanol to yield an additional 3.3g. The total yield was 9.6g (Y = 56%). This product was used in the next step without further purification. A pure sample was obtained by column chromatography (silica gel: Aldrich 100-200 mesh, 60 Å x 7W, eluent $CHCl_3$: hexane = 3.1). NMR($CDCl_3$) δ 8.25 (m, 1H), δ 7.80 (m, 1H), δ 7.65 7.20 (m, 4H), δ 6.75 (d, J = 7 Hz, 1H), δ 4.83 (s, 2H), δ 4,43 (s, 2H).

3-Chloro-1-(1-naphthyloxy)-2-propanone oxime (4a)

A mixture of (3a) (1.0g, 4.26 mmole), hydroxylamine hydrochloride (0.36g, 5.1 mmole), and DMSO (10 ml) was heated at 40-60 °C for half an hour. Water (40 ml) was introduced and the solution was extracted with $CHCl_3$. The organic layer was washed with water several times, dried over anhydrous magnesium sulfate, and concentrated under reduced pressure. The crude yield was 1.1g (Y = 100%). Further purification was carried out by column chromatography (silica gel: Aldrich 100-200 mesh, 60 Å x 30W, eluent: benzene:AcOEt = 4:1). The product was a mixture of Z- and E- isomer (E:Z = 2:1). This isomer mixture could be used in the next step. NMR ($CDCl_3$ + DMSO-d6 (1 drop) δ 10.85 (s, 0.33H, -NOH of z-isomer), δ 10.75 (s, 0.67H, -NOH of E-isomer), δ 8.4-8.1 (m, 1H), δ 7.9-7.65 (m, 1H), δ 7.6-7.2 (m, 4H), δ 6.95-6.7 (m, 1H), δ 5.2 (s, 1.33H, $OCH_2$ of E-isomer), δ 4.9 (s, 0.67H, $OCH_2$ of Z-isomer), δ 4.45 (s, 0.67H, $-CH_2Cl$ of Z-isomer) δ 4.3 (s, 1.33H, $-CH_2Cl$ of E-isomer).

The E-isomer was isolated from the crude product by recrystallization from benzene. M.p. 162-163 °C.

1-(Isopropylamino)-3-(1-naphthyloxy)-2-propanone oxime, Propranolone oxime (5a)

A mixture of (4a) (2.5g, 10 mmole), isopropylamine (6.0g, 8.7 ml, 100 mmole), and THF (50 ml) was heated at 50 °C for 1.5 h. The reaction mixture was concentrated under reduced pressure. To the residue was added dil. $NaHCO_3$ and the solution was extracted with ethyl acetate. After the organic extract was shaken with dil. HCl solution, the separated aqueous layer was made basic with dil. HCl solution, extracted with AcOEt, dried over anhydrous $MgSO_4$, and concentrated under reduced pressure. The crude yield was 2.6g (Y = 95%). The crude product was purified from a mixed solvent of isopropyl ether and hexane. The pure yield was 0.98g (Y = 36%). M.p. 131.5-132.5 °C. This product was the Z-isomer only. NMR($CDCl_3$) δ 8.30-8.20 (m, 1H, one of H of naphthalene), δ 6.90-6.80 (m, 1H, one of H of naphthalene), δ 5.16 (2, 2H, -OCH2-), δ 3.70 (s, 2H, -CH2N-), δ 3.05-2.70 (m, 1H, N-CH<), δ 1.10.

1-(Isopropylamino)-3-(1-naphthyloxy)-2-propanone oxime hydrochloride, Propranolone oxime Hydrochloride (6a)

To diethyl ether saturated with HCl gas was added a solution of propranolone oxime (5a) (0.30g) in diethyl ether. The mixture was stirred at room temperature for 0.5 h. The precipitated white crystals were filtered and dried *in vacuo* overnight. The yield was 0.32g (Y = 94%). This product was essentially pure Z-isomer. NMR (DMSO-d6) δ 12.00 (s, 1H, -NOH), δ 8.30-8.15 (m, 1H, of naphthalene), δ 7.95-7.80 (m, 1H, of naphthalene), δ 7.65-7.30 (m, 4H, part of naphthalene), δ 7.05-6.95) (m, 1H, one of H of naphthalene), δ 5.15 (s, 2H, $-OCH_2$), δ 3.96 (s, 2H, $-CH_2N$), δ 3.55-3.20 (m, 2H, NCH, -NH), δ 1.27 (d, J = 6 Hz, 6H, $-(CH_3)2$).
Anal. ($C_{16}H_{20}O_2N_2 \cdot HCl$) C,H,N.

EXAMPLE 2

1-(tert-Butylamino)-3-[(4-morpholino-1,2,5-thiadiazol-3-yl)oxy]-2-propanone oxime oxalate, timolone oxime oxalate, (6b)

3-Chloro-1-[(4-morpholino-1,2,5-thiadiazol-3-yl)oxy]-2-propanol,

was synthesized according to the method given for (1a). Yield 83%; the crude compound was used in the next step. NMR ($CDCl_3$) δ 4.5 (d, J = 5 Hz, 2H), δ 4.2 (pentet, J = 5 Hz, 1H), δ 3.8- δ 3.65 (m, 6H), δ 3.55-3.40 (m, 4H). The peak of C-OH could not be identified.

3-Chloro-1-[(4-morpholino-1,2,5-thiadiazol-3-yl)oxy]-2-propanone (3b) was synthesized according to the method given for (3a). Yield: 65%. The crude product was used in the next step. Purification of the crude product (recrystallization from 2-propanol) yielded the pure sample. NMR ($CDCl_3$) δ 5.22 (s, 2H), δ 4.13 (s, 2H), δ 3.75 (m, 4H, δ 3.50 (m, 4H).

3-Chloro-1-[(4-morpholino-1,2,5-thiadiazol-3-yl)oxy]-2-propanone oxime (4b)

In a 500 ml round-bottomed flask were placed (3b) (13.69, 49 mmole) and hydroxylamine hydrochloride (5.1g, 73.4 mmole), in an ethanol-DMF mixed solvent (266 ml). The mixture was stirred at room temperature for 18 hours. The reaction mixture was poured into water (2L) and was extracted with ether. The organic extract was washed well with water, dried over anhydrous $MgSO_4$, and was concentrated in vacuo at 30 ° C. The crude yield was 11.8g (Y = 83%). The product was a mixture of Z- and E- isomers (E: Z = 1.1: 1.0). This crude mixture can be used in the next step. NMR ($CDCl_3$) δ 9.30 (broad s, 0.5H, -NOH of Z-isomer), δ 9.10 (broad s, 0.5H -NOH of E-isomer), δ 5.35 (s, 1H, O-$CH_2$ of E-isomer) δ 5.10 (s, 1H, Z-isomer), δ 4.30 (s, 1H, -$CH_2$-N of Z-isomer), δ 4.17 (s, 1H, -$CH_2$-N of E-isomer), δ 4.8-4.7 (m, 4H) δ 3.6-3.4 (m, 4H).

1-(tert-Butylamino)-3-[(4-morpholino-1,2,5-thiadiazol-3-yl)oxy]-2-propanone oxime, timolone oxime (5b)

In a 250 ml round-bottomed flask fitted with a dropping funnel was placed (4b) (9.89, 33.5 mmol) in THF (147 ml). The solution was cooled in an ice bath, and a solution of tert-butylamine (12.2g, 168 mmol) in THF (20 ml) was added through the dropping funnel during 10 minutes keeping the reaction temperature at -5 to 0 ° C (pH 6-7). The stirring was continued for 1 hour at the same temperature. The solvent was evaporated under reduced pressure at 25 ° C. To the residue was added diluted HCl (2.8 ml conc. HCl) and it was extracted with ethyl acetate. To the aqueous layer separated was added a dil. $NaHCO_3$ solution ($NaHCO_3$ 3.1g) at -5 ° C (pH ~ 6-7). It was extracted with ether to remove some impurities. Small amounts of $NaHCO_3$ (0.1-0.3g) were added to the aqueous layer to make it slightly basic, and the mixture was extracted with ether. This procedure was repeated 4 times (pH was about 8) and another 3 times after raising the pH to about 9 with dil. NaOH solution. The organic extracts were combined, washed with water, dried over anhydrous $MgSO_4$, and concentrated in vacuo. Yield 3.1g (Y = 28%). The crude product was triturated with isopropyl ether to yield 2.8g. This was recrystallized from isopropyl ether to yield 1.8g (Y = 16%) of pure compound.

1-(tert-Butylamino)-3-[(4-morpholino-1,2,5-thidiazol-3-yl)oxy]-2-propanone oxime oxalate, timolone oxime oxalate (6b)

In a 50 ml round bottomed flask was placed a solution of oxalic acid (0.20g, 2.22 mmole) in ether (10 ml). To this solution was added a solution of (5b) (0.43g, 1.3 mmole) in ether. The mixture was stirred at room temperature for 1 hour. The white non-hygroscopic crystals were filtered and dried in vacuo. Yield 0.53g (Y = 97%). M.p. 165-166 ° (dec:). NMR ($CDCL_3$) δ 5.3 (s, 2H), δ 3.9-3.7 (m, 4H), δ 3.6-3.4 (m, 6H), δ 1.1 (s, 9H).
Anal. ($C_{18}H_{25}O_7N_5$), C,H,N.

EXAMPLE 3

5-[3-(tert-Butylamino)-2-(hydroxylimino)propoxy]-3,4-dihydrocarbostyril hydrochloride, carteolone oxime HCl (6c)

5-(3-Chloro-2-hydroxypropoxy)-3,4-dihydrocarbostyril (1c)

In a 250 ml round-bottomed flask fitted with a reflux condenser were placed 5-hydroxycarbostyril (15.0g, 92 mmole), epichlorohydrin (34.2g, 0.37 mole), morpholine (1.5 ml), and dioxane (90 ml). The mixture was refluxed for 16 hours, then it was concentrated in vacuo (20 mm/Hg) at 80-90 ° C. To the residue was added 300 ml of 2N HCl, stirred for 15 minutes, then 0.8-1.0 L of ethyl acetate was added and the mixture was stirred vigorously for 0.5 hour. The organic layer was separated, washed well with water, then with dil. $NaHCO_3$ and was concentrated in vacuo. Yield: 19.9g (85%).

NMR (DMSO-d6) δ 10.3 (s, 1H -NH-), δ 7.25-6.50 (m, 3H, Ph), δ 4.20-3.60 (m, 5H, $OCH_2CHCH_2Cl$), δ 3.00-2.30 (m, 4H, $-CH_2CH_2CO-$).

5-(3-Chloro-2-oxo)propoxy)-3,4-dihydrocarbostyril (3c) was synthesized according to the method described for (3a). NMR (DMSO-d6) δ 10.10 (s, 1H, -NH-), δ 7.20-7.00 (m, 1H, Ph), δ 6.65-6.50 (m, 2H, Ph), δ 4.95 (s, 2H, $OCH_2$), δ 4.70 (s, 2H, $CH_2Cl$), δ 3.0-2.8 (m, 2H, $-C-CH_2CO-$), δ 2.5-2.3 (m, 2H, $-CH_2-C-CO-$).

5-[3-Chloro-2-(hydroxyimino)propoxy]-3,4-dihydrocarbostyril (4c) was synthesized similarly to the method given for (4b). NMR (DMSO-d6) δ 11.88 (s, 0.3H), z- of NOH), δ 11.80 (s, 0.7H, E- of NOH) δ 10.08 (s, 1H, -NH-), δ 7.30-6.50 (m, 3H, Ph), δ 4.93 (s, 1.4H, E- of $OCH_2$) δ 4.73 (s, 0.6H, Z- of $OCH_2$), δ 4.38 (s, 2H, Z & E- of $CH_2Cl$), δ 3.00- δ 2.80 (m, 2H, $C-CH_2-CO-$), δ 2.65-2.40 (m, 2H, $CH_2-C-CO$).

5-[3-(tert-Butylamino)-2-(hydroxylimino)propoxy]-3,4-dihydrocarbostyril, carteolone oxime (5c)

In a 100 ml round-bottomed flask fitted with a dropping funnel were placed (4c) (2.0g, 7.45 mmole) and THF (70 ml). The solution was cooled to 0°C and a solution of tert-butylamine (0.82g, 1.17 ml, 11.2 mmole) in THF was introduced through the dropping funnel. The mixture was stirred under cooling for 2 hours. To the reactive mixture was added a solution of oxalic acid (1.48g, 16.4 mmole) in THF. The precipitate was filtered, triturated with water (600-700 ml) by stirring well for 15 minutes, and it was filtered again. The filtrate was extracted with ethyl acetate several times. The aqueous layer was cooled to 0°C, basified with a dil. $NaHCO_3$ solution ($NaHCO_3$ 0.81g), and was immediately extracted with ethyl acetate. The extract was evaporated *in vacuo* (20 mHg) at 30°C. Yield: 0.63g (28%). Recrystallized from i-propanol, the product was Z-isomer. M.p. 177-180°C (dec.)

NMR (DMSO-$d_6$) δ 11.0PPM(s, 1H), δ 10.1 (s, 1H), δ 7.3-7.1 (m, 1H), δ 6.7-6.5 (m, 2H), δ 4.9 (s, 2H), δ 3.3 (s, 3H contain NH), δ 3.0-2.8 (m, 2H), δ 2.6-2.3 (m, 2H), δ 1.05 (s, 9H).

5-[3-(tert-Butylamino)-2-(hydroxylimino)propoxy]-3,4-dihydrocarbostyril Hydrochloride, carteolone oxime hydrochloride (6c)

The free base (5c) was converted to the hydrochloride salt (6c) in ether with HCl gas. M.p. 167-169°C (dec.).

Anal. ($C_{16}H_{24}O_3N_3Cl$) C,H,N.

EXAMPLE 4

**1-(Isopropylamino)-3-[(4-morpholino-1,2,5-thiadiazol-3-yl)oxy]-2-propanone oxime hydrochloride (6d)**

1-(Isopropylamino)-3-[(4-morpholino-1,2,5-thiadiazol-3-yl)oxy]-2-propanone oxime (5d)

In a 200 ml round-bottomed flask were placed 3-chloro-1-[3-(4-morpholino-1,2,5-thiadiazyloxy)]-2-propanone oxime (4b) (3.53g, 12.1 mmole), isopropylamine (3.56g, 60.3 mmole), and THF (71 ml). The mixture was stirred at room temperture for 2.5 hours. The reaction mixture was concentrated *in vacuo* at room temperature. The residue was triturated with isopropyl ether and precipitated crystals were filtered with suction. The crystals were dissolved in dil. HCl solution. To the solution was added ether and $NaHCO_3$ in small portions under vigorous stirring conditions. The organic layer was washed with water and dried over anhydrous $MgSO_4$, and concentrated *in vacuo*. Yield: 0.42g (Y = 11.6%) from isopropyl ether.

1-(Isopropylamino)-3-[(4-morpholino-1,2,5-thiadiazol-3-yl)oxy]-2-propanone oxime Hydrochloride (6d)

The oxime (4b) (0.25g) was dissolved in ether and ether saturated with HCl was introduced dropwise into the solution. The mixture was stirred for 10 minutes, filtered and dried *in vacuo* overnight. Yield: 89%.

Anal. ($C_{12}H_{22}N_5O_3S$ Cl) C,H,N.

Elemental Analyses

1 - 1-(Isopropylamino)-3-(1-naphthyloxy)-2-propanone oxime hydrochloride, propranolone oxime hydrochloride (6a), $C_{16}H_{21}O_2N_2Cl$ .

| | | | |
|---|---|---|---|
| Calc.: | C, 62.23; | H, 6.85; | N, 9.07 |
| Found: | C, 62.32; | H, 6.89; | N, 9.05. |

2 - 1-(tert-Butylamino)-3-[(4-morpholino-1,2,5-triadiazol-3-yl)oxy]-2-propanone oxime oxalate, timolone oxime oxalate (6b), $C_{15}H_{25}O_7N_5S$ $C_{13}H_{23}O_3N_5S$. $(COOH)_2$.

| | | | |
|---|---|---|---|
| Calc.: | C, 42.95; | H, 6.01; | N, 16.70 |
| Found: | C, 43.00; | H, 6.04; | N, 16.67. |

3 - 5-[3-(tert-Butylamino)-2-(hydroxyimino)propoxy]-3,4-dihydrocarbostyril hydrochloride, carteolone oxime HCl (6c), $C_{16}H_{24}O_3N_3Cl$.

| | | | |
|---|---|---|---|
| Calc.: | C, 56.22; | H, 7.08; | N, 12.30 |
| Found: | C, 56.10; | H, 7.13; | N, 12.21 |

4 - 1-(Isopropylamino)-3-[(4-morpholino-1,2,5-thiadiazol-3-yl)oxy]-2-propanone oxime hydrochloride (6d), $C_{12}H_{22}N_5O_3S$ Cl • 1/3 $H_2O$.

| | | | |
|---|---|---|---|
| Calc.: | C, 40.27; | H, 6.38; | N, 19.57 |
| Found: | C, 40.54; | H, 6.42; | N, 19.46 |

The following non-limiting examples illustrate the pharmacological properties of the compounds of the invention.

EXAMPLE 5

Effect on the Intraocular Pressure (IOP) of Rabbits:

Adult male New Zealand albino rabbits weighing 2.5 - 3.5 kg were used. The animals were kept in individual cages with free access to food and water. Intraocular pressure was measured using a Digilab model 30R pneumatonometer. The pneumatonometer readings were checked at least twice a day using the Digilab calibration verifier. All measurements were obtained from unrestrained, unanesthetized rabbits. One drop of 0.5% propacaine (Ophthetic-Allergan Pharmaceuticals, Inc.) diluted 1:2 with saline was instilled in each eye immediately prior to IOP measurement. Drugs were administered as 1 or 2.5% solution in buffer pH 7.4 or in saline in both eyes of a group of at least four rabbits. Another group of at least three rabbits served as control and was administered the carrier only. IOP was recorded after 30 and 60 minutes and then after 2, 3, 4, 6 and 8 hours following the drug or carrier administration. Values are given as means ± standard error (S.E.) of the mean. The significance of the change was determined using the student's t-test.

The animals were also observed for local action of the drugs on the eyes, e.g., irritation, congestion, redness, lacrimation, etc.

The results are set forth in Tables 1 - 3.

Table 1

Effect of 1% solutions of propranolol·HCl (1a) and propranolone oxime·HCl (6a ) on the IOP (mm/Hg) of rabbits.

| Time after administration | Propranolol HCl (1a) 1% Control | Propranolol HCl (1a) 1% Treated | Propranolol HCl (1a) 1% % change after treatment | Propranolone oxime HCl (6a ) (1%) Control | Propranolone oxime HCl (6a ) (1%) Treated | Propranolone oxime HCl (6a ) (1%) % change after treatment |
|---|---|---|---|---|---|---|
| Zero | 28.8 ± 0.33 | 30.8 ± 0.40 | 0.00 | 29.3 ± 0.50 | 27.8 ± 0.62 | 0.00 |
| 30 min. | 32.0 ± 0.44 | 28.0 ± 0.56 | -9.10* | 28.2 ± 0.65 | 26.9 ± 0.58 | -3.23 |
| 60 min. | 32.7 ± 0.26 | 27.6 ± 0.62 | -10.39* | 29.1 ± 0.60 | 24.0 ± 0.60 | -11.51* |
| 2 hrs. | 31.3 ± 0.31 | 29.1 ± 0.38 | - 5.52 | 27.7 ± 0.57 | 23.4 ± 0.51 | -15.82** |
| 3 hrs. | 30.8 ± 0.32 | 27.4 ± 0.54 | -11.04* | 26.3 ± 0.40 | 23.3 ± 0.35 | -16.18** |
| 4 hrs. | 29.9 ± 0.61 | 28.2 ± 0.48 | -8.44* | 26.8 ± 0.33 | 22.2 ± 0.42 | -20.14** |
| 6 hrs. | 30.8 ± 0.38 | 29.0 ± 0.45 | -5.84 | 28.8 ± 0.52 | 25.8 ± 0.56 | -7.08* |
| 8 hrs. | 30.7 ± 0.26 | 30.2 ± 0.43 | -1.95 | 29.2 ± 0.51 | 27.9 ± 0.62 | +0.50 |

*Significant decrease in I.O.P. ($P<0.05$)

**Highly significant decrease in I.O.P. ($P<0.01$)

Table 2

Effect of 2.5% solutions of propranolol·HCl (1a) and propranolone oxime HCl (6a) on the IOP (mm/Hg) of rabbits.

| | Propranolol·HCl (1a) (2.5%) | | | Propranolone oxime·HCl (6a) (2.5%) | | |
|---|---|---|---|---|---|---|
| Time after Administration | Control | Treated | % change after treatment | Control | Treatment | % change after treatment |
| Zero | 26.6±0.56 | 25.4±0.56 | 0.00 | 25.8±0.55 | 26.0±0.48 | 0.00 |
| 30 min. | 28.0±0.93 | 27.6±0.56 | +8.66* | 27.2±0.82 | 26.5±0.63 | +1.92 |
| 60 min. | 26.6±0.51 | 27.9±0.60 | +9.84* | 26.2±0.63 | 23.4±0.55 | -10.00* |
| 2 hrs. | 24.6±0.19 | 25.7±0.55 | +1.18 | 26.3±0.71 | 22.6±0.43 | -13.08* |
| 3 hrs. | 25.6±0.31 | 25.7±0.39 | +1.18 | 26.7±0.66 | 21.2±0.28 | -18.46* |
| 4 hrs. | 25.2±0.64 | 25.6±0.46 | +0.79 | 26.8±0.34 | 20.4±0.34 | -21.54** |
| 6 hrs. | 26.4±0.35 | 25.4±0.54 | 0.00 | 25.9±0.54 | 23.6±0.48 | -9.23* |
| 8 hrs. | 26.0±0.52 | 25.2±0.68 | -0.79 | 26.0±0.47 | 25.8±0.65 | -0.77 |

*Significant change ($P<0.05$)

**Highly significant change ($P<0.01$)

Table 3

Effect of 1% of solutions of timolol maleate (1b) and timolone oxime oxalate (6b) on the IOP (mm/Hg) of rabbits

| Time after administration | Timolol maleate (1b) 1% Control | Drug-treated | %Change after treatment | Timolone oxime oxlate (6b) 1% Control | Drug-treated | % change after treatment |
|---|---|---|---|---|---|---|
| 0 | 28.72±0.54 | 29.19±0.78 | 0.00 | 28.72±0.54 | 28.44±.63 | 0.00 |
| 30 min. | 26.62±0.59 | 26.00±1.23 | -10.93* | 26.62±0.59 | 27.75±0.68 | -2.43 |
| 1 hr. | 28.10±0.85 | 27.25±1.12 | 6.65 | 28.10±0.85 | 24.30±0.63 | -14.56** |
| 2 hr. | 27.02±0.92 | 27.21±0.58 | 6.78 | 27.02±0.92 | 24.00±0.44 | -15.61** |
| 3 hr. | 25.95±0.44 | 25.00±0.87 | 14.35** | 25.95±0.44 | 25.13±0.96 | -11.64** |
| 4 hr. | 26.45±0.57 | 24.88±1.00 | 14.77** | 26.45±0.57 | 26.93±0.48 | -5.31 |
| 5 hr. | 27.09±0.55 | 24.25±1.18 | 16.92** | 27.09±0.55 | 26.88±0.52 | -5.49 |
| 6 hr. | 27.88±0.56 | 25.58±0.85 | 12.37** | 27.88±0.56 | 28.78±0.91 | +1.20 |
| 8 hr. | 27.67±0.63 | 26.33±0.40 | 9.80* | 27.67±0.63 | 26.33±0.40 | -7.42 |

*Significant change ($P<0.05$)

** Highly significant change ($P<0.01$).

These results reveal that the ketoxime analogs of both propranolol and timolol display a certain degree of ocular hypotensive activity. Propranolone ketoxime (6a) has shown the highest activity at both tested concentration levels, 1 and 2.5%. This activity was much more pronounced and prolonged than that of propranolol itself administered at the same dose levels (Tables 1 and 2). In addition, the ketoxime (6a) was completely devoid of the ocular irritation which always accompanied propranolol administration at both dose levels. This irritant activity might have contributed to the reduced action of propranolol on the IOP at the 1% dose level and, also, might have completely masked its ocular hypotensive activity at the 2.5% dose level. Timolone ketoxime (6b) has also shown a significant ocular hypotensive activity which was faster in its onset and shorter in its duration than timolol (1b) itself (Table 3). On the other hand, the other ketoxime precursors, the ones for the N-isopropyl analog (6d) of (6b) and (6c) for carteolol, showed low activity at the dose levels used but showed some $\beta$-antagonist activity.

EXAMPLE 6

Effect on resting heart rate and on isoprenaline-induced tachycardia in rats:

Male Sprague-Dawley rats weighing 150-250 g were used. Each animal was anesthetized with sodium pentobarbital (50 mg/kg) and the jugular vein was cannulated with PE50 tubing. This cannula was subcutaneously threaded around the neck and exteriorized dorsally. The cannula was filled with heparin solution (1000/μl) and sealed with a solid 22-gauge stylet. Animals were housed in individual stainless steel cages and at least 24 hours were allowed for recovery from the surgery. Food and water were provided ad libitum. On the day of the experiment, the heart rate of each rat was monitored with a plethsmograph and the data recorded on a Physioscribe II recorder. One hour was allowed as an equilibration period before any drugs were administered. Drugs were dissolved in normal saline as 0.3% solution and were administered intravenously at a dose of 6 mg/kg. The resting heart rate was then recorded after 1, 3, 5, 10 and 15 minutes following i.v. injection. Isoprenaline (Isoproterenol bitartarate), was then administered subcutaneously at a dose of 50 μg/kg and the heart rate was recorded for 3, 5, 10, 15, 20, 30, 45 and 60 minutes after administration. A control group of seven animals was intravenously administered saline solution and was treated exactly in the same manner as the drug-treated groups.

The significance of the difference between the effect of saline solution and the drugs under investigation on the resting heart rate and on isoprenaline tachycardia was analyzed using the student's "t" test. Values are given as mean ± S.E. of the mean. The results are depicted in Figure 1 which depicts the mean change in heart rate over time for (□) propranolol HCl (1a), (▲) timolol maleate (1b), (●) carteolol HCl (1c), (○) propranolone ketoxime HC1 (6a), (▽) timolone ketoxime oxalate (6b), (△) N-isopropyl timolone ketoxime HCl (6d), (■) carteolone oxime HCl (6c) and (- - -) saline solution.

In another set of experiments the effect of the oral administration of propranolol Hcl (1a) and propranolone oxime HC1 (6a) in doses of 25, 50 and 100 mg/kg on the resting heart rate and isoprenalinetachycardia was evaluated in rats. Drugs were administered to groups of 5 rats using a stomach tube and the heart rate was recorded for 1 hour. Then isoprenaline (50 μg/kg, s.c.) was administered and the heart rate was recorded after 3, 5, 10, 15, 20, 30, 45 and 60 minutes following administration. A control group of 5 rats was treated exactly in the same manner after the oral administration of the appropriate volume of saline solution.

The resting heart rate portions of these studies revealed that most of the tested ketoximes exhibit a negative chronotropic action in rats. Again, the ketoximes of propranol (6a) and timolol (6b) have shown the highest activity in this test, whereas carteolone ketoxime (6c) and the oxime (6d) were less active. It should be also noted that in this test carteolol (1c) itself has shown the lowest activity on the heart rate of rats.

When the potential β-adrenergic antagonist activity of the ketoxime precursors of propranolol, timolol and carteolol was assessed against isoprenaline-tachycardia using the parent compounds as obvious reference drugs as described above, results were in agreement with the findings of the studies on the effect on the IOP and resting heart rate. Thus, the ketoxime precursors of propranolol (6a) and timolol (6b) were the most effective whereas (6c) and (6d) were the least active. See Figure 1.

These results indicate that at least two of the investigated ketoxime precursors (6a and 6b) have an antiglaucoma activity which is probably linked to their β-adrenergic antagonistic properties. Yet, whether these properties are due to an inherent intrinsic activity of the ketoximes themselves or are the result of their active biological conversion to their parent drugs needed to be verified. For this reason the in vivo disposition of the different ketoximes and their parent β-blockers in the different ocular tissues was studied in rabbits.

EXAMPLE 7

In vivo distribution - metabolism studies:

A. In ocular tissues of rabbits:

Adult male New Zealand albino rabbits weighing 2.5-3.5 kg were used. Standard doses of 100 μl of 1% solution of the drugs in saline solution were administered topically to both eyes of each rabbit. After appropriate time intervals (30, 60 and 120 minutes), the animals were sacrificed. Aqueous humor was obtained by making a single puncture at the limbus using a 25 g x 5/8" needle attached to 1 c.c. syringe. Then the cornea and the iris-ciliary body were isolated. The tissues were pooled and homogenized using a Tekmar SDT tissuemizer in ice cold perchloric acid (0.05 M) which contained 0.05% sodium metabisulfite

as antioxidant. Samples were then rehomogenized in $CH_3OH$ to prepare 10% homogenates, transferred to micro-filters and centrifuged for 20 minutes at 10000 r/minute to precipitate proteins. Aqueous humor was analyzed as such without any further dilution. Aliquots of 5-20 µl of the 10% tissue homogenate samples were analyzed by HPLC. Quantitation was done by using a calibration curve obtained by the addition of known amounts of the compound to aqueous humor, iris-ciliary body or cornea obtained from a control rabbit after topical administration of saline solution.

B. In rat's blood:

A group of seven adult male Sprague-Dawley rats weighing 150-250 g was used. Animals were intrajugularly injected with propranolone oxime (6a) at a dose of 6 mg/kg. After 1, 3, 5, 20, 40 and 60 minutes, one ml of blood was withdrawn from the jugular vein and dropped immediately into a tared tube containing 1 ml of ice-cold acetonitrile. The tubes were vigorously shaken, centrifuged, decanted and analyzed for propranolol (1a) and propranolone oxime (5a) by HPLC. Quantitation was done by using a calibration curve obtained by addition of known amounts of propranolol oxime HCl (6a) to blood obtained from a control rat pretreated with saline solution.

The results of the ocular tissue tests are set forth in Tables 4 and 5.

Table 4

Tissue Concentration[a] of propranolol (1a) and propranolone oxime (5a) at various time intervals following topical administration of propranolone oxime.HCl ( 6a ) (1% solution)

| | Concentration of propranolone oxime (5a) (mcg/g tissue) | | | Concentration of Propranolol (1a) (mcg/g tissue) | | |
|---|---|---|---|---|---|---|
| Tissue/Time | 30 min. | 60 min. | 120 min. | 30 min. | 60 min. | 120 min. |
| Cornea | 23.75±4.91 | 16.40±5.80 | 0.00±0.00 | 1.68±0.75 | 1.14±0.29 | 1.14±0.22 |
| Iris-Ciliary | 7.79±1.10 | 0.00±0.00 | 0.00±0.00 | 2.11±0.29 | 1.79±0.20 | 0.43±0.11 |
| Aqueous Humor | 0.82±0.09 | 0.80±0.06 | 0.00±0.00 | 0.04±0.02 | 0.71±0.11 | 0.00±0.00 |

[a]Figures represent the mean ± S.E. of the mean of at least 4 rabbits.

Table 5

| Tissue Concentration[a] of propranolol at various timeintervals following topical administration of propranolol.HCl (1a)(1% solution). | | | |
|---|---|---|---|
| | Concentration of propranolol (mcg/g tissue) | | |
| Tissue/Time | 30 min. | 60 min. | 120 min. |
| Cornea | 47.10±5.57 | 14.54±2.97 | 0.00±0.00 |
| Iris-ciliary body | 8.05±1.47 | 0.00±0.00 | 0.00±0.00 |
| Aqueous humor | 1.28±0.19 | 0.26±0.08 | 0.00±0.00 |

[a]Figures represent the mean ± S.E. of the mean of four rabbits.

The results of the ocular tissue studies show that propranolol (1a) could be detected in measurable concentrations in the different eye compartments for the first two hours following the topical administration of its ketoxime precursor (6a) at its effective ocular hypotensive dose level (1%) (Table 4). On the other hand, propranolol could not be detected in any of the tested eye tissues two hours after its ocular application (Table 5), and it had completely disappeared from the iris-ciliary body which is supposed to be the site of its ocular hypotensive action, one hour after administration. These results might explain the shorter duration of propranolol action on the IOP relative to that of its ketoxime precursor. In addition, these results might also suggest that the ocular hypotensive activity of the oxime is most probably due to its active conversion to propranolol in situ in the ocular tissues of rabbits. This is also supported by the finding that following the ophthalmic administration of the other ketoxime precursors (6b and 6c) of timolol and carteolol, respectively, at the low dose level used, the parent $\beta$-adrenergic antagonists in any of the eye compartments could not be found. This would suggest that either the ketone formed or the reduced form, the active $\beta$-blocker, is disposed of so fast that it cannot be detected, or that the ketones are not such good substrates for the reductase enzyme as the propanolone ketoxime.

The studies in ocular tissue and blood revealed that the metabolic pathway of the oxime in the blood is quite different from that in ocular tissues. Thus, propranolol was not detected in rat's blood following the i.v. administration of the oxime and, instead, another more polar compound was detected. However, 5 minutes after injection even this compound had totally disappeared. The oxime itself appeared to have a very fast metabolism in blood (Fig. 2). The $t1_{/2}$ in blood was equivalent to 7.64 ± 0.55 minutes and one hour after i.v. administration the oxime had completely disappeared from the blood.

While these results would suggest that the propranolol formed in situ in the iris-ciliary body is responsible for the IOP reduction observed, the ketoxime itself might have intrinsic activity.

Based on the previous observation of the necessity to convert adrenalone to lipophilic esters to be reduced, one could expect that the lipophilic propranolone is easily reduced, while the ketones derived from timolol and carteolol, being less lipophilic (heterocyclic substitution of naphthalene), are not reduced that extensively. The N-isopropyl analog (6d) of timolol was synthesized and tested in order to assess the importance of the N-alkyl function. Propranolol contains an i-propyl group, like 6d, but 6d was still found inactive. The difference in the behavior of the 6a vs. 6b-d thus might be due to the difference in the Ar-group which appears to determine the substrate properties necessary to bind to the reductase enzyme. This hypothesis is supported by the relative HPLC retention times of the free bases 5a-5d which were 12.86, 7.22, 3.10, 6.10 for 5a, 5b, 5c and 5d, respectively, indicating that 5a is by far the most lipophilic.

The following example illustrates the HPLC analytical method used to obtain these results.

EXAMPLE 8

ANALYTICAL METHOD

A high pressure liquid chromatography (HPLC) method was developed for the assay of the $\beta$-blockers and their ketoxime analogs in biological fluids. The chromatographic analysis was performed on a system consisting of Beckman Model 112 solvent delivery system, Model 340 Injector, and Waters Model 481 variable wave length LC spectrophotometer. An ASI reverse phase chrompack $C_{18}$ column, operated at ambient temperature, was used for all separations. The mobile phase used for separation of propranolol (1a) and propranolone oxime (5a) consisted of water, 1-heptane sulfonic acid, 0.1M acetic acid, 0.1M triethanolamine and methanol (90, 1g, 100, 799). With a flow rate of 1.5 ml/minute, the two compounds

showed retention times of 2.44 and 3.21 minutes for propranolone oxime and propranolol, respectively. The mobile phase used for separation of carteolol (1c), carteolone oxime (5c), timolol (1b), timolone oxime (5b) and timolone ispropyl oxime (5d) consisted of water, 1-heptane sulfonic acid, 0.1M acetic acid, tetrahydrofuran, 0.1M triethanolamine and methanol (430, 2, 40, 30, 100 and 398). With a flow rate of 1.5 ml/minute, the retention times for these compounds were 3.10, 3.54, 6.10, 7.22 and 9.15 minutes for carteolone oxime (5c), carteolol (1c), timolone isopropyl oxime (5d), timolone oxime (5b) and timolol (1b), respectively.

The results are depicted in Figure 2 which is a plot of blood levels (μg/ml) vs. time of 5a after administration of 6a at a dose level of 6 mg/kg to rats.

It is believed that the compounds of the invention are converted to their parent β-blockers according to the following scheme A which shows the conversion of 5a to 1a. Similar conversions would follow scheme B.

The hydrolytically sensitive precursors of the present invention comprise effective chemical delivery systems (CDS) for the β-blockers and intraocular pressure reducing agents.

SCHEME A

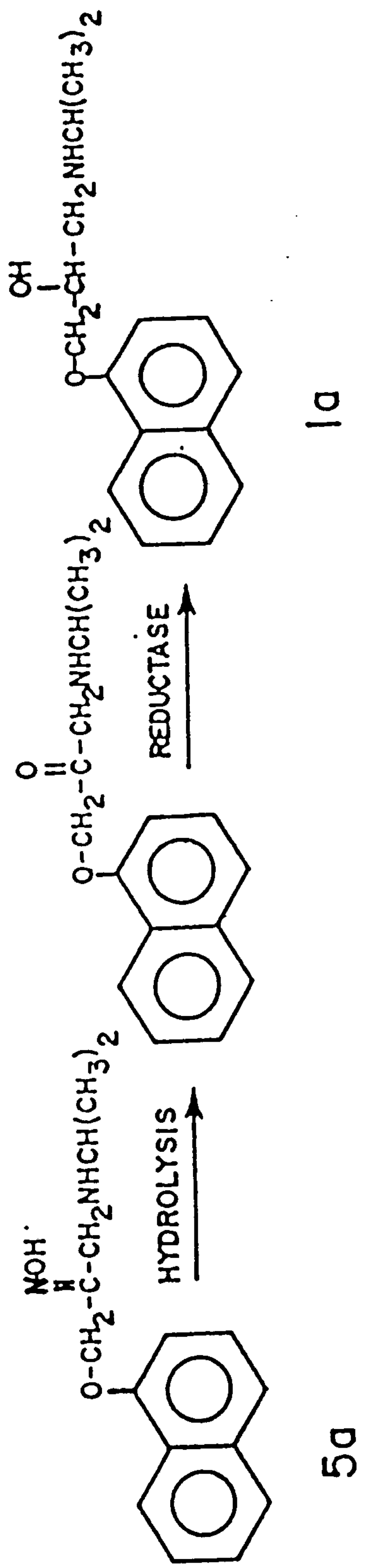


SCHEME B

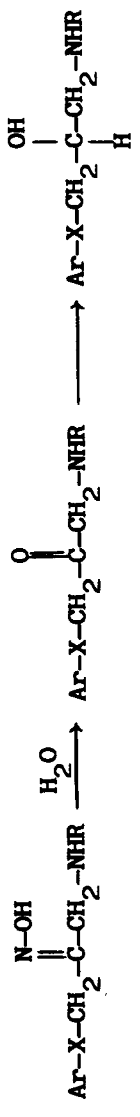


The compounds of the invention may be administered to animals in need thereof by instilling solutions thereof into the eye or via oral tablets, capsules, etc., or any other convenient route of administration at dosages of from about 0.001 to about 20 mg/kg.

The compounds may be formulated with any conventional pharmaceutically acceptable carrier, such as those utilized for the parent amino-alcohol $\beta$-blockers.

## Claims

### Claims for the following Contracting States : AT, BE, CH, DE, FR, GB, IT, LI, LU, NL, SE

**1.** A compound having the formula

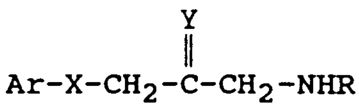 (I)

or a pharmaceutically acceptable acid addition salt thereof, wherein -X- is -O-, $-CH_2-$ or -; =Y is a derivatized keto group which is hydrolyzable or enzymatically convertible to a keto group; R is alkyl having 1 to 12 carbon atoms or aralkyl having from 7 to 20 carbon atoms, the aralkyl group optionally bearing methoxy substituents on the aryl portion thereof; and Ar is the 3-aromatic or heterocyclic residue of a 1-alkylamino-2-propanol having an optionally substituted aromatic or heterocyclic substituent at the 3-position and having β-adrenergic blocking properties.

**2.** A compound or salt according to Claim 1, wherein =Y is $=N-OR_1$, $=N-NH_2$, $=N-NR_1R_2$,

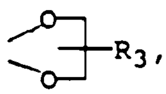 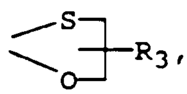 or 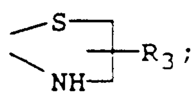

$R_1$ and $R_2$ may be the same or different and are H or alkyl having from 1 to 8 carbon atoms; and $R_3$ is $R_1$, $-COOR_1$ or $-CON(R_1)_2$ wherein $R_1$ is defined as above.

**3.** A compound or salt according to Claim 2, wherein =Y is $=NOR_1$ wherein $R_1$ is H or alkyl having from 1 to 8 carbon atoms.

**4.** A compound or salt according to claim 3, wherein =Y is =NOH.

**5.** A compound or salt according to any one of the preceding claims, wherein -X- is -O-.

**6.** A compound or salt according to any one of Claims 1-5, wherein R is isopropyl or t-butyl.

**7.** A compound or salt according to any one of Claims 1-6, wherein Ar is the 3-aromatic or heterocyclic residue of propanolol, timolol, carteolol, alprenolol, atenolol, befunolol, betaxolol, bevantolol, bufuralol, bunitrolol, bupranolol , celiprolol, cetamolol, labetolol, levobunolol, mepindolol, metipranolol, metoprolol, moprolol, nadolol, oxprenolol, penbutolol, pindolol or toliprolol.

**8.** A compound or salt according to any one of Claims 1-6, wherein Ar is

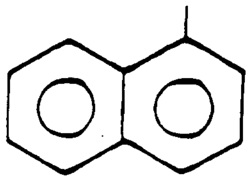 ,

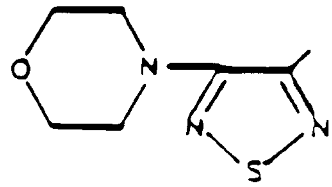 ,

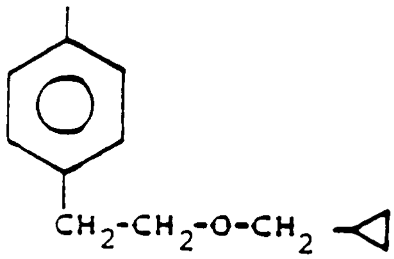 ,

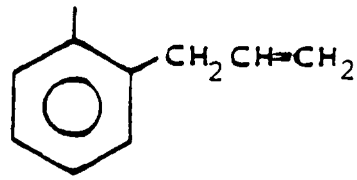 ,

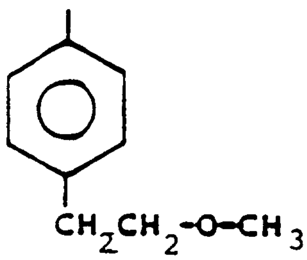 ,

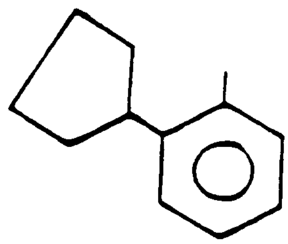 ,

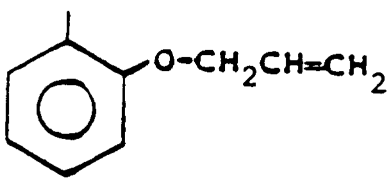 or 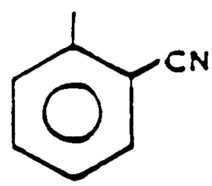 .

**9.** The compound of Claim 1 having the formula

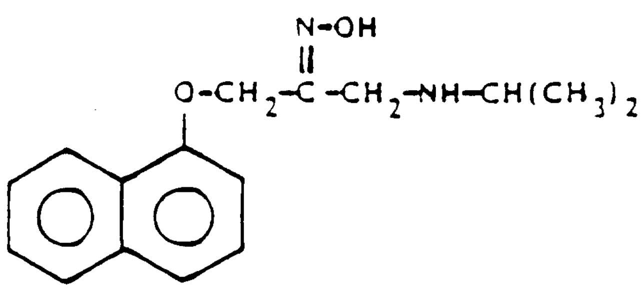

or a pharmaceutically acceptable acid addition salt thereof.

**10.** The compound of Claim 1 having the formula

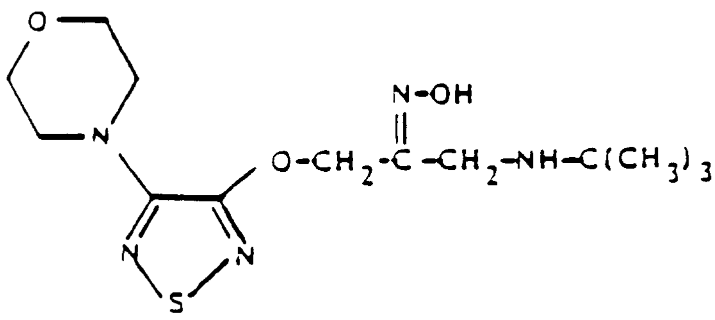

or a pharmaceutically acceptable acid addition salt thereof.

**11.** The compound of Claim 1 having the formula

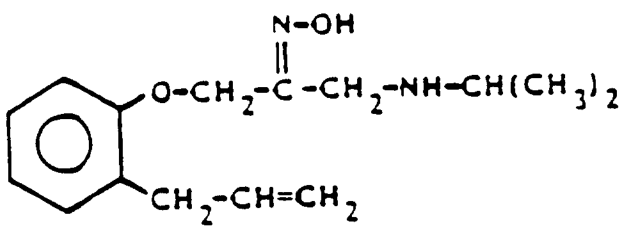

or a pharmaceutically acceptable acid addition salt thereof.

**12.** The compound of Claim 1 having the formula

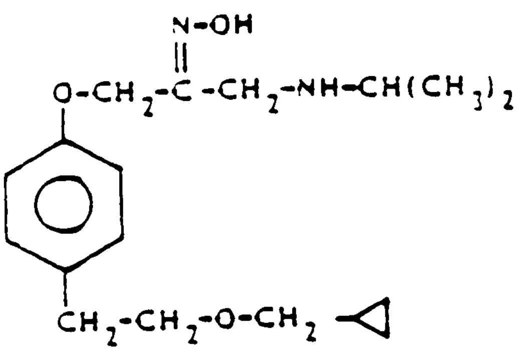

or a pharmaceutically acceptable acid addition salt thereof.

**13.** The compound of Claim 1 having the formula

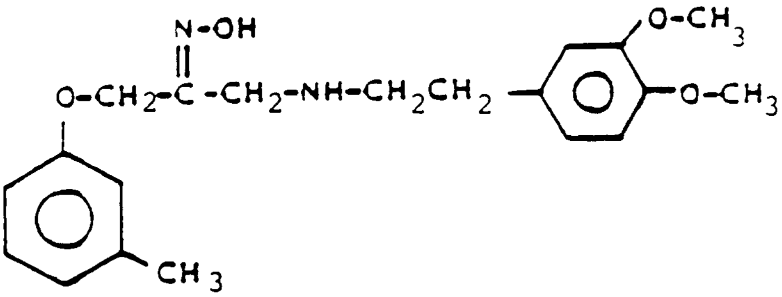

or a pharmaceutically acceptable acid addition salt thereof.

**14.** The compound of Claim 1 having the formula

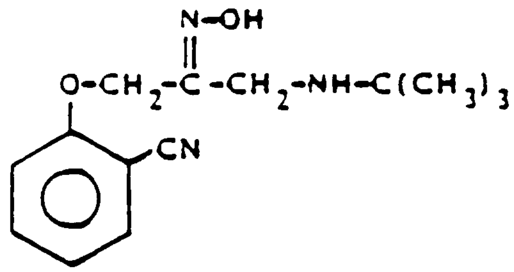

or a pharmaceutically acceptable acid addition salt thereof.

**15.** The compound of Claim 1 having the formula

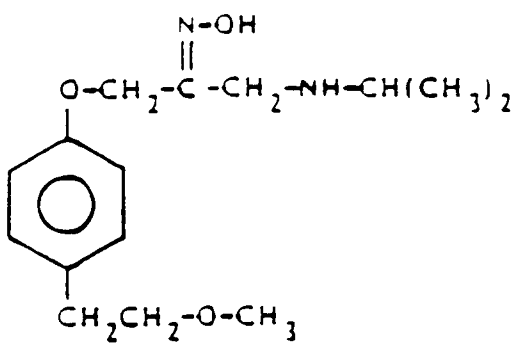

or a pharmaceutically acceptable acid addition salt thereof.

**16.** The compound of Claim 1 having the formula

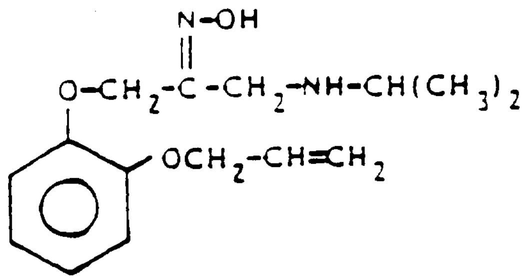

or a pharmaceutically acceptable acid addition salt thereof.

**17.** The compound of Claim 1 having the formula

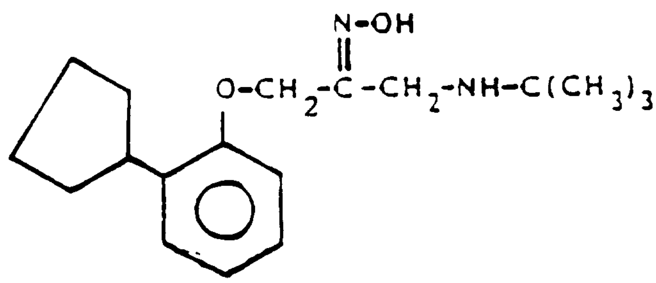

or a pharmaceutically acceptable acid addition salt thereof.

**18.** The compound of Claim 1 having the formula

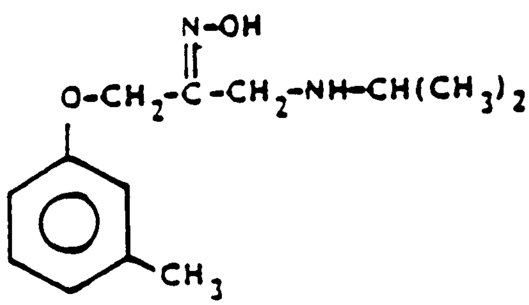

or a pharmaceutically acceptable acid addition salt thereof.

**19.** The compound of Claim 1 having the formula

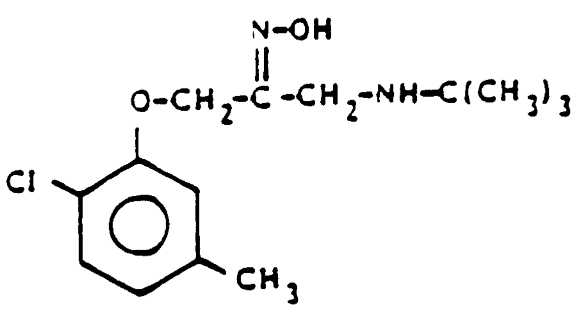

or a pharmaceutically acceptable acid addition salt thereof.

**20.** Use of the compound of any one of claims 1 to 19 or a pharmaceutically acceptable acid addition salt thereof in the preparation of a medicament having a $\beta$-adrenergic blocking effect.

**21.** A pharmaceutical composition of matter, in unit dosage form, for use in eliciting a $\beta$-adrenergic blocking response in a warm-blooded animal, said composition comprising an effective $\beta$-adrenergic blocking amount of a compound as claimed in any one of claims 1 to 19 or a pharmaceutically acceptable acid addition salt thereof and a non-toxic pharmaceutically acceptable carrier therefor.

**22.** Use of a compound having the formula

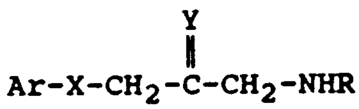 (I)

or a pharmaceutically acceptable acid addition salt thereof, wherein -X- is -O-, $-CH_2-$ or -; =Y is =O or a derivatized keto group which is hydrolyzable or enzymatically convertible to a keto group; R is alkyl having 1 to 12 carbon atoms or aralkyl having from 7 to 20 carbon atoms, the aralkyl group optionally bearing methoxy substituents on the aryl portion thereof; and Ar is the 3-aromatic or heterocyclic residue of a 1-alkylamino-2-propanol having an optionally substituted aromatic or heterocyclic substituent at the 3-position and having $\beta$-adrenergic blocking properties; in the preparation of a medicament having an intraocular pressure lowering effect, especially for use in the treatment of glaucoma.

**23.** Use according to Claim 22, wherein =Y is $=N\text{-}OR_1$, $=N\text{-}NH_2$, $=N\text{-}NR_1R_2$,

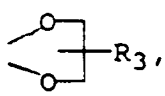 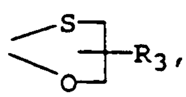 or 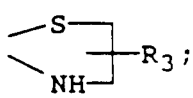

$R_1$ and $R_2$ may be the same or different and are H or alkyl having from 1 to 8 carbon atoms; and $R_3$ is $R_1$, $-COOR_1$ or $-CON(R_1)_2$ wherein $R_1$ is defined as above.

**24.** Use according to Claim 23, wherein =Y is $=NOR_1$ wherein $R_1$ is H or alkyl having from 1 to 8 carbon atoms.

**25.** Use according to any one of Claims 22-24, wherein Ar is the 3-aromatic or heterocyclic residue of propranolol, timolol, carteolol, alprenolol, atenolol, befunolol, betaxolol, bevantolol, bufuralol, bunitrolol, bupranolol, celiprolol, cetamolol, labetolol, levobunolol, mepindolol, metipranolol, metoprolol, moprolol, nadolol, oxprenolol, penbutolol, pindolol or toliprolol.

**26.** Use according to Claim 22, wherein the compound is selected from the group consisting of

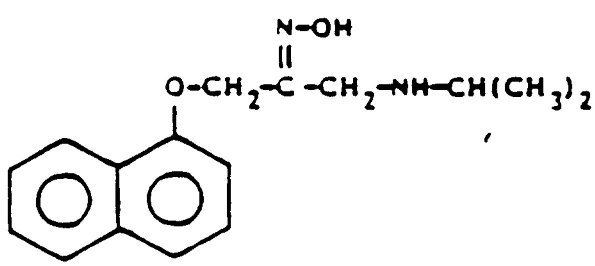

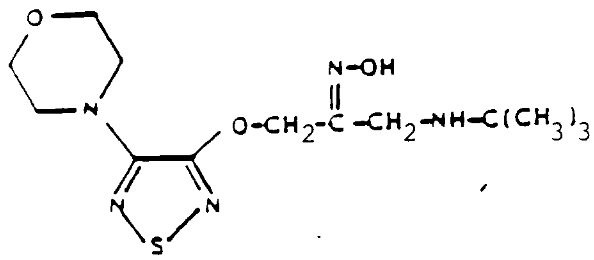

,

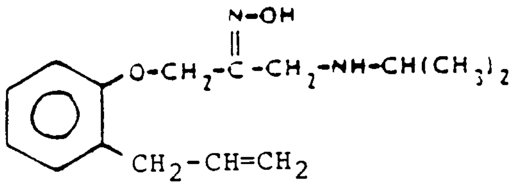

,

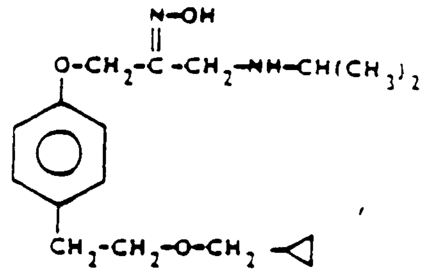

,

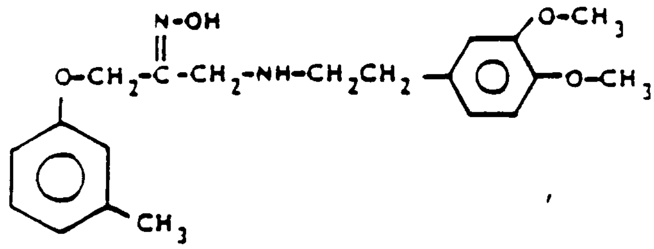

,

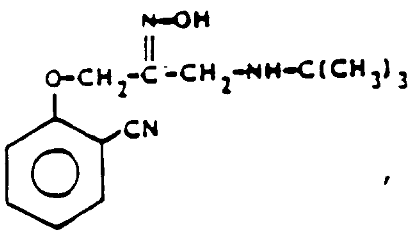

,

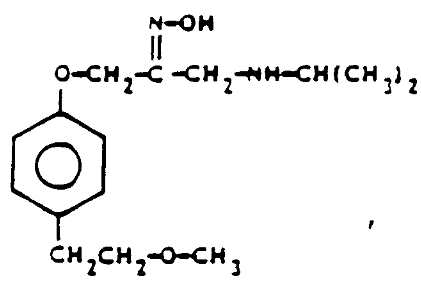

,

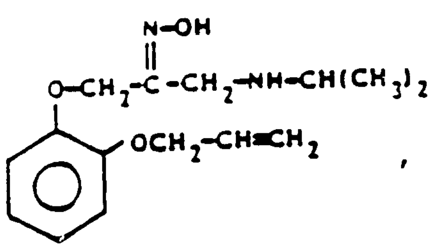

,

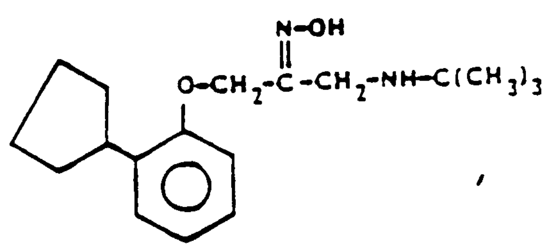

,

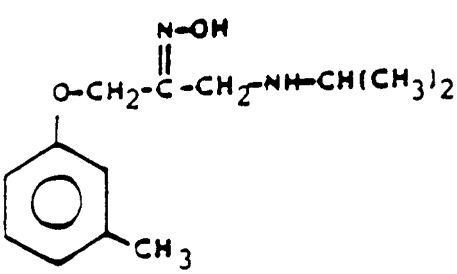

and

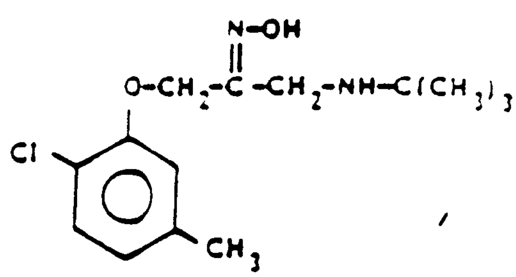

,

and the pharmaceutically acceptable acid addition salts thereof.

**27.** An ophthalmic pharmaceutical composition of matter, in unit dosage form, for use in the lowering of intraocular pressure in a warm-blooded animal, especially in the treatment of glaucoma, said composition comprising an effective intraocular pressure reducing amount of a compound having the formula

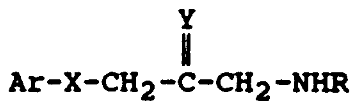 (I)

or a pharmaceutically acceptable acid addition salt thereof as defined in claim 22 and a non-toxic ophthalmically acceptable carrier therefor.

**28.** A composition according to Claim 27, wherein =Y is =N-OR$_1$, =N-NH$_2$, =N-NR$_1$R$_2$,

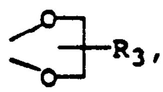, 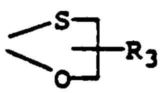, or 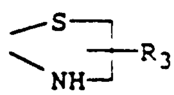;

R$_1$ and R$_2$ may be the same or different and are H or alkyl having from 1 to 8 carbon atoms; and R$_3$ is R$_1$, -COOR$_1$ or -CON(R$_1$)$_2$ wherein R$_1$ is defined as above.

**29.** A composition according to Claim 28, wherein =Y is =NOR$_1$ wherein R$_1$ is H or alkyl having from 1 to 8 carbon atoms.

**30.** A composition according to any one of Claims 27-29, wherein Ar is the 3-aromatic or heterocyclic residue of propranolol, timolol, carteolol, alprenolol, atenolol, befunolol, betaxolol, bevantolol, bufuralol, bunitrolol, bupranolol, celiprolol, cetamolol, labetolol, levobunolol, mepindolol, metipranolol, metoprolol, moprolol, nadolol, oxprenolol, penbutolol, pindolol or toliprolol.

**31.** A composition according to Claim 27, comprising an effective intraocular pressure reducing amount of a compound having the formula

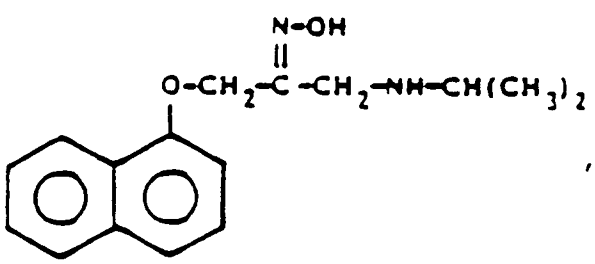,

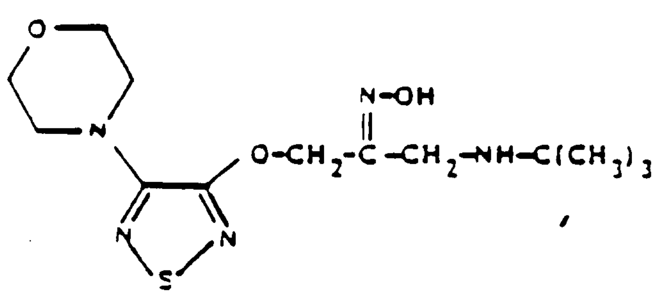,

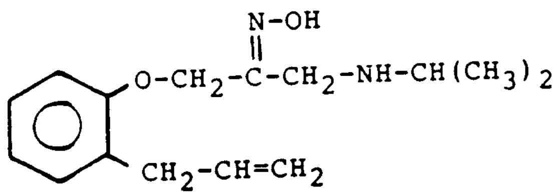

,

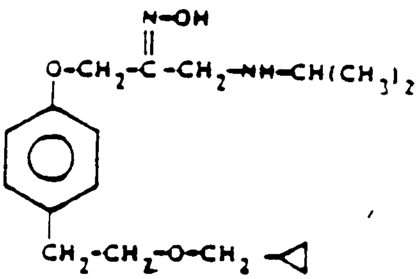

,

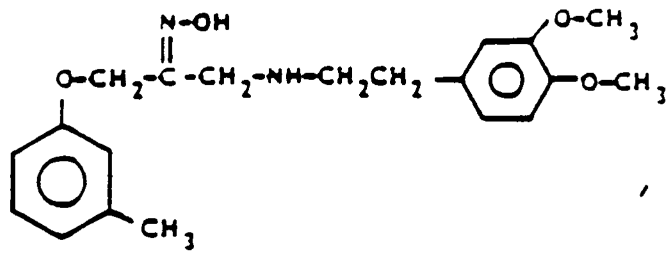

,

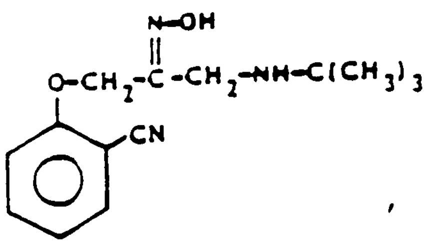

,

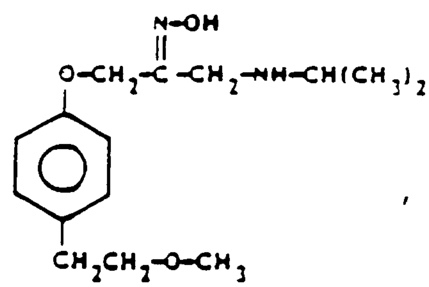

,

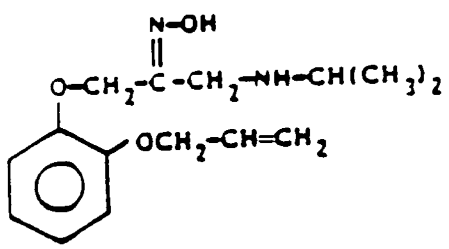

,

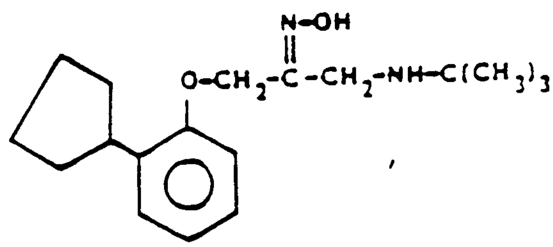

,

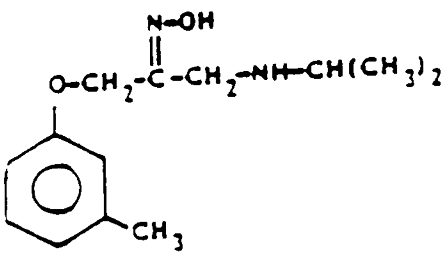

or

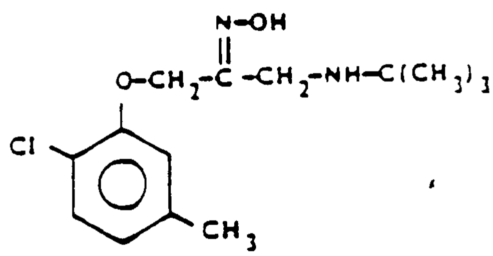

,

or a pharmaceutically acceptable acid addition salt thereof.

**32.** The compounds of claims 1 to 19 for use in therapy.

**Claims for the following Contracting States : ES, GR**

**1.** A process of preparing a pharmaceutical composition of matter for use in eliciting a $\beta$-adrenergic blocking response in a warm-blooded animal, said process comprising mixing a compound having the formula

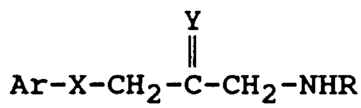 (I)

or a pharmaceutically acceptable acid addition salt thereof, wherein -X- is -O-, $-CH_2-$ or -; =Y is a derivatized keto group which is hydrolyzable or enzymatically convertible to a keto group; R is alkyl having 1 to 12 carbon atoms or aralkyl having from 7 to 20 carbon atoms, the aralkyl group optionally bearing methoxy substituents on the aryl portion thereof; and Ar is the 3-aromatic or heterocyclic residue of a 1-alkylamino-2-propanol having an optionally substituted aromatic or heterocyclic substituent at the 3-position and having $\beta$-adrenergic blocking properties with a non-toxic pharmaceutically acceptable carrier therefor and dividing the resulting mixture into unit dosage forms, each containing an effective $\beta$-adrenergic blocking amount of the compound of the formula I or a salt thereof.

**2.** A process according to claim 1, wherein =Y is $=N\text{-}OR_1$, $=N\text{-}NH_2$, $=N\text{-}NR_1R_2$,

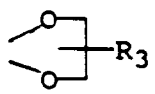 , 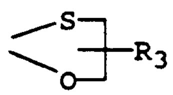 , or 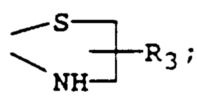 ;

$R_1$ and $R_2$ may be the same or different and are H or alkyl having from 1 to 8 carbon atoms; and $R_3$ is $R_1$, $-COOR_1$ or $-CON(R_1)_2$ wherein $R_1$ is defined as above.

**3.** A process according to claim 2, wherein =Y is $=NOR_1$ wherein $R_1$ is H or alkyl having from 1 to 8 carbon atoms.

**4.** A process according to claim 3, wherein =Y is =NOH.

**5.** A process according to any one the preceding claims, wherein -X- is -O-.

**6.** A process according to any one of claims 1-5, wherein R is isopropyl or t-butyl.

**7.** A process according to any one of claims 1-6, wherein Ar is the 3-aromatic or heterocyclic residue of propanolol, timolol, carteolol, alprenolol, atenolol, befunolol, betaxolol, bevantolol, bufuralol, bunitrolol, bupranolol , celiprolol, cetamolol, labetolol, levobunolol, mepindolol, metipranolol, metoprolol, moprolol, nadolol, oxprenolol, penbutolol, pindolol or toliprolol.

**8.** A process according to any one of claims 1-6, wherein Ar is

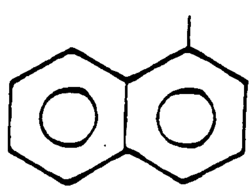 ,

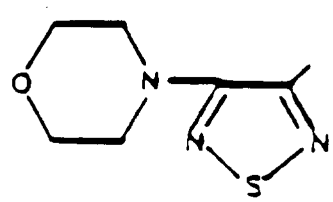 ,

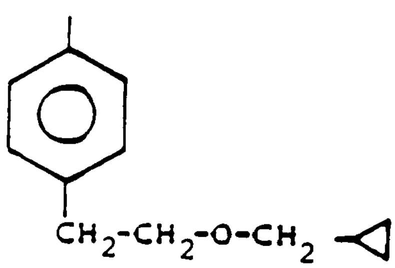 ,

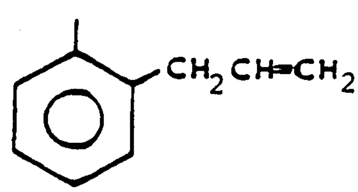 ,

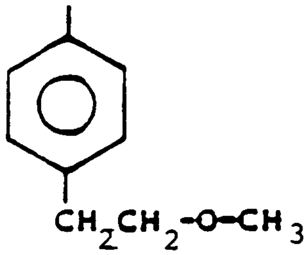 , 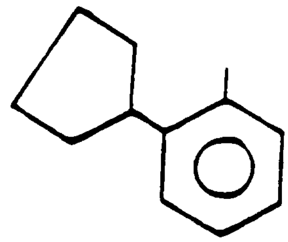 ,

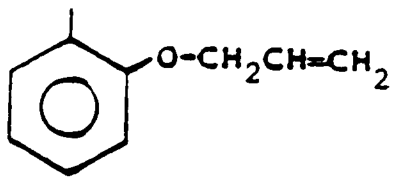 or 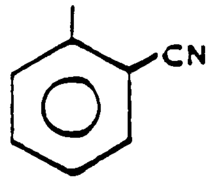 .

**9.** A process according to claim 1, wherein the compound of the general formula I is:

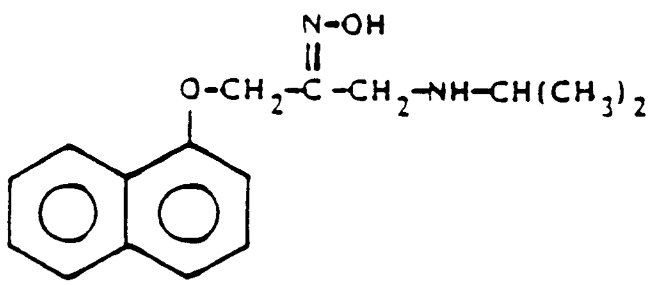

or a pharmaceutically acceptable acid addition salt thereof.

**10.** A process according to claim 1, wherein the compound of the general formula I is:

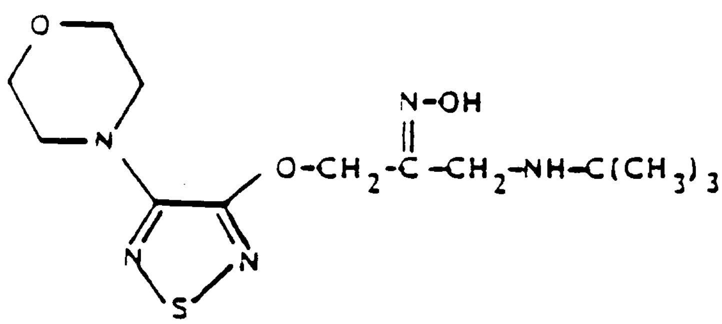

or a pharmaceutically acceptable acid addition salt thereof.

**11.** A process according to claim 1, wherein the compound of the general formula I is:

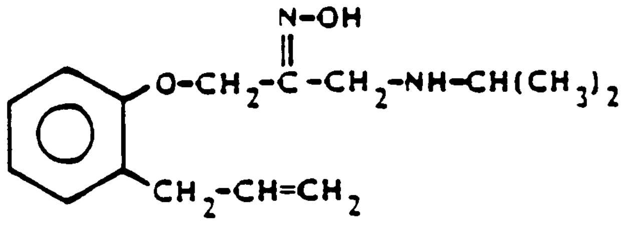

or a pharmaceutically acceptable acid addition salt thereof.

**12.** A process according to claim 1, wherein the compound of the general formula I is:

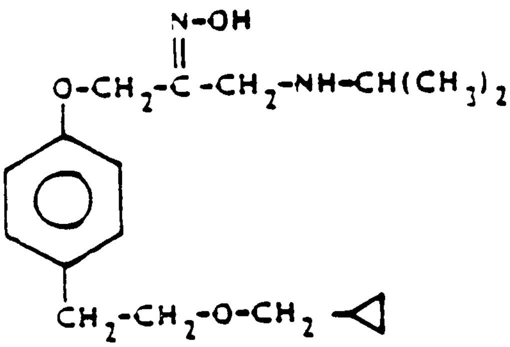

or a pharmaceutically acceptable acid addition salt thereof.

**13.** A process according to claim 1, wherein the compound of the general formula I is:

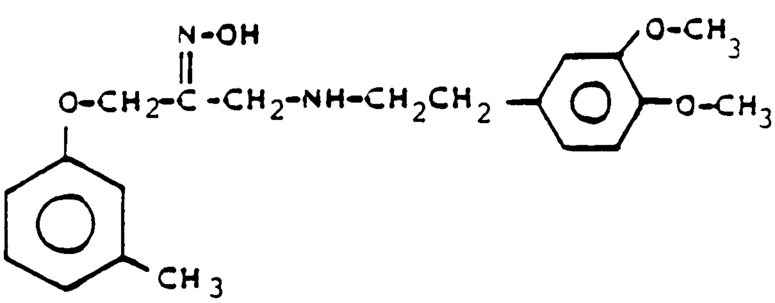

or a pharmaceutically acceptable acid addition salt thereof.

**14.** A process according to claim 1, wherein the compound of the general formula I is:

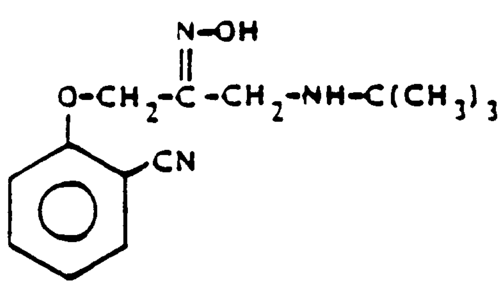

or a pharmaceutically acceptable acid addition salt thereof.

**15.** A process according to claim 1, wherein the compound of the general formula I is:

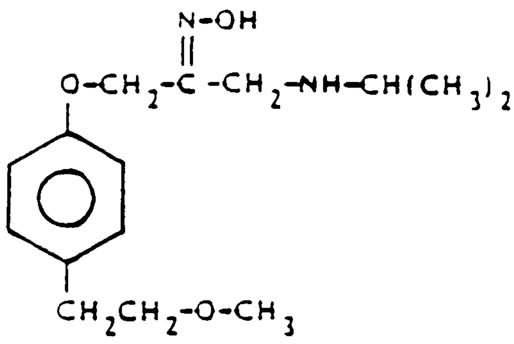

or a pharmaceutically acceptable acid addition salt thereof.

**16.** A process according to claim 1, wherein the compound of the general formula I is:

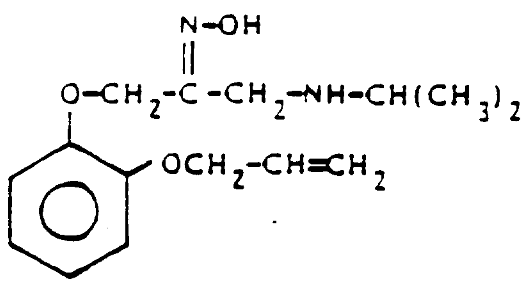

or a pharmaceutically acceptable acid addition salt thereof.

**17.** A process according to claim 1, wherein the compound of the general formula I is:

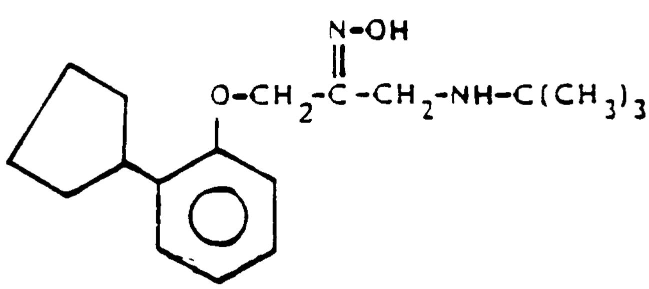

or a pharmaceutically acceptable acid addition salt thereof.

**18.** A process according to claim 1, wherein the compound of the general formula I is:

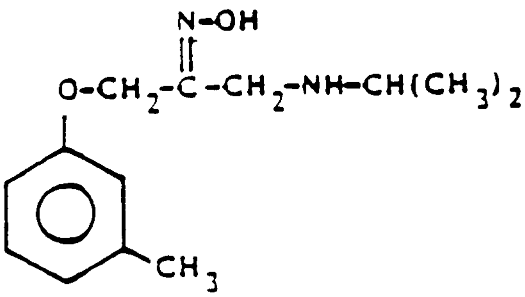

or a pharmaceutically acceptable acid addition salt thereof.

**19.** A process according to claim 1, wherein the compound of the general formula I is:

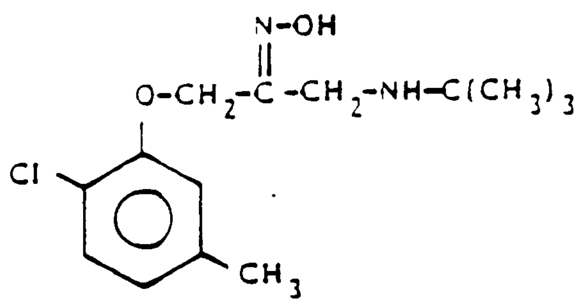

or a pharmaceutically acceptable acid addition salt thereof.

**20.** Use of a compound having the formula I or a pharmaceutically acceptable acid addition salt thereof as defined in any one of claims 1 to 19 in the preparation of a medicament having a $\beta$-adrenergic blocking effect.

**21.** A process of preparing an ophthalmic pharmaceutical composition of matter for use in the lowering of intraocular pressure in a warm-blooded animal, especially in the treatment of glaucoma, said process comprising mixing a compound having the formula

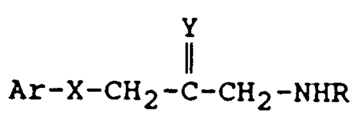

(I)

or a pharmaceutically acceptable acid addition salt thereof, wherein -X- is -O-, $-CH_2-$ or -; =Y is =O or a derivatized keto group which is hydrolyzable or enzymatically convertible to a keto group; R is alkyl having 1 to 12 carbon atoms or aralkyl having from 7 to 20 carbon atoms, the aralkyl group optionally bearing methoxy substituents on the aryl portion thereof; and Ar is the 3-aromatic or heterocyclic residue of a 1-alkylamino-2-propanol having an optionally substituted aromatic or heterocyclic substituent at the 3-position and having $\beta$-adrenergic blocking properties with a non-toxic ophthalmically acceptable carrier therefor and dividing the resulting mixture into unit dosage forms, each containing an effective intraocular pressure reducing amount of the compound of the formula I or a salt thereof.

**22.** A process according to claim 21, wherein =Y is =N-OR$_1$, =N-NH$_2$, =N-NR$_1$R$_2$,

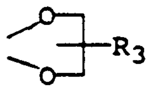 , 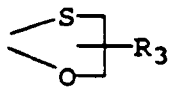 , or 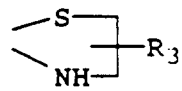 ;

R$_1$ and R$_2$ may be the same or different and are H or alkyl having from 1 to 8 carbon atoms; and R$_3$ is R$_1$, -COOR$_1$ or -CON(R$_1$)$_2$ wherein R$_1$ is defined as above.

**23.** A process according to Claim 22, wherein =Y is =NOR$_1$ wherein R$_1$ is H or alkyl having from 1 to 8 carbon atoms.

**24.** A process according to any one of Claims 21-23, wherein Ar is the 3-aromatic or heterocyclic residue of propranolol, timolol, carteolol, alprenolol, atenolol, befunolol, betaxolol, bevantolol, bufuralol, bunitrolol, bupranolol, celiprolol, cetamolol, labetolol, levobunolol, mepindolol, metipranolol, metoprolol, moprolol, nadolol, oxprenolol, penbutolol, pindolol or toliprolol.

**25.** A process according to Claim 21 comprising an effective intraocular pressure reducing amount of a compound having the formula

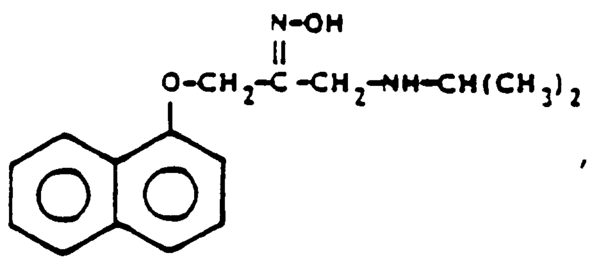 ,

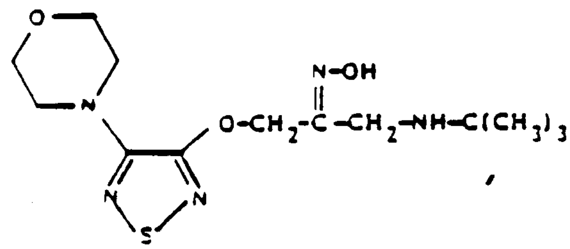

,

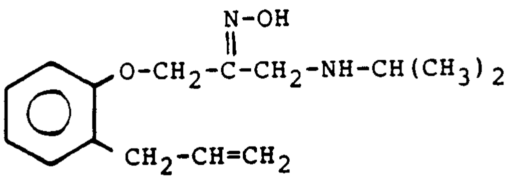

,

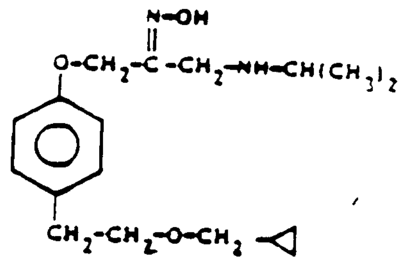

,

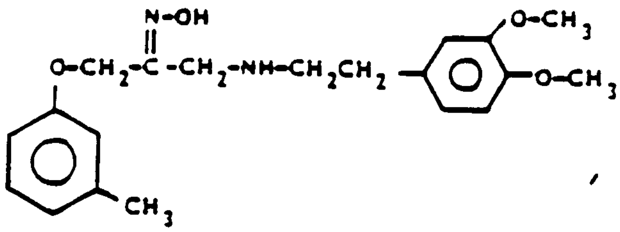

,

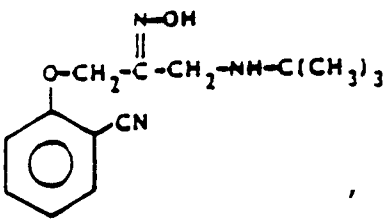

,

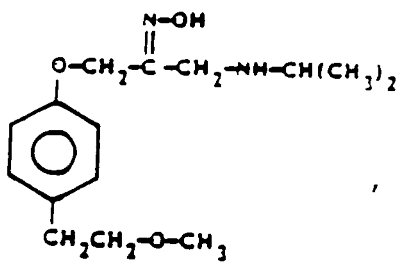

,

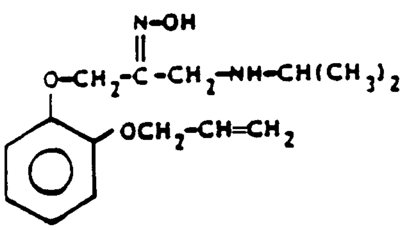

,

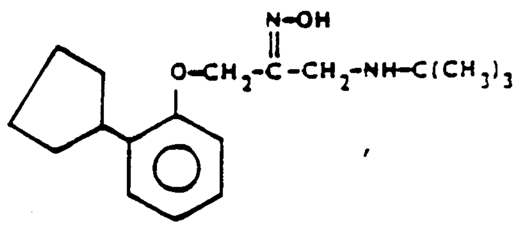

,

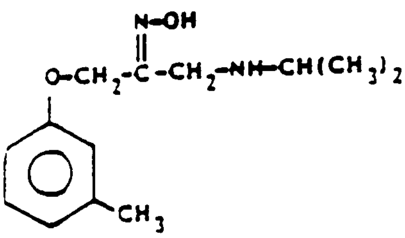

or

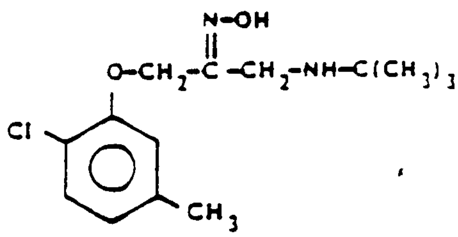

,

or a pharmaceutically acceptable acid addition salt thereof.

**26.** Use of a compound having the formula

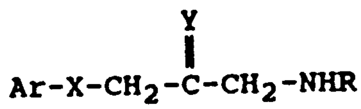

(I)

or a pharmaceutically acceptable acid addition salt thereof as defined in claim 21 in the preparation of a medicament having an intraocular pressure lowering effect, especially for use in the treatment of glaucoma.

**27.** Use according to Claim 26, wherein $=Y$ is $=N\text{-}OR_1$, $=N\text{-}NH_2$, $=N\text{-}NR_1R_2$,

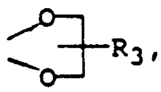 , 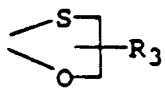 , or 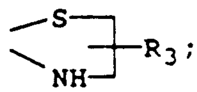 ;

$R_1$ and $R_2$ may be the same or different and are H or alkyl having from 1 to 8 carbon atoms; and $R_3$ is $R_1$, $-COOR_1$ or $-CON(R_1)_2$ wherein $R_1$ is defined as above.

**28.** Use according to Claim 27, wherein $=Y$ is $=NOR_1$ wherein $R_1$ is H or alkyl having from 1 to 8 carbon atoms.

**29.** Use according to any one of Claims 26-28, wherein Ar is the 3-aromatic or heterocyclic residue of propranolol, timolol, carteolol, alprenolol, atenolol, befunolol, betaxolol, bevantolol, bufuralol, bunitrolol, bupranolol, celiprolol, cetamolol, labetolol, levobunolol, mepindolol, metipranolol, metoprolol, moprolol, nadolol, oxprenolol, penbutolol, pindolol or toliprolol.

**30.** Use according to Claim 26, wherein the compound 5 is selected from the group consisting of

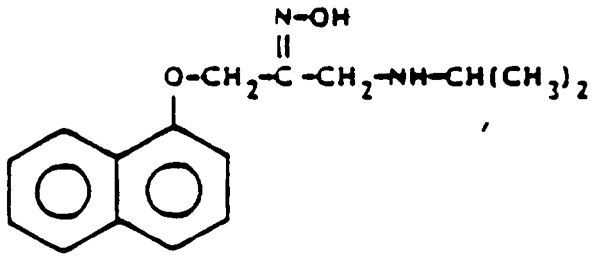

,

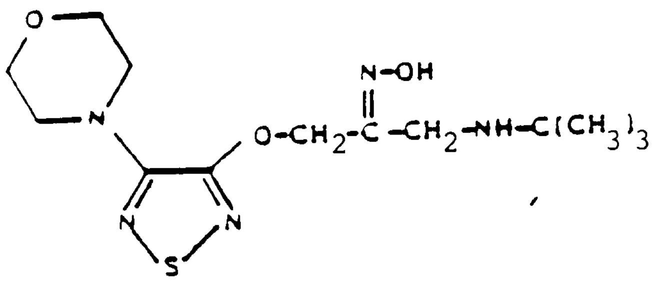

,

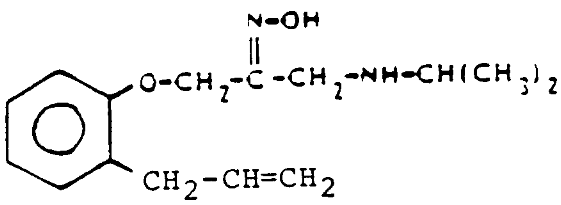

,

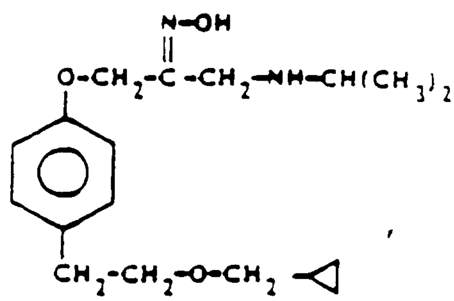

,

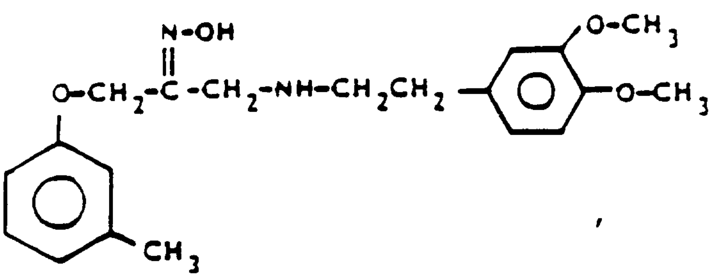

,

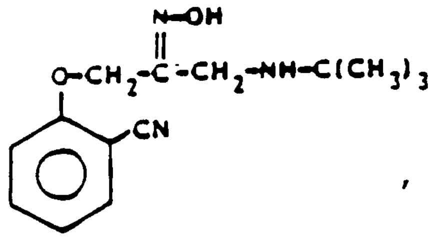
,
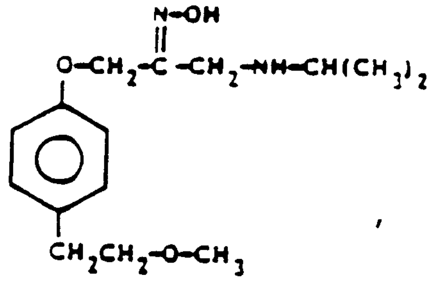
,
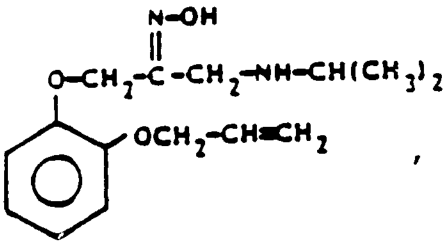
,
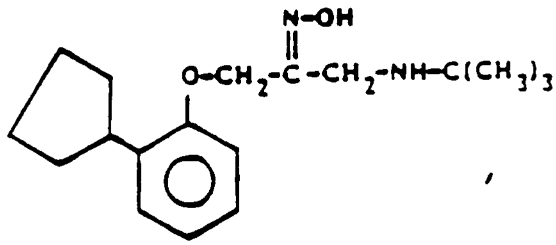
,
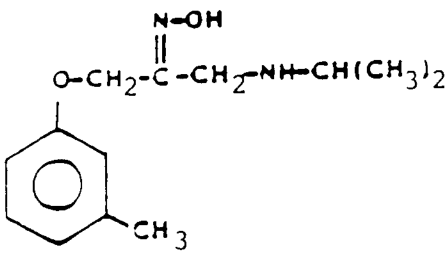
and
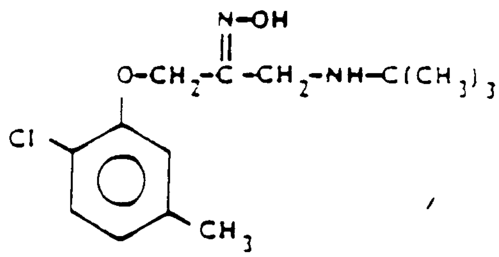
,

and the pharmaceutically acceptable acid addition salts thereof.

**Patentansprüche**
**Patentanspruch für folgende Vertragsstaaten : AT, BE, CH, DE, FR, GB, IT, LI, LU, NL, SE**

**1.** Verbindung der Formel

$$Ar-X-CH_2-\overset{\overset{Y}{\|}}{C}-CH_2-NHR \qquad (I)$$

oder deren pharmazeutisch verträgliche Säureadditionssalze, worin -X- -O-, $-CH_2-$ ist oder =Y eine derivatisierte Ketogruppe ist, die hydrolisierbar oder enzymatisch zu einer Ketogruppe umwandelbar ist, R Alkyl mit 1 bis 12 Kohlenstoffatomen oder Aralkyl mit 7 bis 20 Kohlenstoffatomen ist, wobei die Aralkylgruppe gegebenfalls am Arylteil Methoxy-Substituenten aufweist und Ar ein aromatischer oder substituierter heterocyclischer Rest in 3-Stellung eines 1-Alkylamino-2-propanols ist, das in der 3-Stellung einen gegebenfalls substituierten, aromatischen oder heterocyclischen Substituenten aufweist, und die über die Eigenschaft verfügt, $\beta$-adrenerge Rezeptoren zu blockieren.

2. Verbindung oder Salz nach Anspruch 1, worin =Y =N-OR$_1$, =N-NH$_2$,

=N-NR$_1$R$_2$, 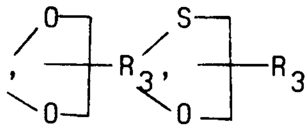 oder 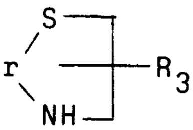

ist, wobei R$_1$ und R$_2$ gleich oder verschieden sein können und H oder Alkyl mit 1 bis 8 Kohlenstoffatomen sind, und R$_3$ R$_1$, -COOR$_1$ oder -CON(R$_1$)$_2$ ist, worin R$_1$ die obige Bedeutung besitzt.

3. Verbindung oder Salz nach Anspruch 2, worin =Y =NOR$_1$ ist, und R$_1$ H oder Alkyl mit 1 bis 8 Kohlenstoffatomen bedeutet.

4. Verbindung oder Salz nach Anspruch 3, worin =Y =NOH ist.

5. Verbindung oder Salz nach einem der vorhergehenden Ansprüche, worin -X- -O- ist.

6. Verbindung oder Salz nach einem der vorhergehenden Ansprüche 1 bis 5, worin R Isopropyl oder t-Butyl ist.

7. Verbindung oder Salz nach einem der Ansprüche 1 bis 6, worin Ar der in 3-Stellung aromatisch oder heterocyclisch substituierte Rest von Propanolol, Timolol, Carteolol, Alprenolol, Atenolol, Befunolol, Betaxolol, Bevantolol, Bufuralol, Bunitrolol, Bupranolol, Celipropol, Cetamolol, Labetolol, Levobunolol, Mepindolol, Metipranolol, Metoprolol, Moprolol, Nadolol, Oxprenolol, Penbutolol, Pindolol oder Toliprolol ist.

8. Verbindung oder Salz nach einem der Ansprüche 1 bis 6, worin Ar

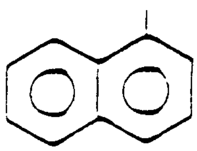 , 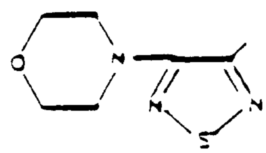 ,

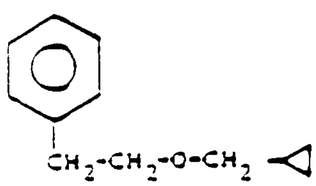 , 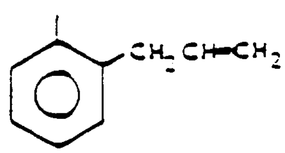 ,

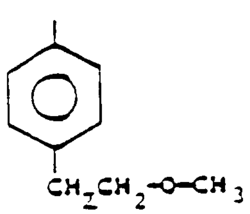 , 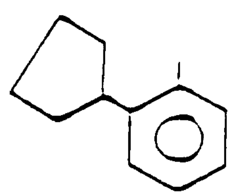 ,

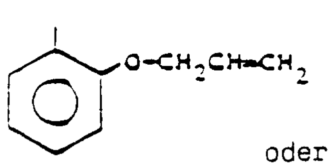 oder 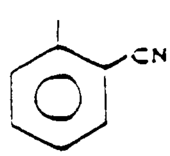 .

**9.** Verbindung nach Anspruch 1 der Formel

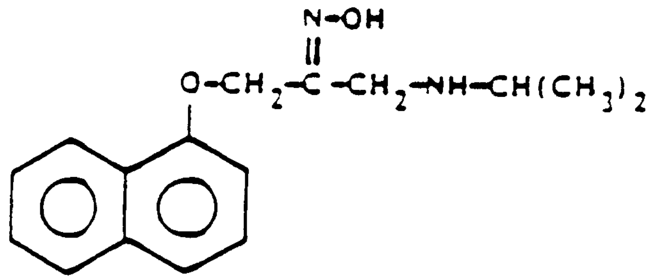

oder eines pharmazeutisch verträglichen Säureadditionssalzes davon.

**10.** Verbindung nach Anspruch 1 der Formel

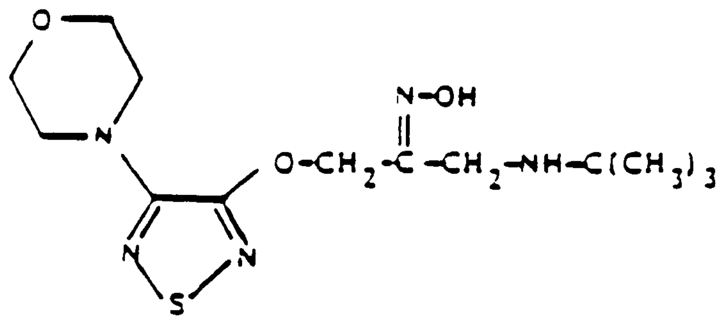

oder eines pharmazeutisch verträglichen Säureadditionssalzes davon.

**11.** Verbindung nach Anspruch 1 der Formel

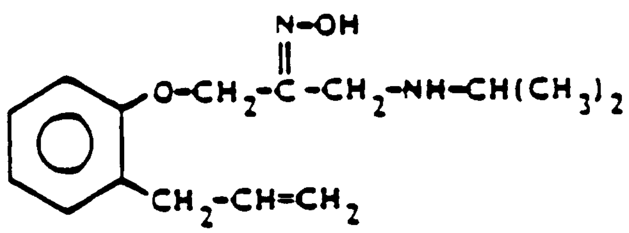

oder eines pharmazeutisch verträglichen Säureadditionssalzes davon.

**12.** Verbindung nach Anspruch 1 der Formel

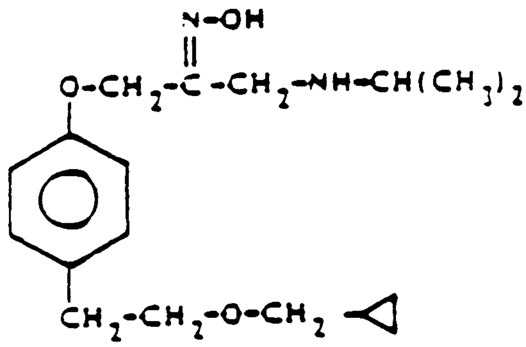

oder eines pharmazeutisch verträglichen Säureadditionssalzes davon.

**13.** Verbindung nach Anspruch 1 der Formel

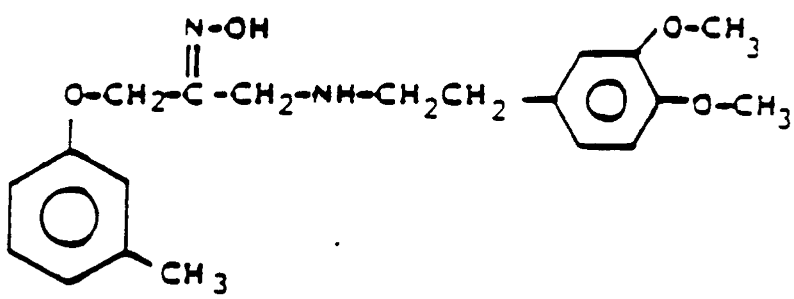

oder eines pharmazeutisch verträglichen Säureadditionssalzes davon

**14.** Verbindung nach Anspruch 1 der Formel

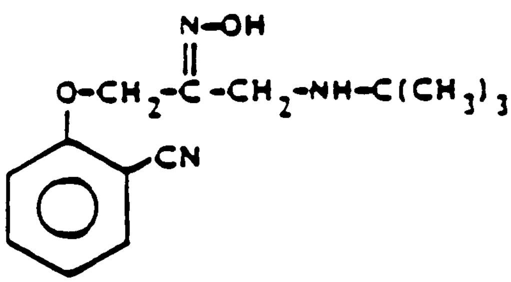

oder eines pharmazeutisch verträglichen Säureadditonssalzes davon.

**15.** Verbindung nach Anspruch 1 der Formel

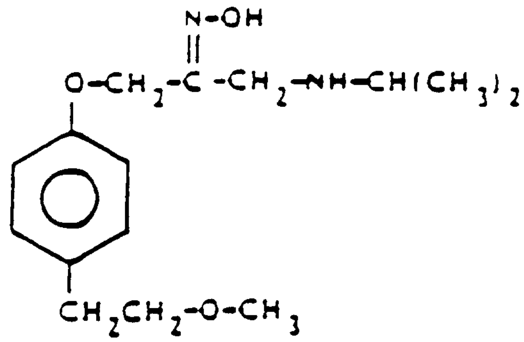

oder eines pharmazeutisch verträglichen Säureadditionssalzes davon.

**16.** Verbindung nach Anspruch 1 der Formel

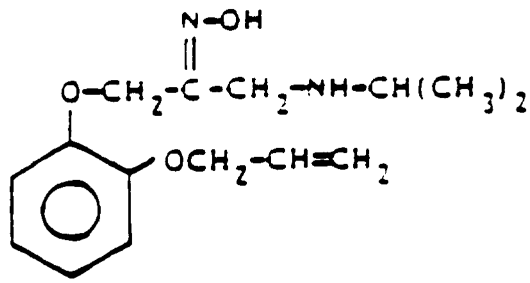

oder eines pharmazeutisch verträglichen Säureadditionssalzes davon.

**17.** Verbindung nach Anspruch 1 der Formel

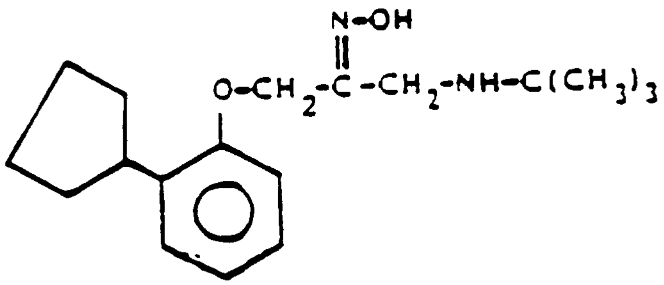

oder eines pharmazeutisch verträglichen Säureadditionssalzes davon.

**18.** Verbindung nach Anspruch 1 der Formel

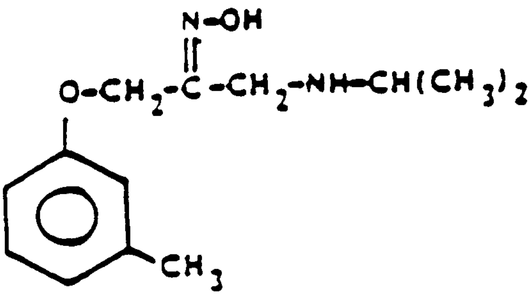

oder eines pharmazeutisch verträglichen Säureadditionssalzes davon.

**19.** Verbindung nach Anspruch 1 der Formel

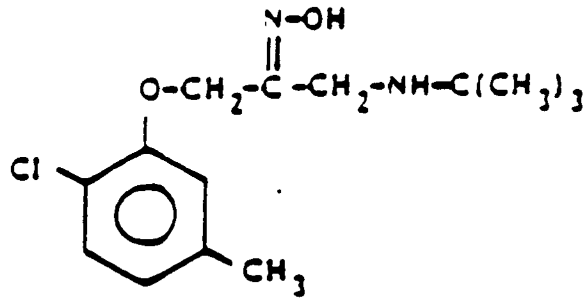

oder eines pharmazeutisch verträglichen Säureadditionssalzes davon.

**20.** Verwendung einer Verbindung nach einem der Ansprüche 1 bis 19 oder deren pharmazeutisch verträgliche Säureadditonssalze zur Herstellung eines Arzneimittels mit einer $\beta$-Rezeptoren blockierenden Wirkung.

**21.** Pharmazeutische Zubereitung in Einzeldosisform zur Verwendung zum Auslösen einer Blockierung der $\beta$-Rezeptoren in Warmblütlern, wobei diese Zubereitung eine wirksame Menge zum Blockieren der $\beta$-Rezeptoren einer Verbindung, wie sie in einem der Ansprüche 1 bis 19 beansprucht worden ist oder eines pharmazeutisch verträglichen Säureadditionssalzes davon, und einen nichttoxischen pharmazeutisch verträglichen Träger dafür enthält.

**22.** Verwendung einer Verbindung der Formel

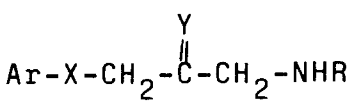 (I)

oder eines pharmazeutisch verträglichen Säureadditionssalzes davon, worin -X- -O- und $-CH_2-$ ist oder =Y =O oder eine abgewandelte Ketogruppe ist, die hydrolisierbar oder enzymatisch in eine Ketogruppe umwandelbar ist, R Alkyl mit 1 bis 12 Kohlenstoffatomen oder Aralkyl mit 7 bis 20 Kohlenstoffatomen ist, wobei die Aralkylgruppe gegebenfalls im Arylteil Methoxy-Substituenten aufweist, und Ar der aromatische oder heterocyclische Rest in 3-Stellung eines 1-Alkylamino-2-propanols ist, welches einen gegebenfalls substituierten, aromatischen oder heterocyclischen Substituenten in der 3-Stellung aufweist, und die über die Eigenschaft verfügt, $\beta$-adrenerge Rezeptoren zu blockieren, zur Herstellung eines Arzneimittels mit einer den intraokularen Druck erniedrigenden Wirkung, insbesondere zur Verwendung bei der Behandlung des Glaucoms.

**23.** Verwendung nach Anspruch 22, worin =Y =N-OR$_1$, =N-NH$_2$,

=N-NR$_1$R$_2$, 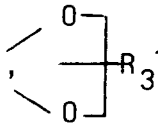, 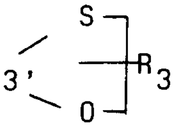 oder 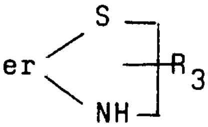

ist, und R$_1$ und R$_2$ gleich oder verschieden sein können, und H oder Alkyl mit 1 bis 8 Kohlenstoffatomen sind, und R$_3$ R$_1$, -COOR$_1$ oder -CON (R$_1$)$_2$ ist, worin R$_1$ die obige Bedeutung besitzt.

**24.** Verwendung nach Anspruch 23, worin =Y =NOR$_1$ ist und R$_1$ H oder Alkyl mit 1 bis 8 Kohlenstoffatomen bedeutet.

**25.** Verwendung nach einem der Ansprüche 22 bis 25, worin Ar ein aromatischer oder heterocyclischer Rest in 3-Stellung von Propanolol, Timolol, Carteolol, Alprenolol, Atenolol, Befunolol, Betaxolol, Bevantolol, Bufuralol, Bunitrolol, Bupranolol, Celiprolol, Cetamolol, Labetolol, Levobunolol, Mepindolol, Metipranolol, Metoprolol, Moprolol, Nadolol, Oxprenolol, Penbutolol, Pindolol oder Toliprolol ist.

**26.** Verwendung nach Anspruch 22, worin die Verbindung ausgewählt ist aus der Gruppe, bestehend aus

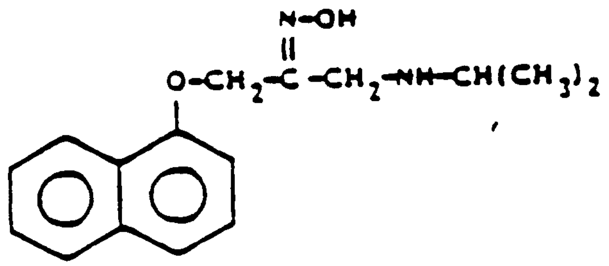

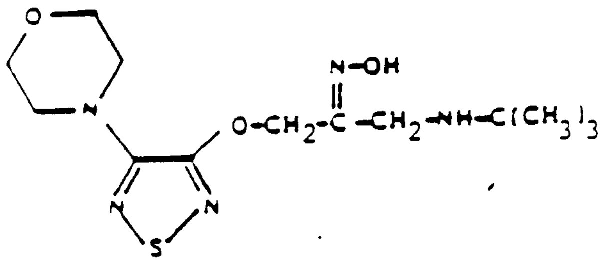

,

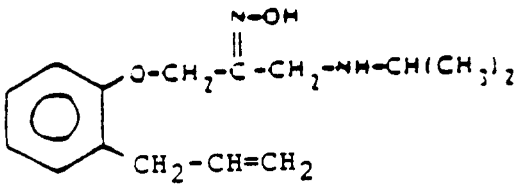

,

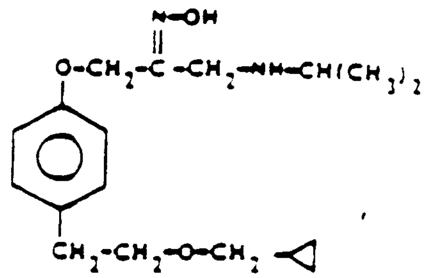

,

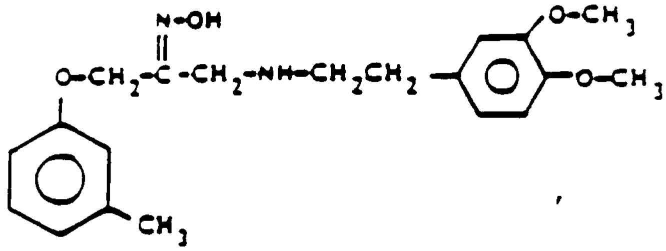

,

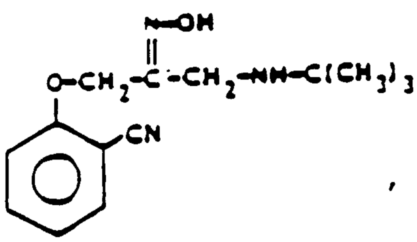

,

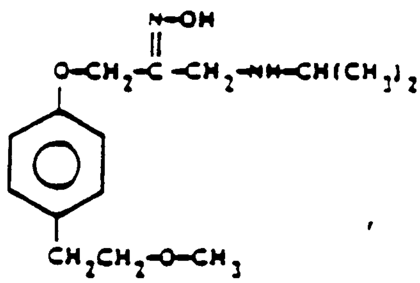

,

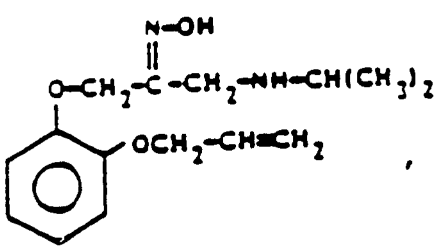

,

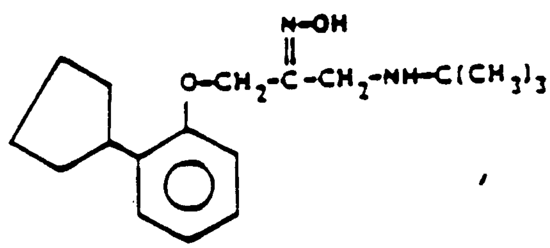

,

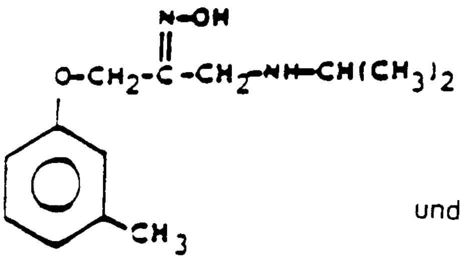

und

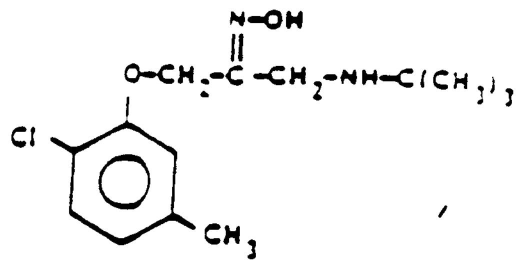

,

und des pharmazeutisch verträglichen Säureadditionssalzes davon.

**27.** Eine ophtamologische pharmazeutische Zubereitung in Einzeldosisform zur Erniedrigung des intraokularen Drucks in Warmblütlern, insbesondere zur Behandlung des Glaucoms, wobei diese Zubereitung eine zur Verringerung des intraokularen Drucks wirksame Menge einer Verbindung der Formel

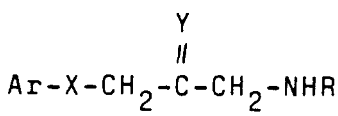

(I)

oder eines pharmazeutisch verträglichen Säureadditionssalzes davon, wie in Anspruch 22 definiert ist und eines nichttoxischen, ophtamologischen verträglichen Trägers dafür.

**28.** Zubereitung nach Anspruch 27, worin $=Y$ $=N-OR_1$, $=N-NH_2$,

$=N-NR_1R_2$, 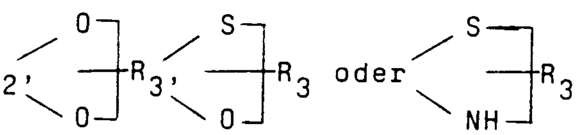 oder

ist und $R_1$ und $R_2$ gleich oder verschieden sein können und H oder Alkyl mit 1 bis 8 Kohlenstoffatomen bedeuten, und $R_3$ $R_1$, $-COOR_1$ oder $-CON(R_1)_2$ ist, worin $R_1$ die obige Bedeutung besitzt.

**29.** Zubereitung nach Anspruch 28, worin $=Y$ $=NOR_1$ ist und $R_1$ H oder Alkyl mit 1 bis 8 Kohlenstoffatomen bedeutet.

**30.** Zubereitung nach einem der Ansprüche 27 bis 29, worin Ar der aromatische oder heterocyclische Rest in 3-Stellung von Propanolol, Timolol, Carteolol, Alprenolol, Atenolol, Befunolol, Betaxolol, Bevantolol, Bufuralol, Bunitrolol, Bupranolol, Celiprolol, Cetamolol, Labetolol, Levobunolol, Mepindolol, Metipranolol, Metoprolol, Moprolol, Nadolol, Oxprenolol, Penbutolol, Pindolol oder Toliprolol ist.

**31.** Zubereitung nach Anspruch 27, enthaltend eine zur Verringerung des intraokularen Drucks wirksamen Menge einer Verbindung der Formel

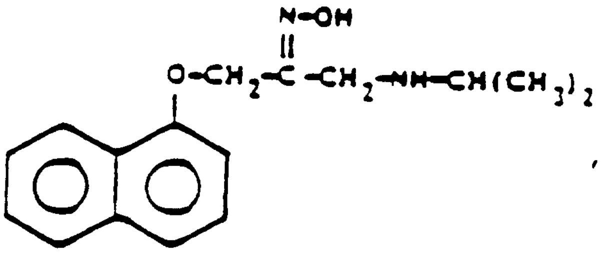

,

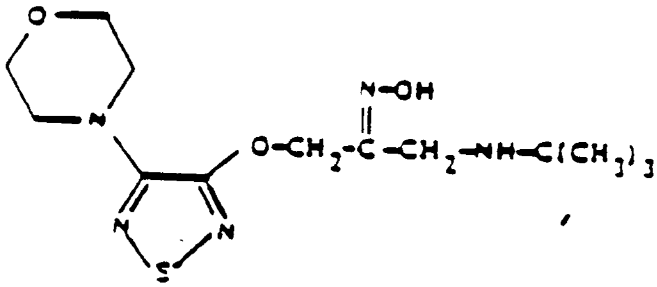

,

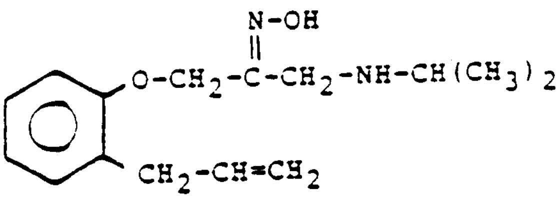

,

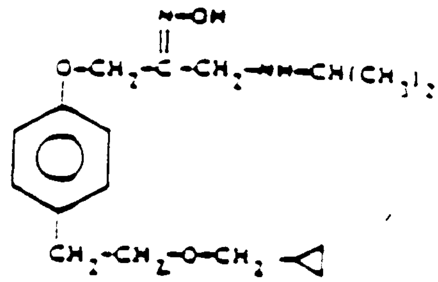

,

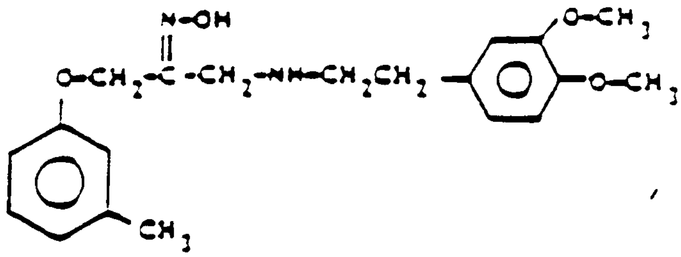

,

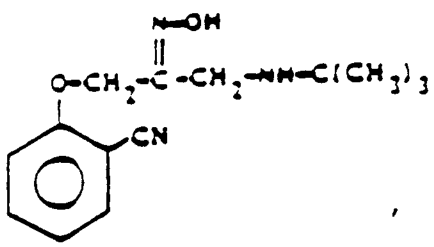

,

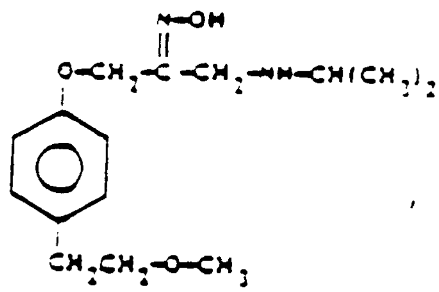

,

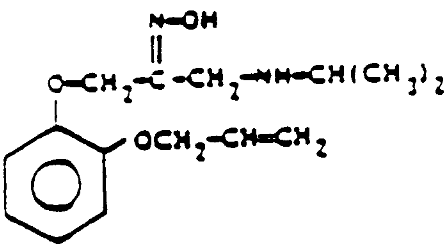

,

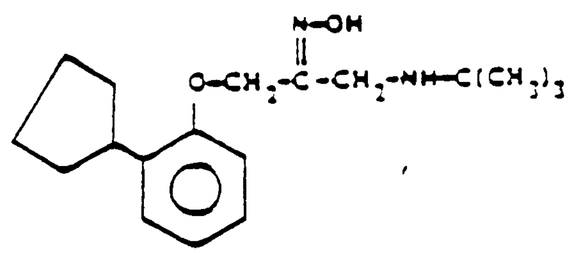

,

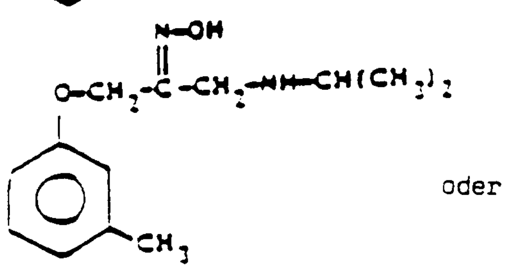

oder

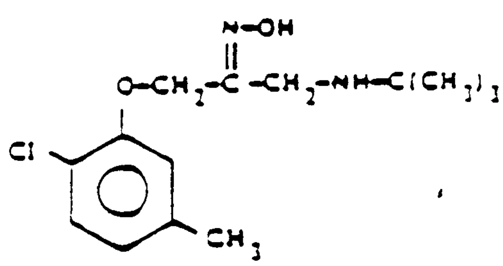

,

oder eines pharmazeutisch verträglichen Säureadditionssalzes davon.

**32.** Verbindungen der Ansprüche 1 bis 19 zur Verwendung in der Therapie.

**Patentansprüche für folgende Vertragsstaaten : ES, GR**

**1.** Verfahren zur Herstellung einer pharmazeutischen Zusammensetzung zum Blockieren $\beta$-adrenerger Rezeptoren in Warmblütlern, wobei dieses Verfahren das Vermischen einer Verbindung der Formel

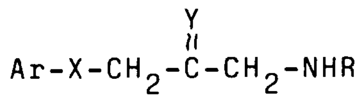 (I)

oder deren pharmazeutisch verträglicher Säureadditionssalze, worin -X- -O-, $-CH_2-$ ist oder =Y eine derivatisierte Ketogruppe ist, die hydrolisierbar oder enzymatisch zu einer Ketogruppe umwandelbar ist, R Alkyl mit 1 bis 12 Kohlenstoffatomen oder Aralkyl mit 7 bis 20 Kohlenstoffatomen ist, wobei die Aralkylgruppe gegebenfalls am Arylteil Methoxy-Substituenten aufweist und Ar ein aromatischer oder substituierter heterocyclischer Rest in 3-Stellung eines 1-Alkylamino-2-propanols ist, das in der 3-Stellung einen gegebenfalls substituierten, aromatischen oder heterocyclischen Substituenten aufweist, und die über die Eigenschaft verfügt, β-adrenerge Rezeptoren zu blockieren mit einem nichttoxischen pharmazeutisch verträglichen Träger dafür und Zerteilen der erhaltenen Mischung in eine Einzeldosiszubereitung umfaßt, von der jede eine zur Blockierung der β-adrenergen Rezeptoren wirksame Menge der Verbindung der Formel I oder eines Salzes davon enthält.

**2.** Verfahren nach Anspruch 1, worin =Y =N-$OR_1$, =N-$NH_2$,

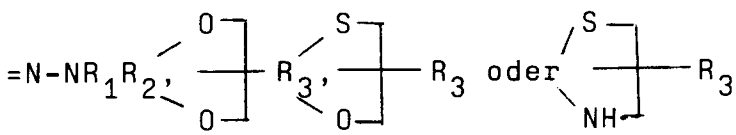

ist, wobei $R_1$ und $R_2$ gleich oder verschieden sein können und H oder Alkyl mit 1 bis 8 Kohlenstoffatomen sind, und $R_3$ $R_1$, -$COOR_1$ oder -$CON(R_1)_2$ ist, worin $R_1$ die obige Bedeutung besitzt.

**3.** Verfahren nach Anspruch 2, worin =Y =$NOR_1$ ist und $R_1$ H oder Alkyl mit 1 bis 8 Kohlenstoffatomen bedeutet.

**4.** Verfahren nach Anspruch 3, worin =Y =NOH ist.

**5.** Verfahren nach einem der vorhergehenden Ansprüche, worin -X- -O- ist.

**6.** Verfahren nach einem der vorhergehenden Ansprüche 1 bis 5, worin R Isopropyl oder t-Butyl ist.

**7.** Verfahren nach einem der Ansprüche 1 bis 6, worin Ar der in 3-Stellung aromatisch oder heterocyclisch substituierte Rest von Propanolol, Timolol, Carteolol, Alprenolol, Atenolol, Befunolol, Betaxolol, Bevantolol, Bufuralol, Bunitrolol, Bupranolol, Celipropol, Cetamolol, Labetolol, Levobunolol, Mepindolol, Metipranolol, Metoprolol, Moprolol, Nadolol, Oxprenolol, Penbutolol, Pindolol oder Toliprolol ist.

8. Verfahren nach einem der Ansprüche 1 bis 6, worin Ar

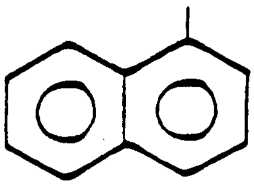

,

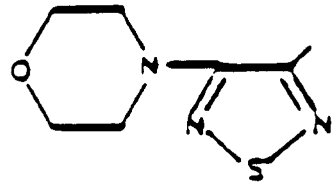

,

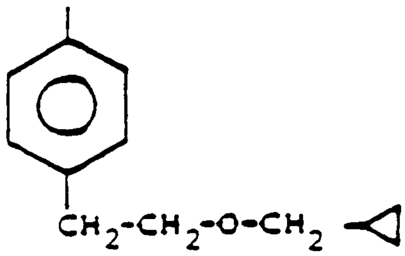

,

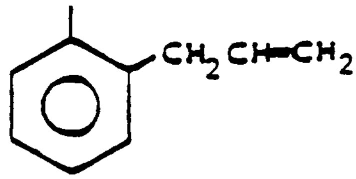

,

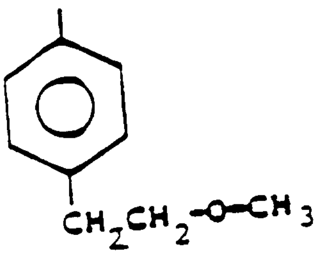

,

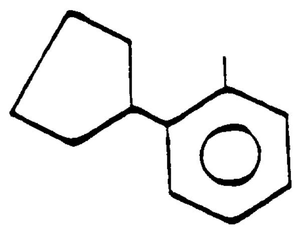

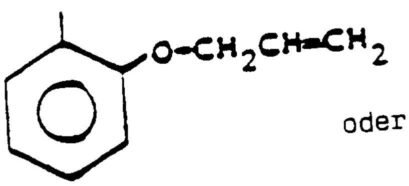

oder

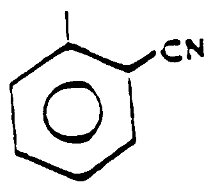

ist.

9. Verfahren nach Anspruch 1, worin die Verbindung der allgemeinen Formel I

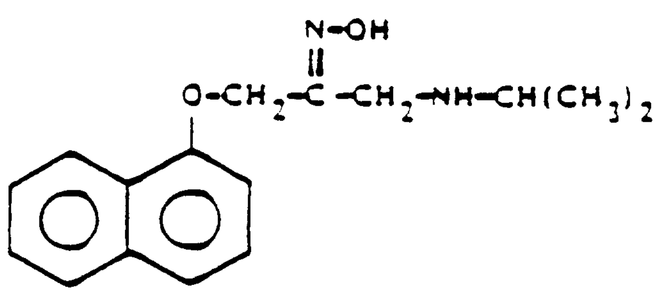

oder ein pharmazeutisch verträgliches Säureadditionssalz davon ist.

**10.** Verfahren nach Anspruch 1, worin die Verbindung der allgemeinen Formel I

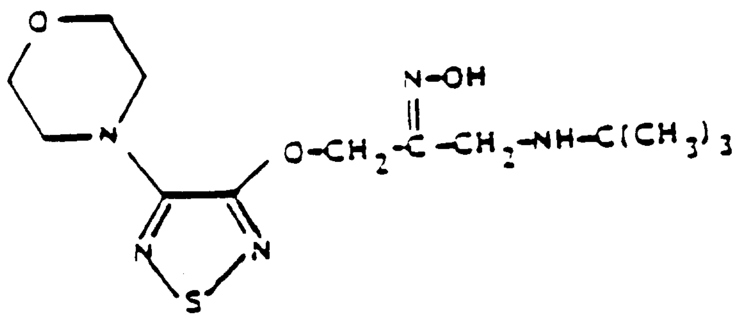

oder ein pharmazeutisch verträgliches Säureadditionssalz davon ist.

**11.** Verfahren nach Anspruch 1, worin die Verbindung der allgemeinen Formel I

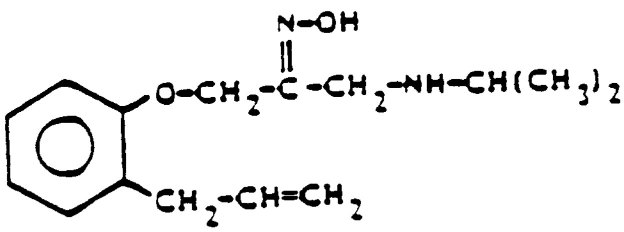

oder ein pharmazeutisch verträgliches Säureadditionssalz davon ist.

**12.** Verfahren nach Anspruch 1, worin die Verbindung der allgemeinen Formel I

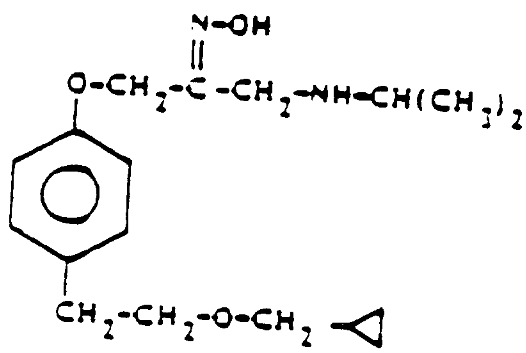

oder ein pharmazeutisch verträgliches Säureadditionssalz davon ist.

**13.** Verfahren nach Anspruch 1, worin die Verbindung der allgemeinen Formel I

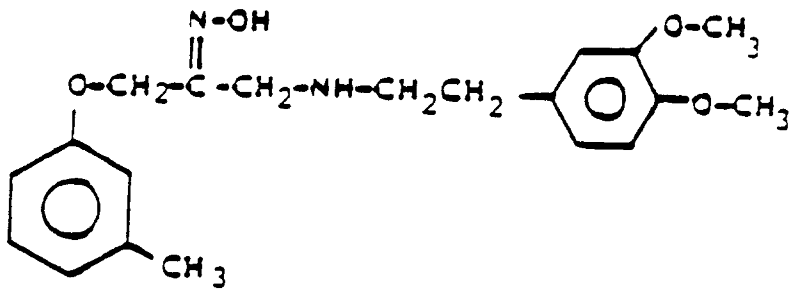

oder ein pharmazeutisch verträgliches Säureadditionssalz davon ist.

**14.** Verfahren nach Anspruch 1, worin die Verbindung der allgemeinen Formel I

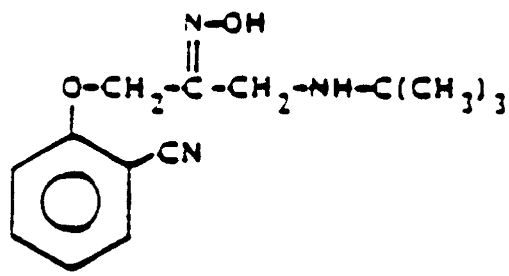

oder ein pharmazeutisch verträgliches Säureadditonssalz davon ist.

**15.** Verfahren nach Anspruch 1, worin die Verbindung der allgemeinen Formel I

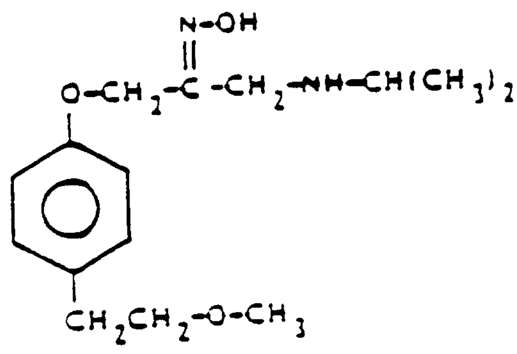

oder ein pharmazeutisch verträgliches Säureadditionssalz davon ist.

**16.** Verfahren nach Anspruch 1, worin die Verbindung der allgemeinen Formel I

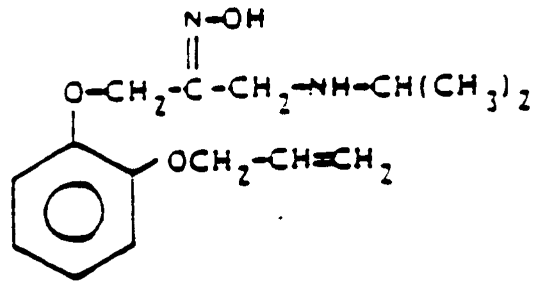

oder ein pharmazeutisch verträgliches Säureadditionssalz davon ist.

**17.** Verfahren nach Anspruch 1, worin die Verbindung der allgemeinen Formel I

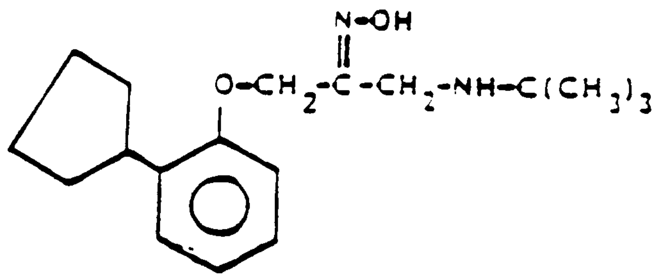

oder ein pharmazeutisch verträgliches Säureadditionssalz davon ist.

**18.** Verfahren nach Anspruch 1, worin die Verbindung der allgemeinen Formel I

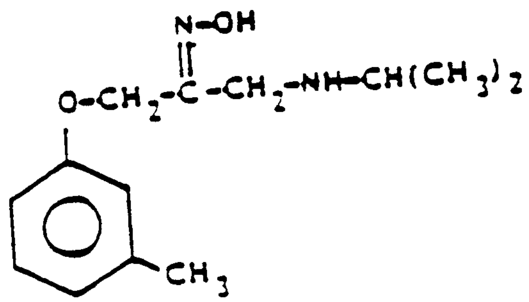

oder ein pharmazeutisch verträgliches Säureadditionssalz davon ist.

**19.** Verfahren nach Anspruch 1, worin die Verbindung der allgemeinen Formel I

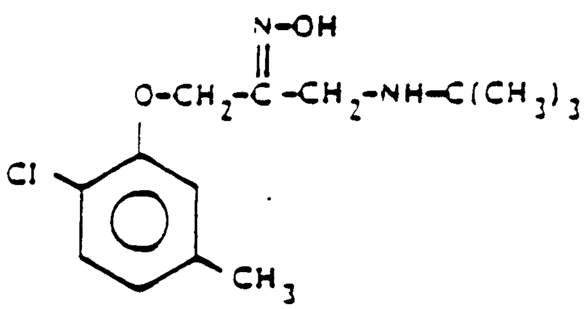

oder ein pharmazeutisch verträgliches Säureadditionssalz davon ist.

**20.** Verwendung einer Verbindung der Formel I oder deren pharmazeutisch verträglichen Säureadditionssalzes nach einem der Ansprüche 1 bis 19 zur Herstellung eines Arzneimittels mit einer $\beta$-Rezeptoren blockierenden Wirkung.

**21.** Verfahren zur Herstellung einer ophthalmologischen pharmazeutischen Zubereitung zur Verringerung des intraokularen Drucks in Warmblütlern, insbesondere bei der Behandlung des Glaucoms, wobei dieses Verfahren das Vermischen einer Verbindung der Formel

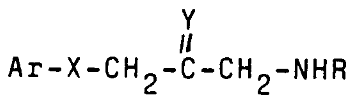

(I)

oder eines pharmazeutisch verträglichen Säureadditionssalzes davon, worin -X- -O- und $-CH_2-$ ist oder =Y =O oder eine abgewandelte Ketogruppe ist, die hydrolisierbar oder enzymatisch in eine Ketogruppe umwandelbar ist, R Alkyl mit 1 bis 12 Kohlenstoffatomen oder Aralkyl mit 7 bis 20 Kohlenstoffatomen ist, wobei die Aralkylgruppe gegebenfalls im Arylteil Methoxy-Substituenten aufweist, und Ar der aromatische oder heterocyclische Rest in 3-Stellung eines 1-Alkylamino-2-propanols ist, welches einen gegebenfalls substituierten, aromatischen oder heterocyclischen Substituenten in der 3-Stellung aufweist, und die über die Eigenschaft verfügt, $\beta$-adrenerge Rezeptoren zu blockieren, mit einem nichttoxischen ophthalmologisch verträglichen Träger dafür und Zerteilen der erhaltenen Mischung in Einzeldosisformen umfaßt, wobei jede eine zur Verringerung des intraokularen Drucks wirksame Menge der Verbindung der Formel I oder eines Salzes davon enthält.

**22.** Verfahren nach Anspruch 21, worin =Y $=N-OR_1$, $=N-NH_2$,

$=N-NR_1R_2$, 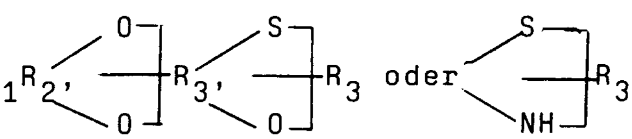 oder

ist, und $R_1$ und $R_2$ gleich oder verschieden sein können, und H oder Alkyl mit 1 bis 8 Kohlenstoffatomen sind, und $R_3$ $R_1$, $-COOR_1$ oder $-CON(R_1)_2$ ist, worin $R_1$ die obige Bedeutung besitzt.

**23.** Verfahren nach Anspruch 22, worin =Y $=NOR_1$ ist und $R_1$ H oder Alkyl mit 1 bis 8 Kohlenstoffatomen bedeutet.

**24.** Verfahren nach einem der Ansprüche 21 bis 23, worin Ar ein aromatischer oder heterocyclischer Rest in 3-Stellung von Propanolol Timolol, Carteolol, Alprenolol, Atenolol, Befunolol, Betaxolol, Bevantolol, Bufuralol, Bunitrolol, Bupranolol, Celiprolol, Cetamolol, Labetolol, Levobunolol, Mepindolol, Metipranolol, Metoprolol, Moprolol, Nadolol, Oxprenolol, Penbutolol, Pindolol oder Toliprolol ist.

**25.** Verfahren nach Anspruch 21, enthaltend eine zur Verringerung des intraokularen Drucks wirksame Menge einer Verbindung der Formel

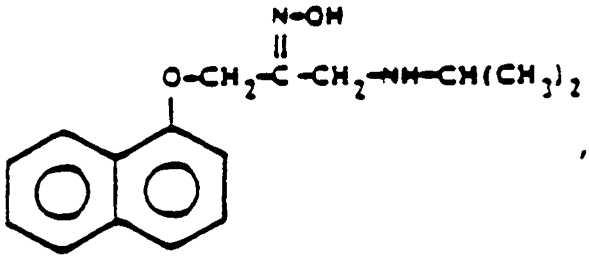

,

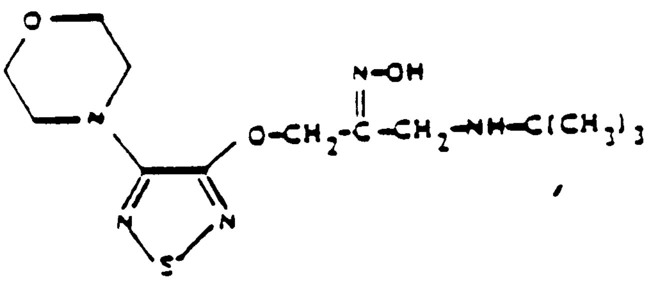

,

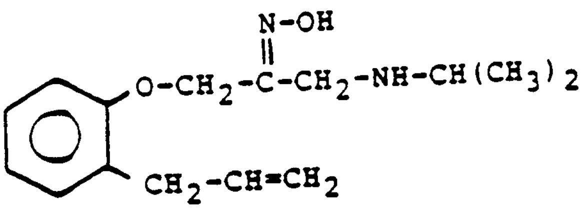

,

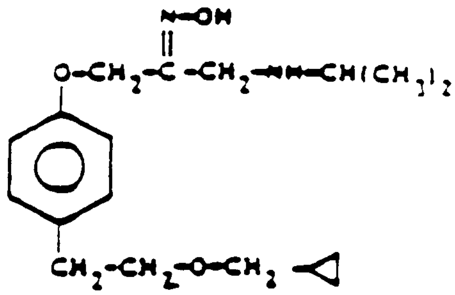

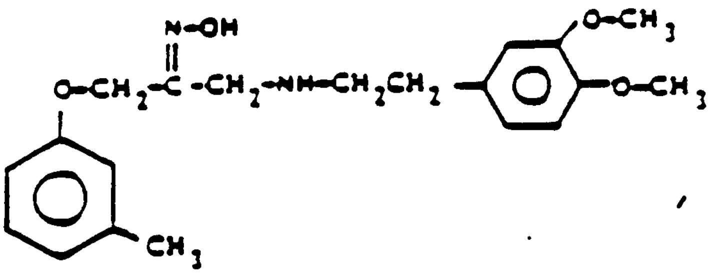

,

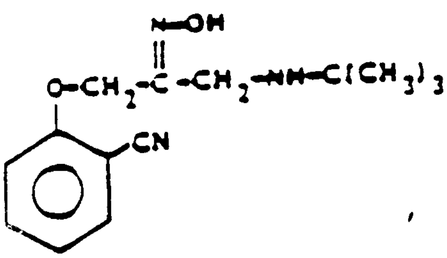

,

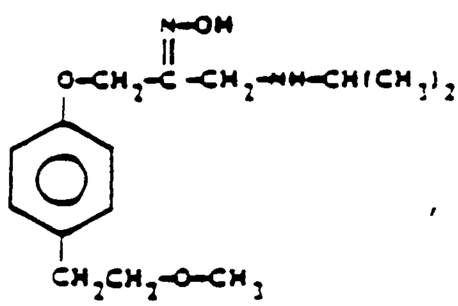

,

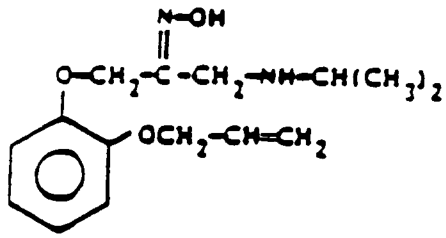 ,

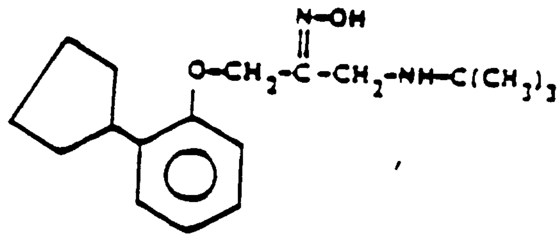 ,

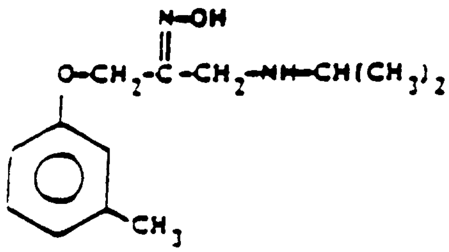 oder 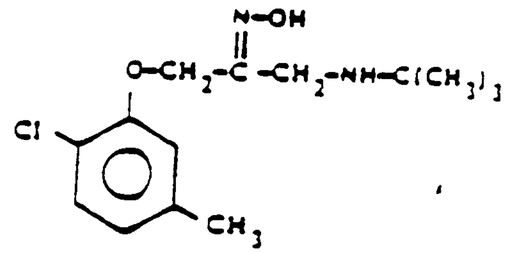 ,

oder eines pharmazeutisch verträglichen Säureadditionssalzes davon.

**26.** Verwendung einer Verbindung der Formel

$$Ar-X-CH_2-\overset{\overset{\displaystyle Y}{\|}}{C}-CH_2-NHR \qquad (I)$$

oder eines pharmazeutisch verträglichen Säureadditionssalzes davon, wie in Anspruch 21 definiert, zur Herstellung eines Arzneimittels mit einer den intraokularen Druck verringernden Wirkung, insbesondere zur Behandlung des Glaucoms.

**27.** Verwendung nach Anspruch 26, worin $=Y$ $=N-OR_1$, $=N-NH_2$,

$=N-NR_1R_2$, 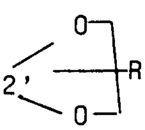 , 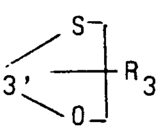 oder 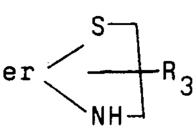

ist und $R_1$ und $R_2$ gleich oder verschieden sein können und H oder Alkyl mit 1 bis 8 Kohlenstoffatomen bedeuten, und $R_3$ $R_1$, $-COOR_1$ oder $-CON(R_1)_2$ ist, worin $R_1$ die obige Bedeutung besitzt.

**28.** Verwendung nach Anspruch 27, worin $=Y$ $=NOR_1$ ist und $R_1$ H oder Alkyl mit 1 bis 8 Kohlenstoffatomen bedeutet.

**29.** Verwendung nach einem der Ansprüche 26 bis 28, worin Ar der aromatische oder heterocyclische Rest in 3-Stellung von Propanolol, Timolol, Carteolol, Alprenolol, Atenolol, Befunolol, Betaxolol, Bevantolol, Bufuralol, Bunitrolol, Bupranolol, Celiprolol, Cetamolol, Labetolol, Levobunolol, Mepindolol, Metipranolol, Metoprolol, Moprolol, Nadolol, Oxprenolol, Penbutolol, Pindolol oder Toliprolol ist.

**30.** Verwendung nach Anspruch 26, worin die Verbindung ausgewählt ist aus der Gruppe bestehend aus

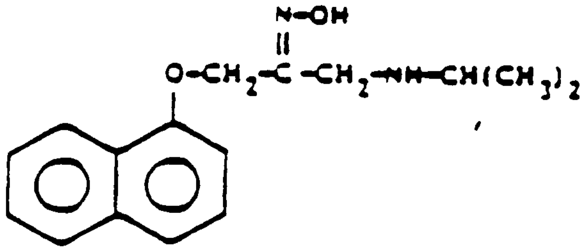

,

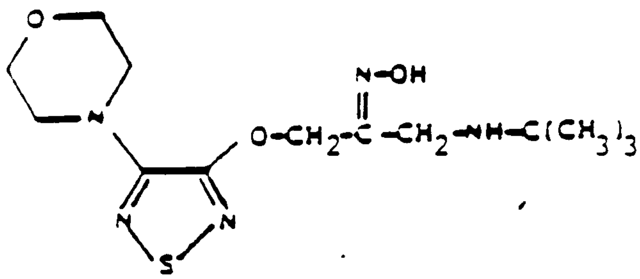

,

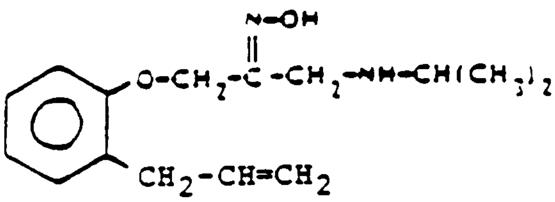

,

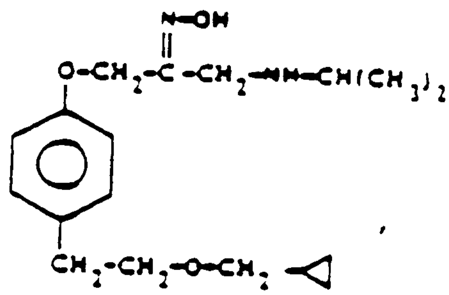

,

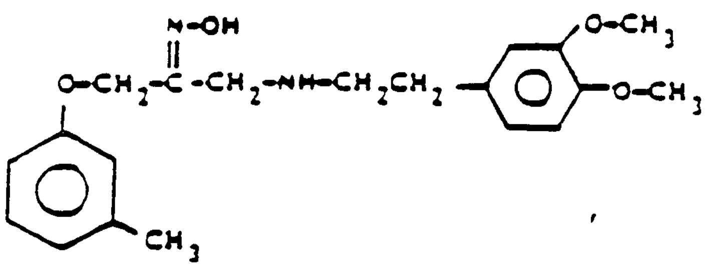

,

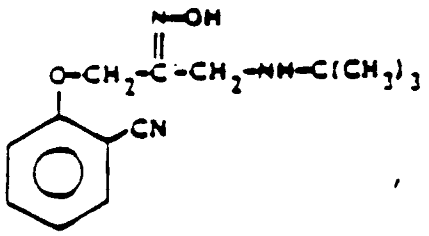

,

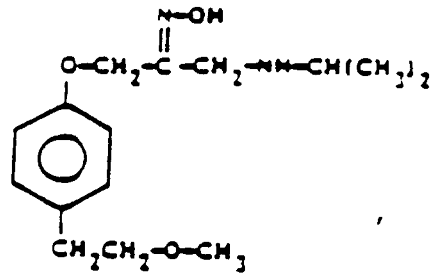

,

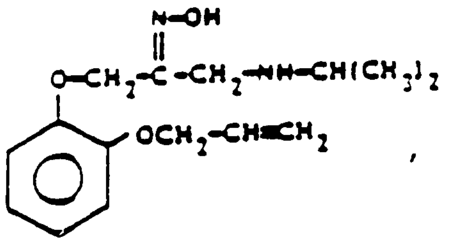

,

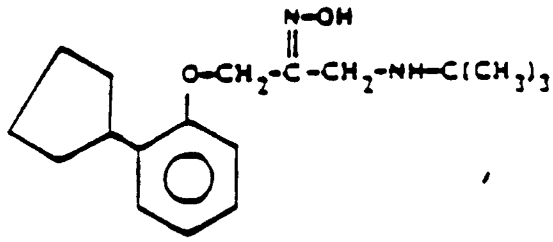

,

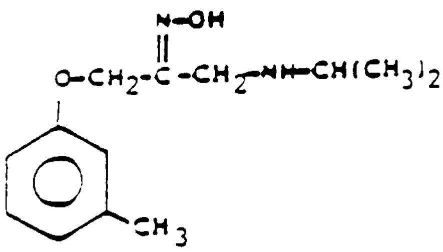

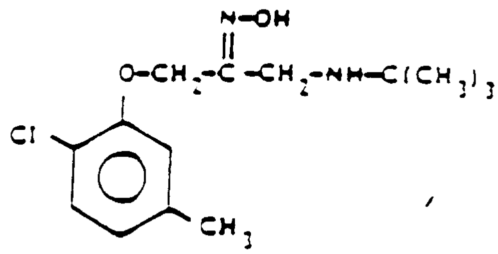

,

und deren pharmazeutisch verträgliche Säureadditionssalze.

**Revendications**

**Revendications pour les Etats contractants suivants : AT, BE, CH, DE, FR, GB, IT, LI, LU, NL, SE**

**1.** Composé de formule :

$$Ar-X-CH_2-\overset{\overset{Y}{\|}}{C}-CH_2-NHR \qquad (I)$$

ou sel d'addition acide pharmaceutiquement acceptable d'un tel composé, dans lequel -X- représente -O-, $-CH_2-$ ou - ; =Y est un groupe céto dérivé qui est hydrolysable ou transformable enzymatiquement en groupe céto ; R est un groupe alkyle ayant de 1 à 12 atomes de carbone ou aralkyle ayant de 7 à 20 atomes de carbone, le groupe aralkyle portant éventuellement des substituants méthoxy sur sa partie aryle ; et Ar est le résidu 3-aromatique ou hétérocyclique d'un 1-alkylamino-2-propanol ayant, en position-3, un substituant aromatique ou hétérocyclique éventuellement substitué et ayant des propriétés bloquantes vis-à-vis de récepteurs $\beta$-adrénergiques.

**2.** Composé ou sel selon la revendication 1, dans lequel $=Y$ représente $=N\text{-}OR_1$, $=N\text{-}NH_2$, $=N\text{-}NR_1R_2$,

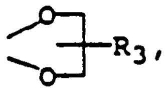, 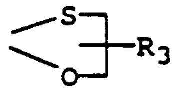, ou 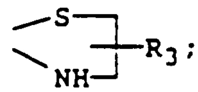;

$R_1$ et $R_2$ peuvent être identiques ou différents et représentent H ou alkyle ayant de 1 à 8 atomes de carbone ; et $R_3$ représente $R_1$, $-COOR_1$ ou $-CON(R_1)_2$ où $R_1$ est tel que défini ci-dessus.

**3.** Composé ou sel selon la revendication 2, dans lequel $=Y$ représente $=NOR_1$ où $R_1$ représente H ou alkyle ayant de 1 à 8 atomes de carbone.

**4.** Composé ou sel selon la revendication 3, dans lequel $=Y$ représente $=NOH$.

**5.** Composé ou sel selon l'une quelconque des revendications précédentes, dans lequel -X- représente -O-.

**6.** Composé ou sel selon l'une quelconque des revendications 1 à 5, dans lequel R est un groupe isopropyle ou t-butyle.

**7.** Composé ou sel selon l'une quelconque des revendications 1 à 6, dans lequel Ar est le résidu 3-aromatique ou hétérocyclique du propanolol, timolol, cartéolol, alprénolol, aténolol, béfunolol, bétaxolol, bévantolol, bufuralol, bunitrolol, bupranolol, céliprolol, cétamolol, labétolol, lévobunolol, mépindolol, métipranolol, métoprolol, moprolol, nadolol, oxprénolol, penbutolol, pindolol ou du toliprolol.

**8.** Composé ou sel selon l'une quelconque des revendications 1 à 6, dans lequel Ar représente

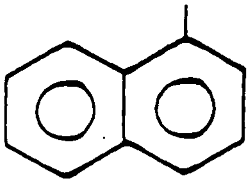 ,

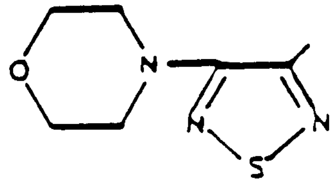 ,

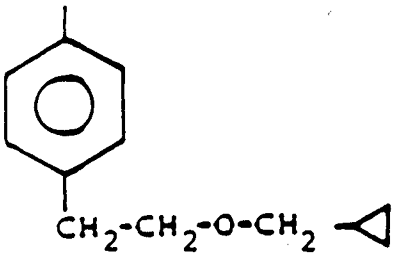 ,

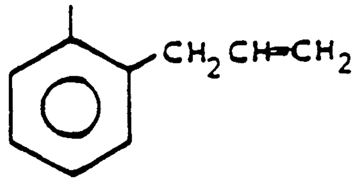 ,

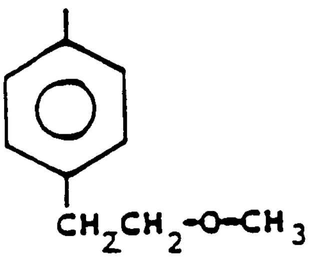 ,

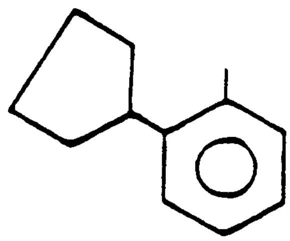 ,

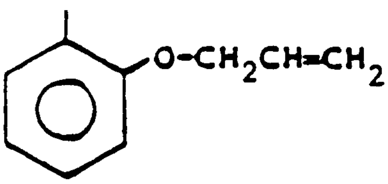 ou 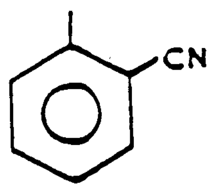 .

**9.** Composé selon la revendication 1 répondant à la formule

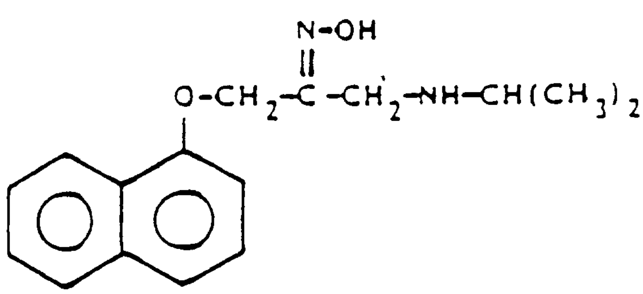

ou sel d'addition acide pharmaceutiquement acceptable d'un tel composé.

**10.** Composé selon la revendication 1 répondant à la formule

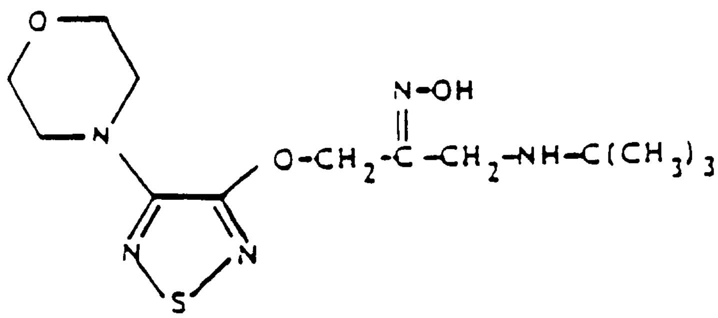

ou sel d'addition acide pharmaceutiquement acceptable d'un tel composé.

**11.** Composé selon la revendication 1 répondant à la formule

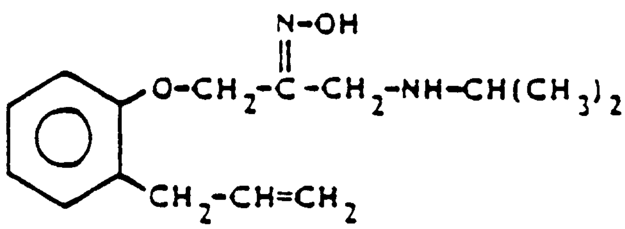

ou sel d'addition acide pharmaceutiquement acceptable d'un tel composé.

**12.** Composé selon la revendication 1 répondant à la formule

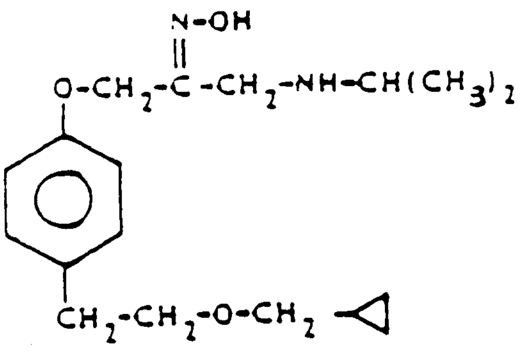

ou sel d'addition acide pharmaceutiquement acceptable d'un tel composé.

**13.** Composé selon la revendication 1 répondant à la formule

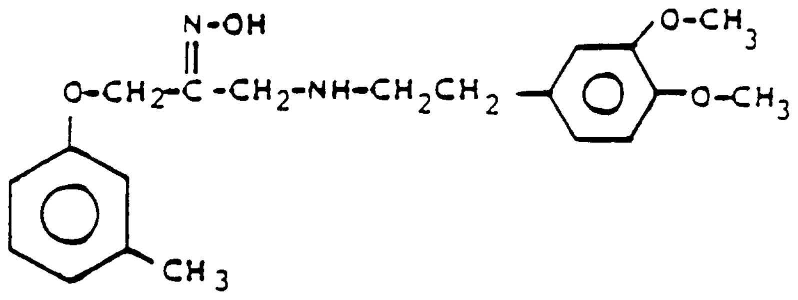

ou sel d'addition acide pharmaceutiquement acceptable d'un tel composé.

**14.** Composé selon la revendication 1 répondant à la formule

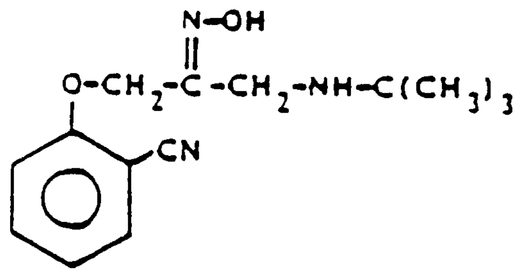

ou sel d'addition acide pharmaceutiquement acceptable d'un tel composé.

**15.** Composé selon la revendication 1 répondant à la formule

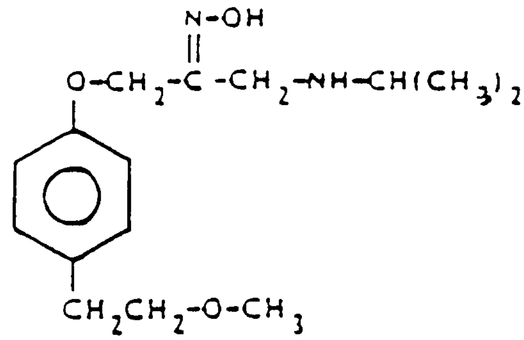

ou sel d'addition acide pharmaceutiquement acceptable d'un tel composé.

**16.** Composé selon la revendication 1 répondant à la formule

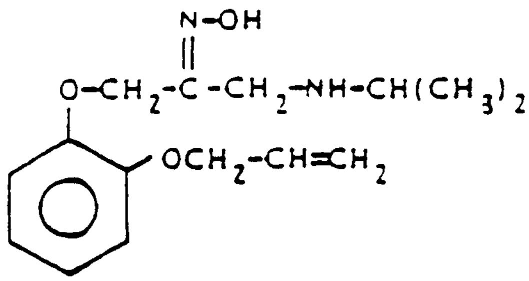

ou sel d'addition acide pharmaceutiquement acceptable d'un tel composé.

**17.** Composé selon la revendication 1 répondant à la formule

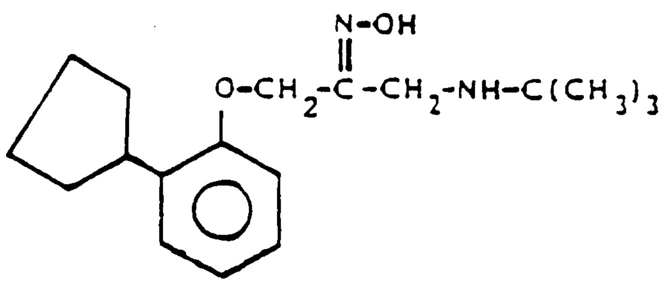

ou sel d'addition acide pharmaceutiquement acceptable d'un tel composé.

**18.** Composé selon la revendication 1 répondant à la formule

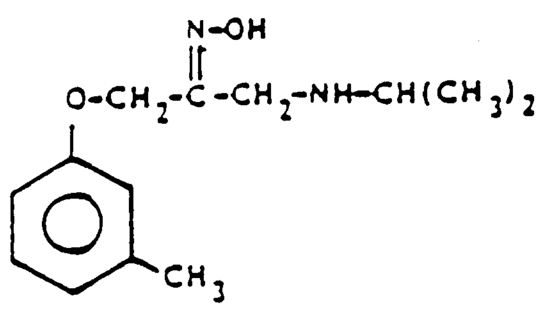

ou sel d'addition acide pharmaceutiquement acceptable d'un tel composé.

**19.** Composé selon la revendication 1 répondant à la formule

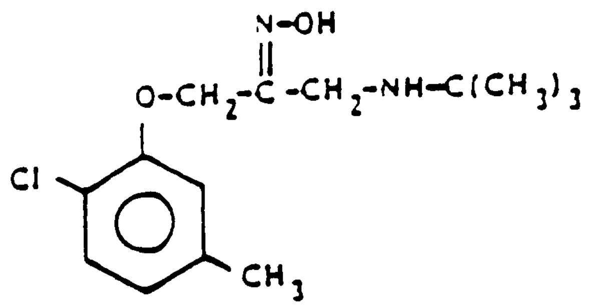

ou sel d'addition acide pharmaceutiquement acceptable d'un tel composé.

**20.** Utilisation du composé selon l'une quelconque des revendications 1 à 19 ou sel d'addition acide pharmaceutiquement acceptable d'un tel composé dans la préparation d'un médicament ayant un effet bloquant vis-à-vis de récepteurs $\beta$-adrénergiques.

**21.** Composition pharmaceutique de matière, en forme dosée unitaire, pour une utilisation dans la provocation d'une réponse de blocage de récepteurs $\beta$-adrénergiques chez un animal à sang chaud, ladite composition comprenant une quantité efficace au blocage de récepteurs $\beta$-adrénergiques d'un composé selon l'une quelconque des revendications 1 à 19, ou d'un sel d'addition acide pharmaceutiquement acceptable d'un tel composé, et un véhicule pharmaceutiquement acceptable, non toxique, pour un tel composé ou sel.

**22.** Utilisation d'un composé de formule

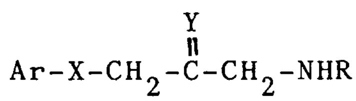

(I)

ou d'un sel d'addition acide pharmaceutiquement acceptable d'un tel composé, dans lequel -X-représente -O-, $-CH_2$-ou - ; =Y représente =O ou un groupe céto dérivé qui est hydrolysable ou enzymatiquement transformable en un groupe céto ; R est un groupe alkyle ayant de 1 à 12 atomes de carbone ou aralkyle ayant de 7 à 20 atomes de carbone, le groupe aralkyle portant éventuellement des substituants méthoxy sur sa partie aryle ; et Ar est le résidu 3-aromatique ou hétérocyclique d'un 1-alkylamino-2-propanol ayant, en position-3, un substituant aromatique ou hétérocyclique éventuellement substitué et ayant des propriétés bloquantes vis-à-vis de récepteurs $\beta$-adrénergiques ; dans la préparation d'un médicament ayant un effet d'abaissement de la pression intra-oculaire, en particulier pour une utilisation dans le traitement du glaucome.

**23.** Utilisation selon la revendication 22, dans laquelle =Y représente $=N-OR_1$, $=N-NH_2$, $=N-NR_1R_2$,

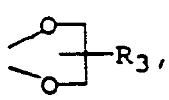 , 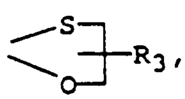 , ou 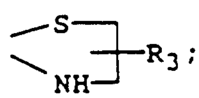 ;

$R_1$ et $R_2$ peuvent être identiques ou différents et représentent H ou alkyle ayant de 1 à 8 atomes de carbone ; et $R_3$représente $R_1$, $-COOR_1$ ou $-CON(R_1)_2$ où $R_1$ est tel que défini ci-dessus.

**24.** Utilisation selon la revendication 23, dans laquelle =Y représente $=NOR_1$ où $R_1$ représente H ou alkyle ayant de 1 à 8 atomes de carbone.

**25.** Utilisation selon l'une quelconque des revendications 22 à 24, dans laquelle Ar est le résidu 3-aromatique ou hétérocyclique du propanolol, timolol, cartéolol, alprénolol, aténolol, béfunolol, bétaxolol, bévantolol, bufuralol, bunitrolol, bupranolol, céliprolol, cétamolol, labétolol, lévobunolol, mépindolol, métipranolol, métoprolol, moprolol, nadolol, oxprénolol, penbutolol, pindolol ou tolipropol.

**26.** Utilisation selon la revendication 22, dans laquelle le composé est choisi dans le groupe comprenant

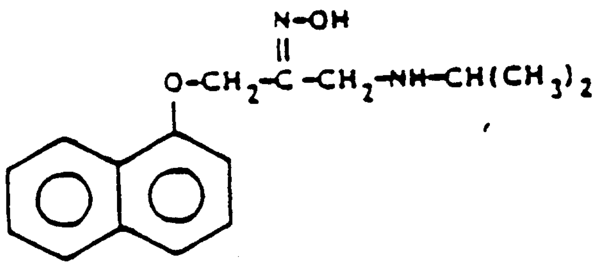

,

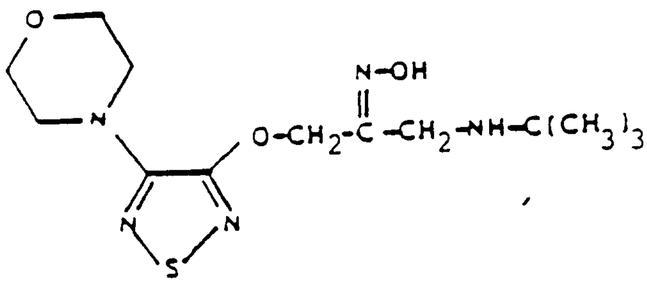

,

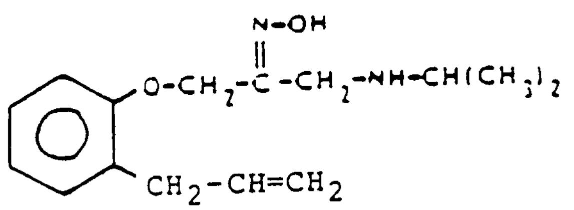

,

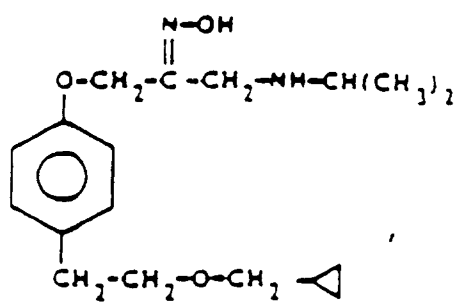

,

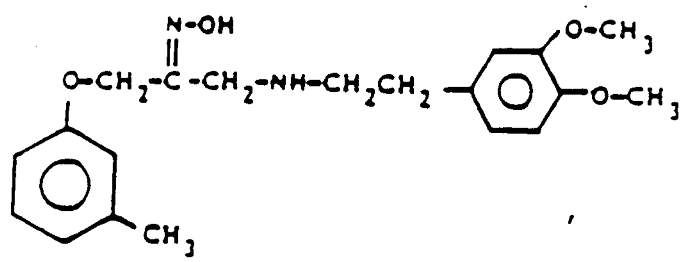

,

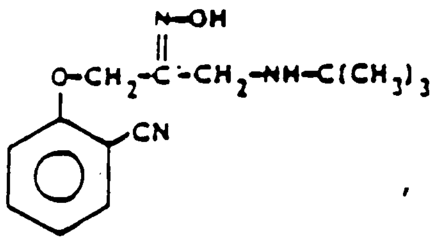

,

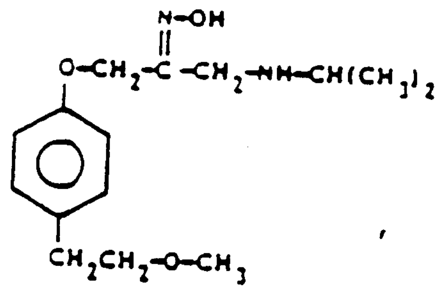

,

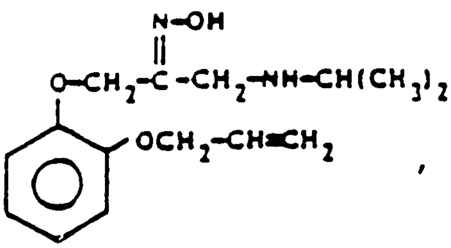

,

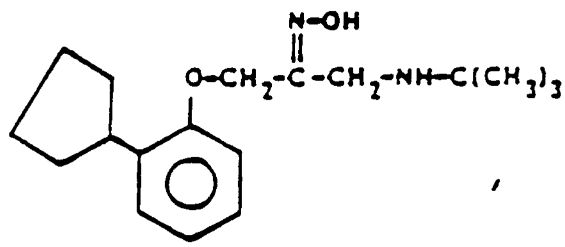

,

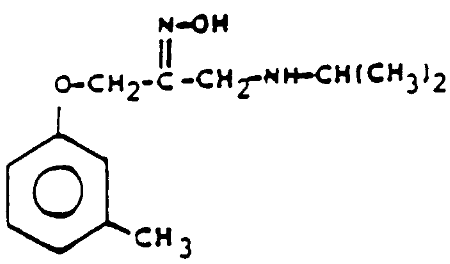

et

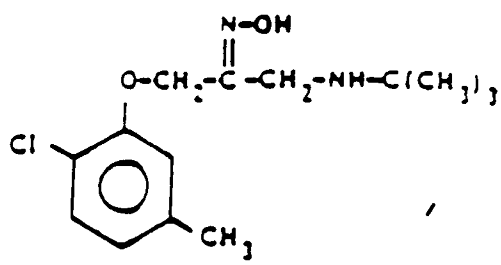

,

et les sels d'addition acide pharmaceutiquement acceptables de tels composés.

**27.** Composition pharmaceutique ophtalmique de matière, en forme dosée unitaire, pour une utilisation dans l'abaissement de la pression intra-oculaire chez un animal à sang chaud, en particulier pour le traitement du glaucome, ladite composition comprenant une quantité efficace à la réduction de la pression intra-oculaire d'un composé de formule

$$Ar\text{-}X\text{-}CH_2\text{-}\overset{\overset{\displaystyle Y}{\|}}{C}\text{-}CH_2\text{-}NHR \qquad (I)$$

ou d'un sel d'addition acide pharmaceutiquement acceptable d'un tel composé, selon la revendication 22, et un véhicule ophtalmologiquement acceptable, non toxique, pour un tel composé ou sel.

**28.** Composition selon la revendication 27, dans laquelle =Y représente =N-OR$_1$, =N-NH$_2$, =N-NR$_1$R$_2$,

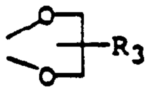, 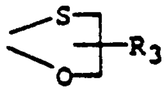, ou 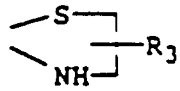;

R$_1$ et R$_2$ peuvent être identiques ou différents et représentent H ou alkyle ayant de 1 à 8 atomes de carbone ; et R$_3$ représente R$_1$, -COOR$_1$ ou -CON(R$_1$)$_2$ où R$_1$ est tel que défini ci-dessus.

**29.** Composition selon la revendication 28, dans laquelle =Y représente =NOR$_1$ où R$_1$ représente H ou alkyle ayant de 1 à 8 atomes de carbone.

**30.** Composition selon l'une quelconque des revendications 27 à 29, dans laquelle Ar est le résidu 3-aromatique ou hétérocyclique du propanolol, timolol, cartéolol, alprénolol, aténolol, béfunolol, bétaxolol, bévantolol, bufuralol, bunitrolol, bupranolol, céliprolol, cétamolol, labétolol, lévobunolol, mépindolol, métipranolol, métoprolol, moprolol, nadolol, oxprénolol, penbutolol, pindolol ou toliprolol.

**31.** Composition selon la revendication 27, renfermant une quantité efficace à la réduction de la pression intra-oculaire d'un composé de formule

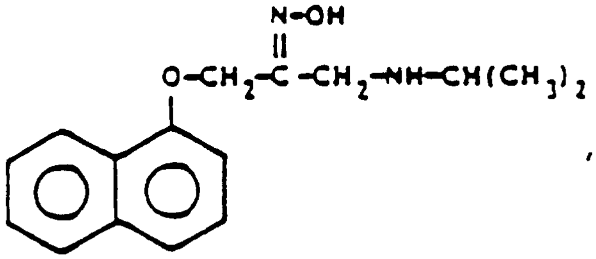

,

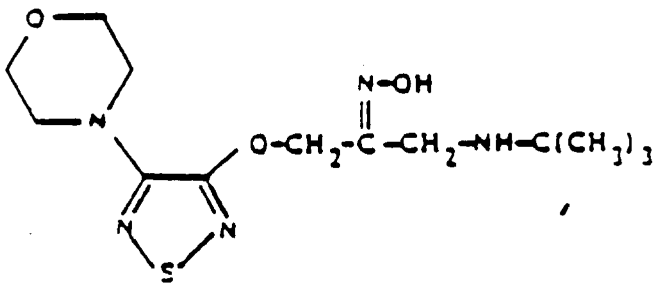

,

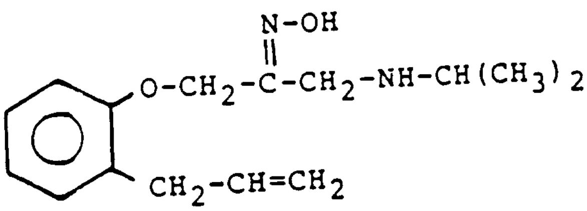

,

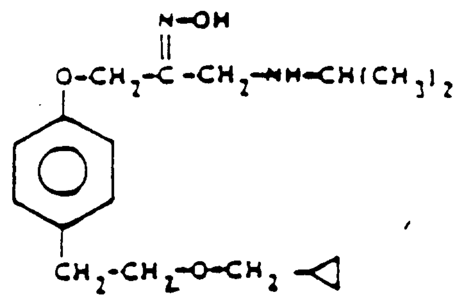

,

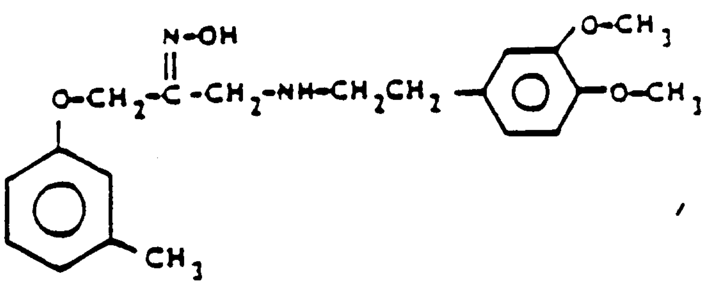

,

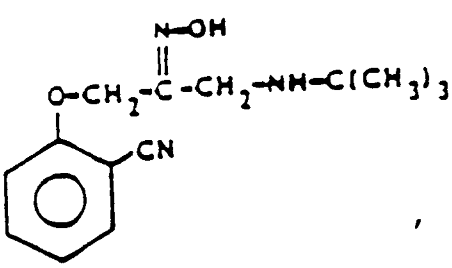

,

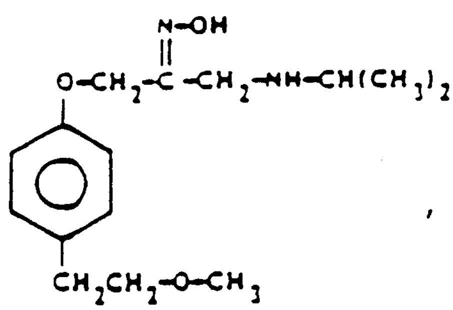

,

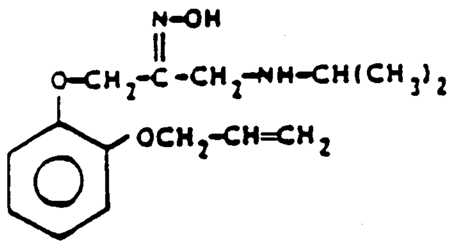,

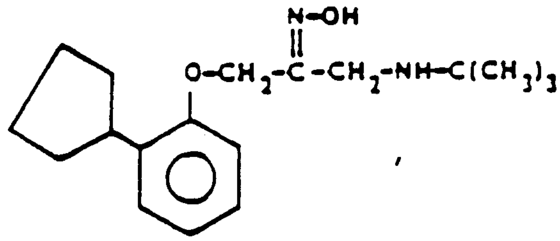,

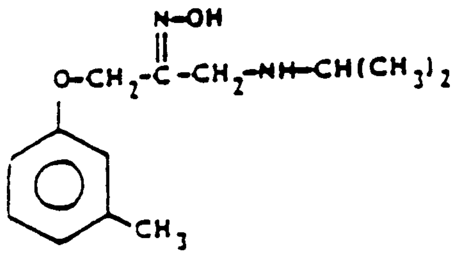

ou

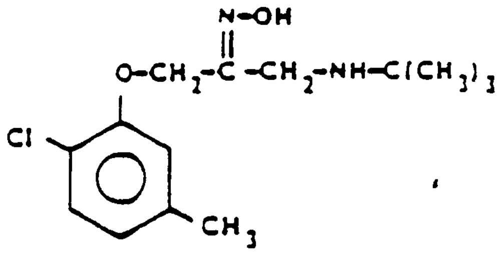,

ou d'un sel d'addition acide pharmaceutiquement acceptable d'un tel composé.

**32.** Composés selon les revendications 1 à 19 pour une utilisation en thérapie.

**Revendications pour les Etats contractants suivants : ES, GR**

**1.** Procédé de préparation d'une composition pharmaceutique de matière pour une utilisation dans la provocation d'une réponse de blocage de récepteurs $\beta$-adrénergiques, chez un animal à sang chaud, ledit procédé consistant à mélanger un composé de formule :

$$Ar-X-CH_2-\overset{\overset{Y}{\|}}{C}-CH_2-NHR \qquad (I)$$

ou un sel d'addition acide pharmaceutiquement acceptable d'un tel composé, dans lequel -X- représente -O-, $-CH_2$-ou - ; =Y est un groupe céto dérivé qui est hydrolysable ou transformable enzymatiquement en groupe céto ; R est un groupe alkyle ayant de 1 à 12 atomes de carbone ou aralkyle ayant de 7 à 20 atomes de carbone, le groupe aralkyle portant éventuellement des substituants méthoxy sur sa partie aryle ; et Ar est le résidu 3-aromatique ou hétérocyclique d'un 1-alkylamino-2-propanol ayant, en position-3, un substituant aromatique ou hétérocyclique éventuellement substitué et ayant des propriétés de blocage vis-à-vis de récepteurs $\beta$-adrénergiques, avec un véhicule pharmaceutiquement acceptable, non toxique, pour un tel composé ou un sel, et à diviser le mélange résultant en formes dosées unitaires, dont chacune renferme une quantité efficace au blocage de récepteurs $\beta$-adrénergiques du composé de formule (I) ou d'un sel d'un tel composé.

**2.** Procédé selon la revendication 1, dans lequel =Y représente $=N-OR_1$, $=N-NH_2$, $=N-NR_1R_2$,

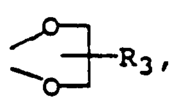, 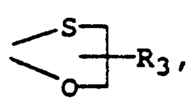, ou 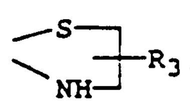;

$R_1$ et $R_2$ peuvent être identiques ou différents et représentent H ou alkyle ayant de 1 à 8 atomes de carbone ; et $R_3$ représente $R_1$, $-COOR_1$ ou $-CON(R_1)_2$ où $R_1$ est tel que défini ci-dessus.

3. Procédé selon la revendication 2, dans lequel =Y représente =$NOR_1$ où $R_1$ représente H ou alkyle ayant de 1 à 8 atomes de carbone.

4. Procédé selon la revendication 3, dans lequel =Y représente =NOH.

5. Procédé selon l'une quelconque des revendications précédentes, dans lequel -X- représente -O-.

6. Procédé selon l'une quelconque des revendications 1 à 5, dans lequel R est un groupe isopropyle ou t-butyle.

7. Procédé selon l'une quelconque des revendications 1 à 6, dans lequel Ar est le résidu 3-aromatique ou hétérocyclique du propanolol, timolol, cartéolol, alprénolol, aténolol, béfunolol, bétaxolol, bévantolol, bufuralol, bunitrolol, bupranolol, céliprolol, cétamolol, labétolol, lévobunolol, mépindolol, métipranolol, métoprolol, moprolol, nadolol, oxprénolol, penbutolol, pindolol ou du toliprolol.

8. Procédé selon l'une quelconque des revendications 1 à 6, dans lequel Ar représente

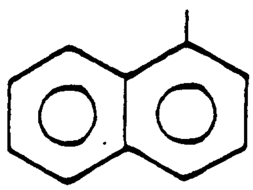 ,

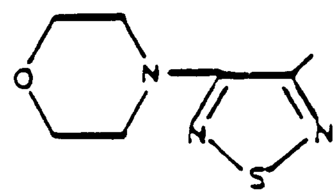 ,

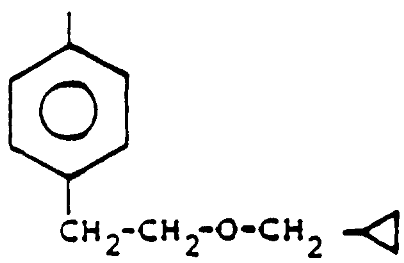 ,

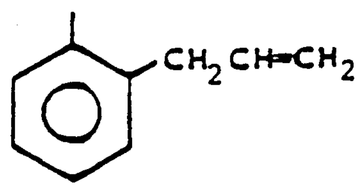 ,

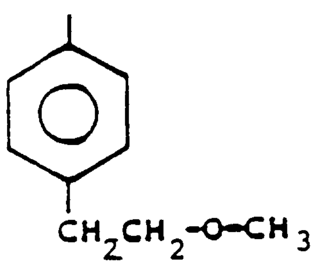 ,

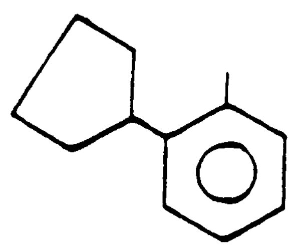 ,

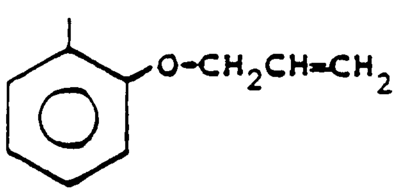 ou 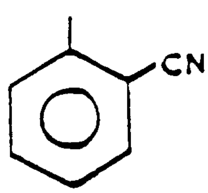 .

9. Procédé selon la revendication 1, dans lequel le composé de formule générale I est

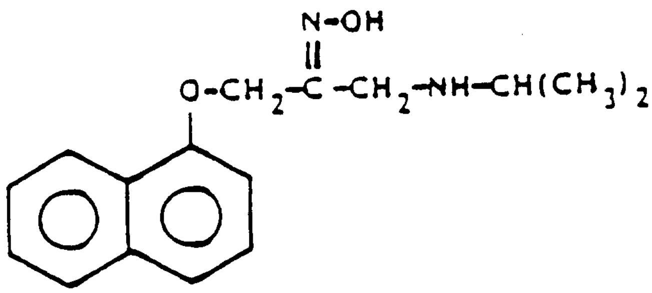

ou un sel d'addition acide pharmaceutiquement acceptable d'un tel composé.

10. Procédé selon la revendication 1, dans lequel le composé de formule générale I est

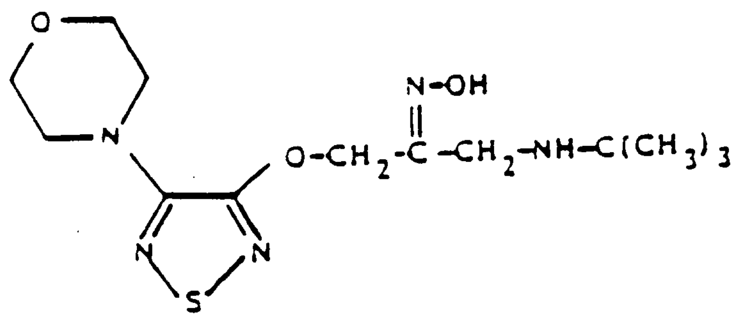

ou un sel d'addition acide pharmaceutiquement acceptable d'un tel composé.

11. Procédé selon la revendication 1, dans lequel le composé de formule générale I est

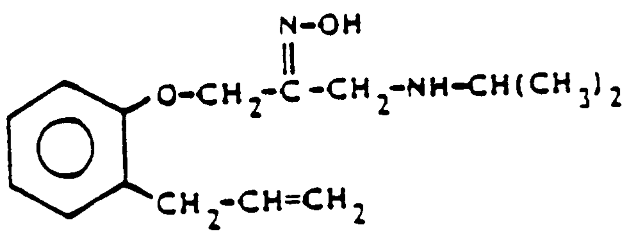

ou un sel d'addition acide pharmaceutiquement acceptable d'un tel composé.

**12.** Procédé selon la revendication 1, dans lequel le composé de formule générale I est

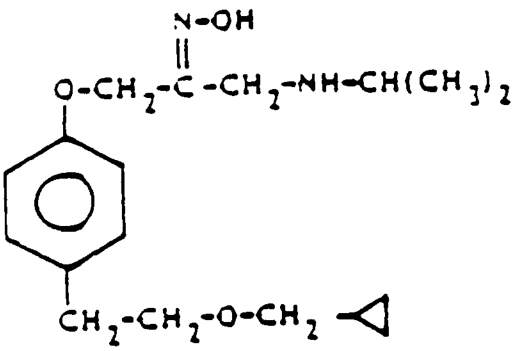

ou un sel d'addition acide pharmaceutiquement acceptable d'un tel composé.

**13.** Composé selon la revendication 1, dans lequel le composé de formule générale I est

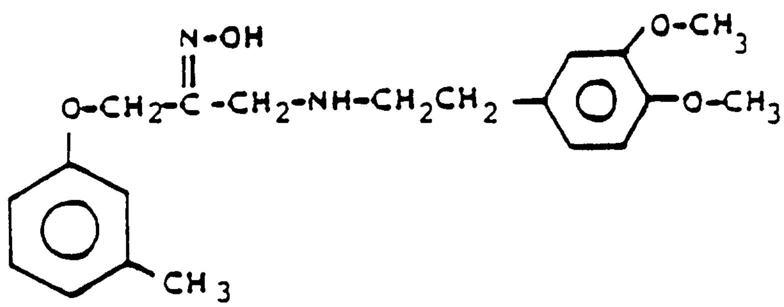

ou un sel d'addition acide pharmaceutiquement acceptable d'un tel composé.

**14.** Procédé selon la revendication 1, dans lequel le composé de formule générale I est

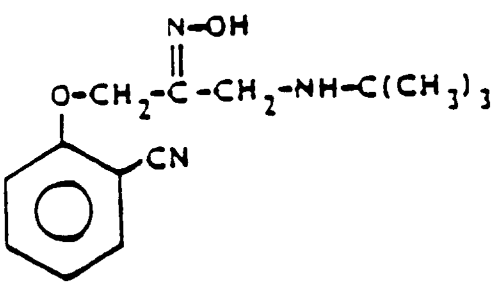

ou un sel d'addition acide pharmaceutiquement acceptable d'un tel composé.

**15.** Procédé selon la revendication 1, dans lequel le composé de formule générale I est

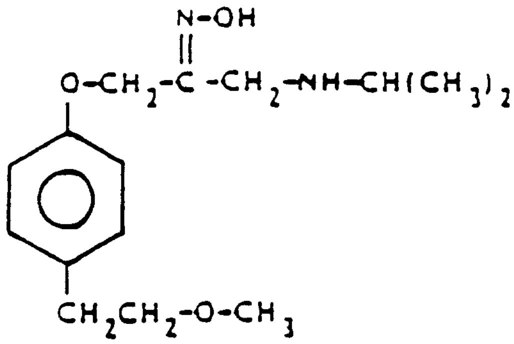

ou un sel d'addition acide pharmaceutiquement acceptable d'un tel composé.

**16.** Procédé selon la revendication 1, dans lequel le composé de formule générale I est

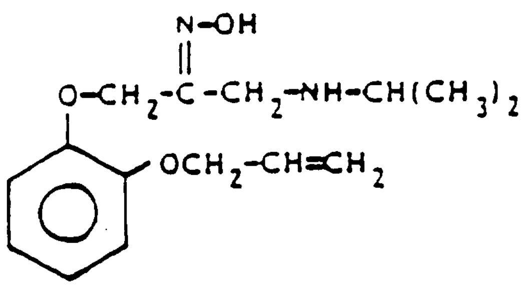

ou un sel d'addition acide pharmaceutiquement acceptable d'un tel composé.

**17.** Procédé selon la revendication 1, dans lequel le composé de formule générale I est

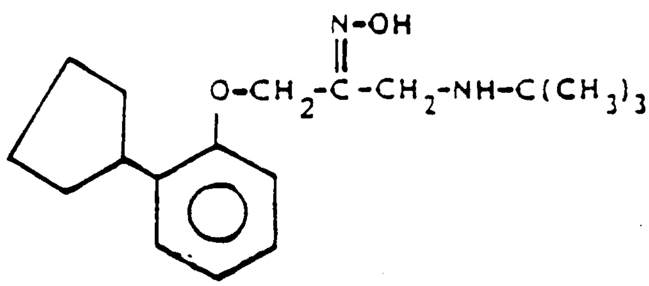

ou un sel d'addition acide pharmaceutiquement acceptable d'un tel composé.

**18.** Procédé selon la revendication 1, dans lequel le composé de formule générale I est

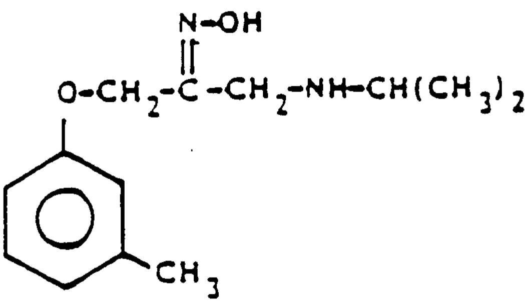

ou un sel d'addition acide pharmaceutiquement acceptable d'un tel composé.

**19.** Procédé selon la revendication 1, dans lequel le composé de formule générale I est

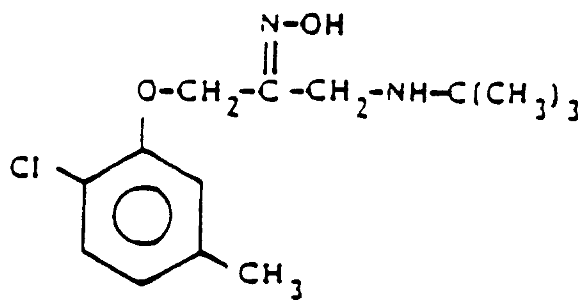

ou un sel d'addition acide pharmaceutiquement acceptable d'un tel composé.

**20.** Utilisation d'un composé de formule I ou d'un sel d'addition acide pharmaceutiquement acceptable d'un tel composé, tel que défini dans l'une quelconque des revendications 1 à 19, dans la préparation d'un médicament ayant un effet bloquant vis-à-vis de récepteurs $\beta$-adrénergiques.

**21.** Procédé de préparation d'une composition de matière pharmaceutique ophtalmique pour une utilisation dans l'abaissement de la pression intra-oculaire chez un animal à sang chaud, en particulier dans le traitement du glaucome, ledit procédé consistant à mélanger un composé de formule

$$Ar-X-CH_2-\overset{\overset{\displaystyle Y}{\|}}{C}-CH_2-NHR \qquad (I)$$

ou un sel d'addition acide pharmaceutiquement acceptable d'un tel composé, dans lequel -X- représente -O-, $-CH_2-$ou - ; =Y représente =O ou un groupe céto dérivé qui est hydrolysable ou enzymatiquement transformable en un groupe céto ; R est un groupe alkyle ayant de 1 à 12 atomes de carbone ou aralkyle ayant de 7 à 20 atomes de carbone, le groupe aralkyle portant éventuellement des substituants méthoxy sur sa partie aryle ; et Ar est le résidu 3-aromatique ou hétérocyclique d'un 1-alkylamino-2-propanol ayant, en position-3, un substituant aromatique ou hétérocyclique éventuellement substitué et ayant des propriétés bloquantes vis-à-vis de récepteurs $\beta$-adrénergiques ; avec un véhicule ophtalmologiquement acceptable, non toxique, pour un tel composé ou un sel, et à diviser le mélange résultant en formes dosées unitaires dont chacune renferme une quantité du composé de formule I, ou d'un sel d'un tel composé, efficace à réduire la pression intra-oculaire.

**22.** Procédé selon la revendication 21, dans laquelle =Y représente =N-OR$_1$, =N-NH$_2$, =N-NR$_1$R$_2$,

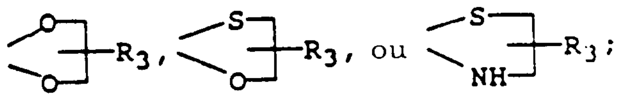

$R_1$ et $R_2$ peuvent être identiques ou différents et représentent H ou alkyle ayant de 1 à 8 atomes de carbone ; et $R_3$ représente $R_1$, -COOR$_1$ ou -CON(R$_1$)$_2$ où $R_1$ est tel que défini ci-dessus.

**23.** Utilisation selon la revendication 22, dans laquelle =Y représente =NOR$_1$ où $R_1$ représente H ou alkyle ayant de 1 à 8 atomes de carbone.

**24.** Procédé selon l'une quelconque des revendications 21 à 23, dans laquelle Ar est le résidu 3-aromatique ou hétérocyclique du propanolol, timolol, cartéolol, alprénolol, aténolol, béfunolol, bétaxolol, bévantolol, bufuralol, bunitrolol, bupranolol, céliprolol, cétamolol, labétolol, lévobunolol, mépindolol, métipranolol, métoprolol, moprolol, nadolol, oxprénolol, penbutolol, pindolol ou tolipropol.

**25.** Procédé selon la revendication 21, mettant en oeuvre une quantité d'un composé de formule

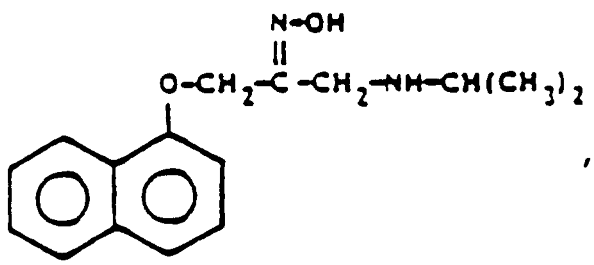

,

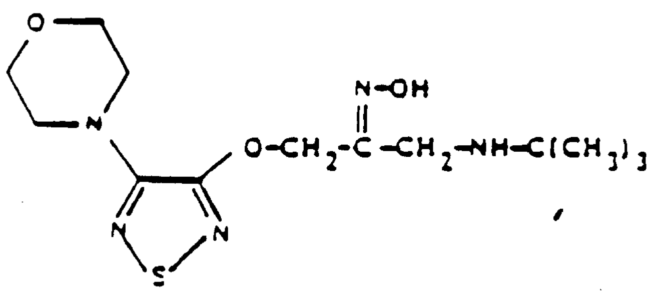

,

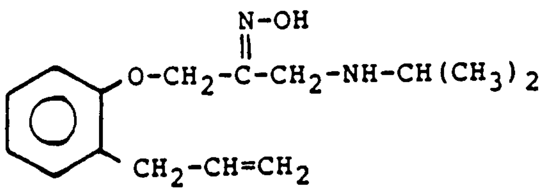 ,

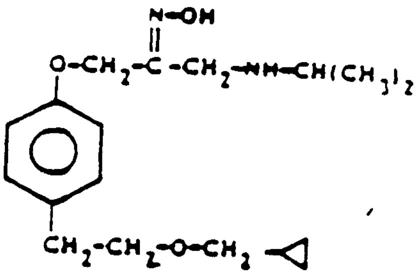 ,

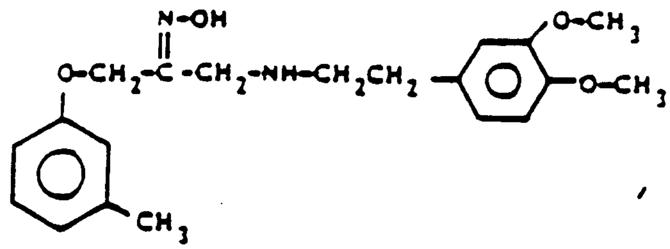 ,

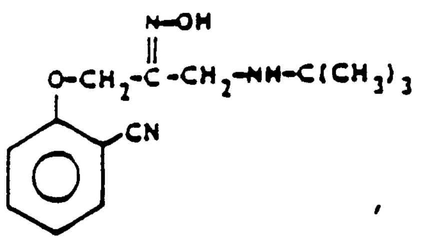 ,

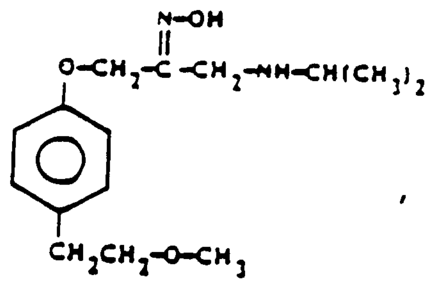 ,

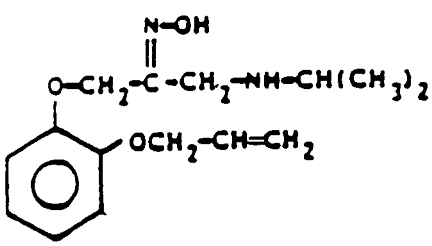 ,

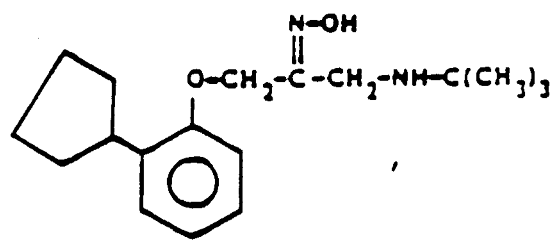 ,

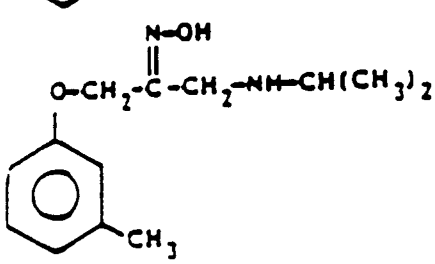 ou

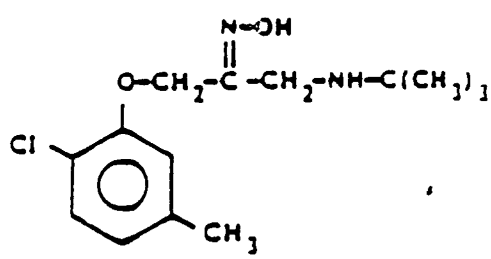 ,

ou d'un sel d'addition acide pharmaceutiquement acceptable d'un tel composé, efficace à la réduction de la pression intra-oculaire.

**26.** Utilisation d'un composé de formule

$$Ar-X-CH_2-\overset{\overset{Y}{\|}}{C}-CH_2-NHR \qquad (I)$$

ou d'un sel d'addition acide pharmaceutiquement acceptable d'un tel composé, selon la revendication 21, dans la préparation d'un médicament ayant un effet abaissant la pression intra-oculaire, en particulier pour le traitement du glaucome.

**27.** Utilisation selon la revendication 26, dans laquelle =Y représente =N-O$R_1$, =N-$NH_2$, =N-N$R_1R_2$,

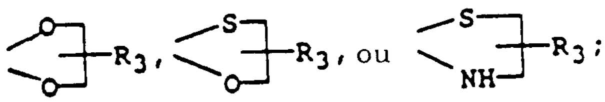

$R_1$ et $R_2$ peuvent être identiques ou différents et représentent H ou alkyle ayant de 1 à 8 atomes de carbone ; et $R_3$ représente $R_1$, -COO$R_1$ ou -CON$(R_1)_2$ où $R_1$ est tel que défini ci-dessus.

**28.** Utilisation selon la revendication 27, dans laquelle =Y représente =NO$R_1$ où $R_1$ représente H ou alkyle ayant de 1 à 8 atomes de carbone.

**29.** Utilisation selon l'une quelconque des revendications 26 à 28, dans laquelle Ar est le résidu 3-aromatique ou hétérocyclique du propanolol, timolol, cartéolol, alprénolol, aténolol, béfunolol, bétaxolol, bévantolol, bufuralol, bunitrolol, bupranolol, céliprolol, cétamolol, labétolol, lévobunolol, mépindolol, métipranolol, métoprolol, moprolol, nadolol, oxprénolol, penbutolol, pindolol ou toliprolol.

**30.** Utilisation selon la revendication 26, dans laquelle le composé est choisi dans le groupe comprenant :

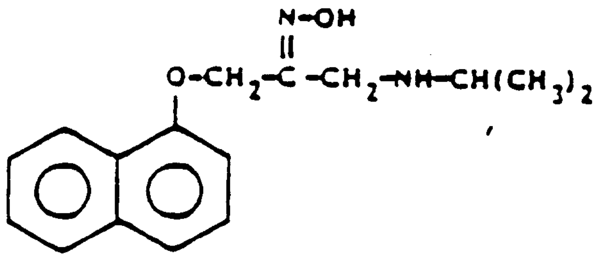

,

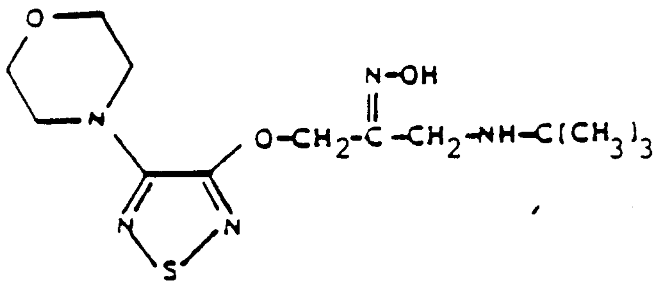

,

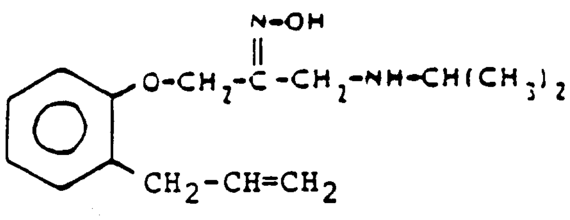

,

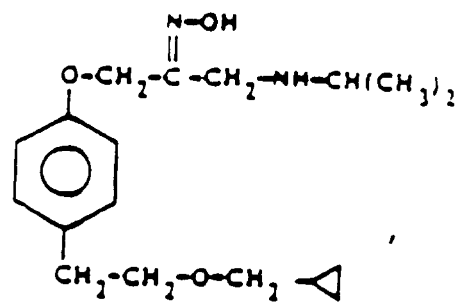

,

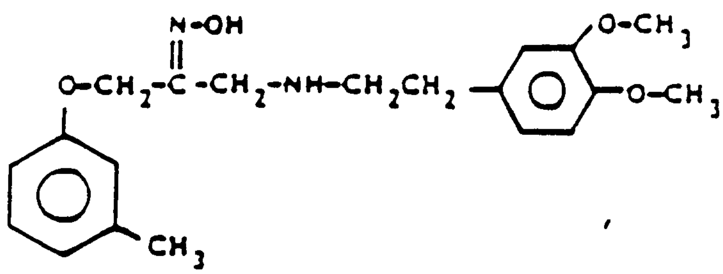

,

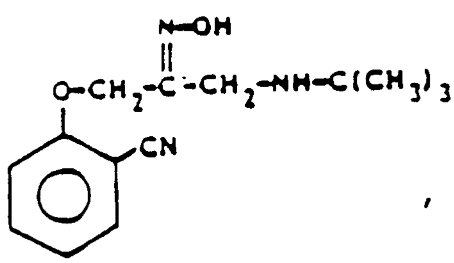

,

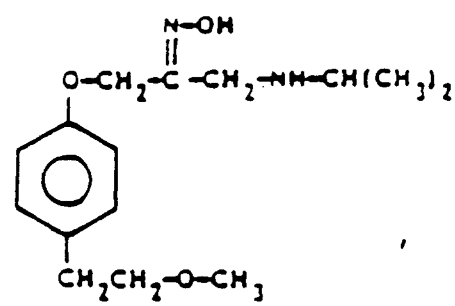

,

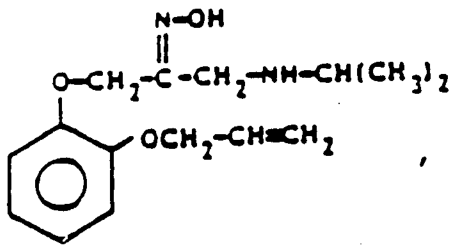

,

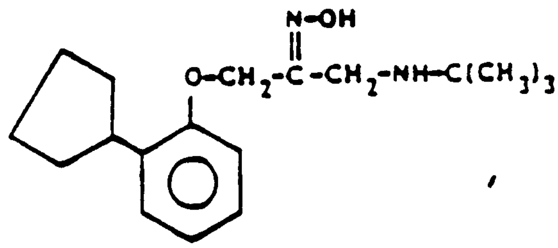

,

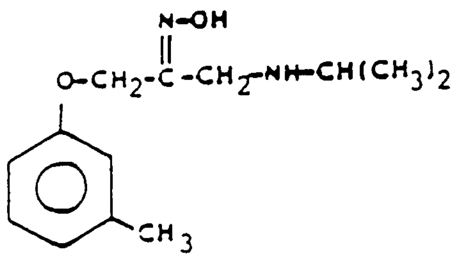

et

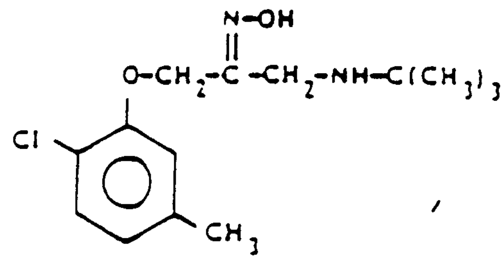

,

et les sels d'addition acide pharmaceutiquement acceptables de tels composés.

Fig 1

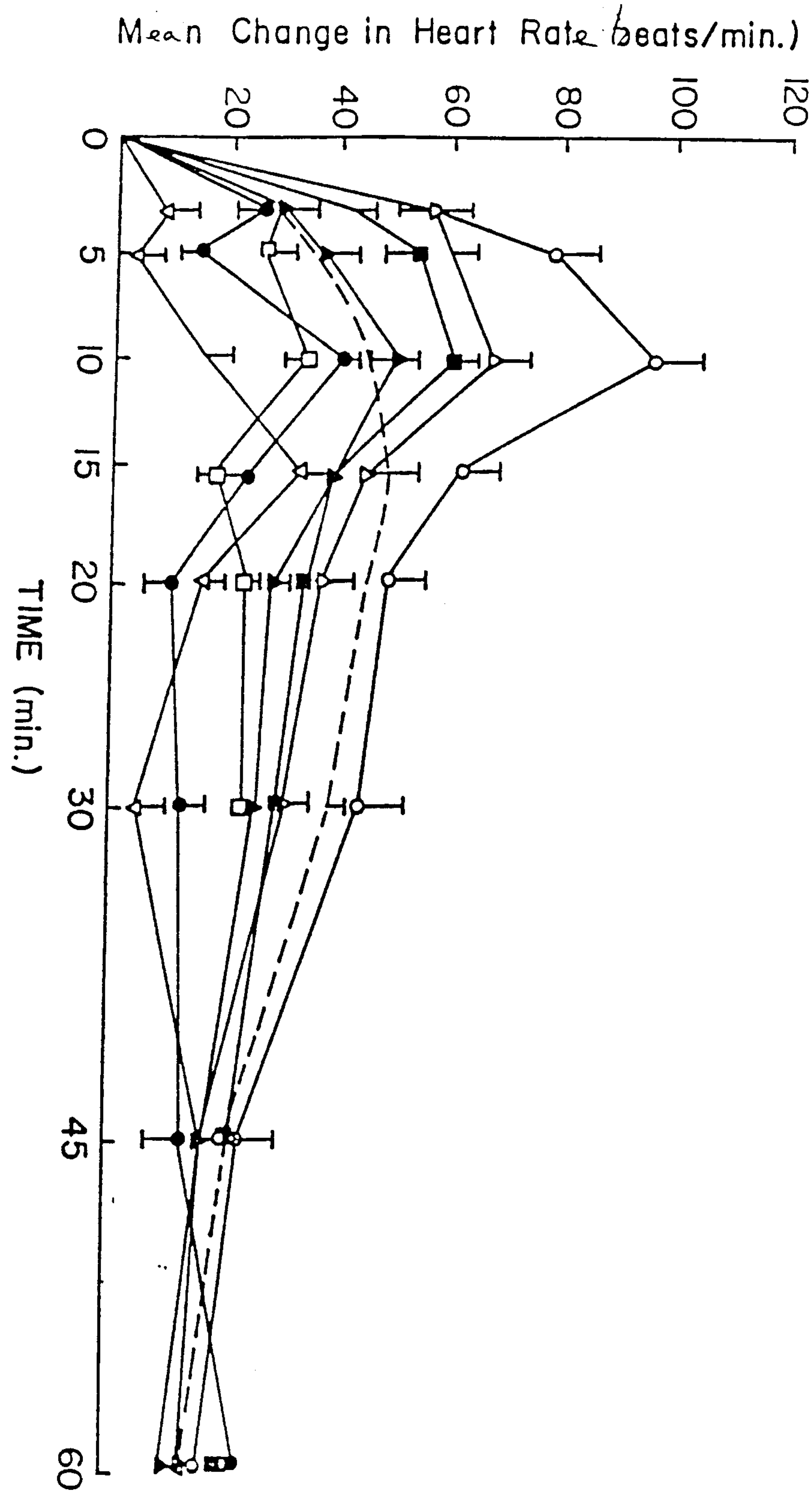
Mean Change in Heart Rate (beats/min.)
0
20
40
60
80
100
120
TIME (min.)
0
5
10
15
20
30
45
60

Fig 2

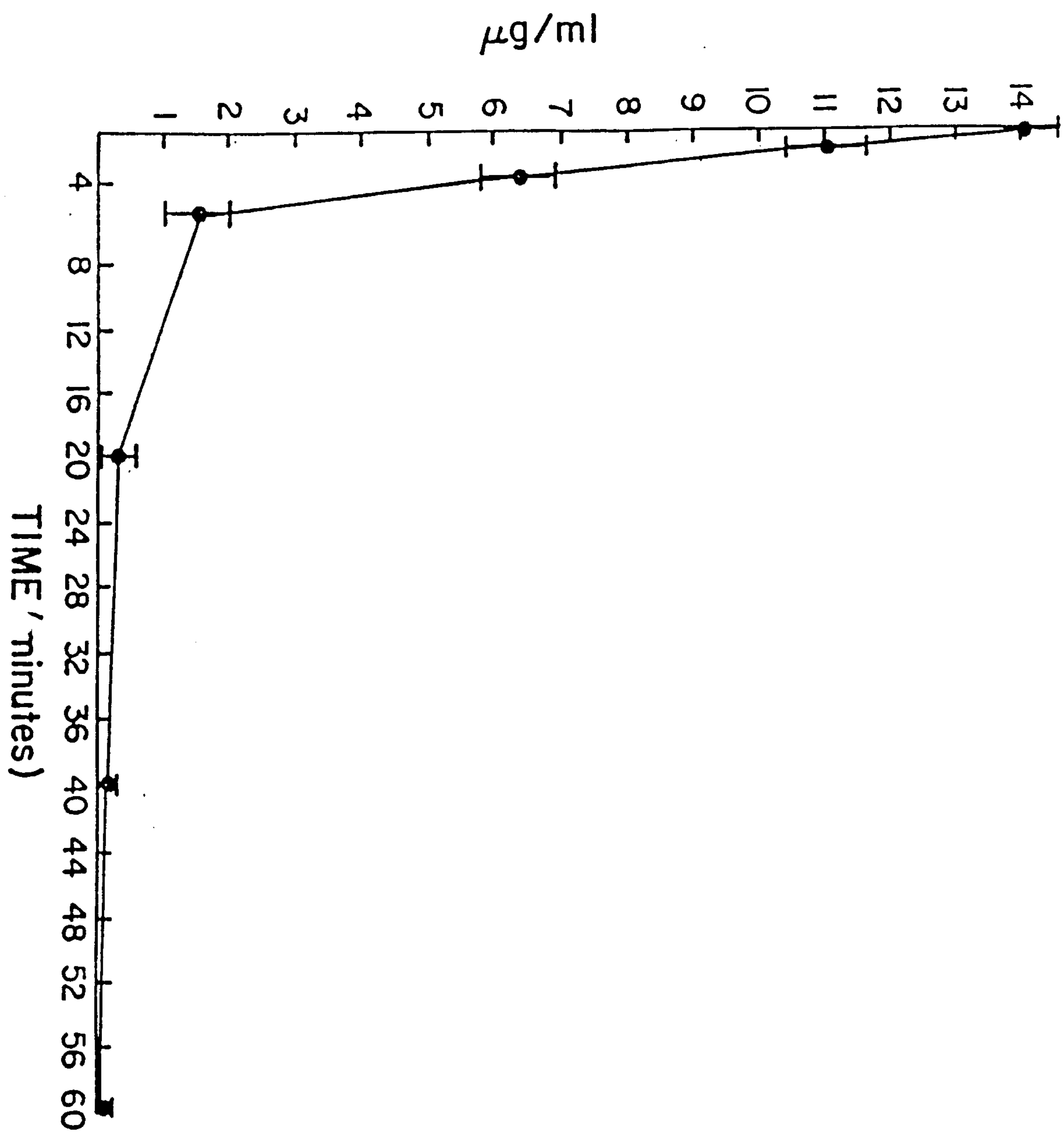
μg/ml
1 2 3 4 5 6 7 8 9 10 11 12 13 14
4 8 12 16 20 24 28 32 36 40 44 48 52 56 60
TIME (minutes)